# EUROPEAN PATENT SPECIFICATION

(11) **EP 3 679 027 B1**
(45) Date of publication and mention of the grant of the patent: **09.12.2026**
(21) Application number: 18762093.5
(22) Date of filing: 03.09.2018
(51) Int. Cl.: C07D 401/14, A61K 31/4709, A61K 31/5517, C07D 401/04, C07D 401/12, C07D 235/26, C07D 403/04, C07D 403/06, C07D 403/14, C07D 417/04, C07D 487/04, C07D 495/14, C07D 519/00

(54) **DIHYDROBENZIMIDAZOLONES**
DIHYDROBENZIMIDAZOLONE
DIHYDROBENZIMIDAZOLONES

(30) Priority: 04.09.2017 EP 17189228
(43) Date of publication of application: 15.07.2020
(73) Proprietor: C4 Therapeutics, Inc., Watertown, MA 02472 (US)
(72) Inventor: GOERGLER, Annick, 4070 Basel (CH); NORCROSS, Roger, 4070 Basel (CH); DEY, Fabian, 4070 Basel (CH); KUSZNIR, Eric Andre, 4070 Basel (CH)
(74) Representative: Bassil, Nicholas Charles
(86) International application number: PCT/EP2018/073584
(87) International publication number: WO 2019/043217

(56) References cited:
- EP-A2- 0 385 850
- WO-A1-01/92239
- WO-A1-2005/060967
- WO-A1-2007/065518
- WO-A1-2009/135651
- WO-A1-2010/085684
- WO-A1-2010/130794
- WO-A1-2011/035124
- WO-A1-2011/130628
- WO-A1-2012/021382
- WO-A1-2012/174199
- WO-A1-2016/176449
- WO-A1-2016/176460
- WO-A1-2017/046318
- WO-A2-2011/156245
- US-A1- 2003 092 749
- US-A1- 2010 081 643
- US-A1- 2016 008 356
- CHEN H Y ET AL: "Synthesis of structurally diverse benzimidazolyl benzimidazolones by application of soluble polymer support", TETRAHEDRON, ELSEVIER SIENCE PUBLISHERS, AMSTERDAM, NL, vol. 64, no. 27, 30 June 2008 (2008-06-30), pages 6387 - 6394, XP022695616, ISSN: 0040-4020, [retrieved on 20080425], DOI: 10.1016/J.TET.2008.04.081
- TEREFENKO E A ET AL: "SAR studies of 6-aryl-1,3-dihydrobenzimidazol-2-ones as progesterone receptor antagonists", BIOORGANIC & MEDICINAL CHEMISTRY LET, vol. 15, no. 15, 1 August 2005 (2005-08-01), PERGAMON, AMSTERDAM, NL, pages 3600 - 3603, XP004969903, ISSN: 0960-894X, DOI: 10.1016/J.BMCL.2005.05.082
- SMOLYAR N N ET AL: "Synthesis of 1,3-dialkyl-5-(hetaryl-1-yl)-1,3-dihydrobenzimidazol-2-ones", RUSSIAN JOURNAL OF ORGANIC CHEMISTRY, vol. 47, no. 8, 11 September 2011 (2011-09-11), NAUKA/INTERPERIODICA, MO, pages 1190 - 1193, XP019950597, ISSN: 1608-3393, DOI: 10.1134/S1070428011080112
- JUN YON CHOI ETAL.: "Structure guided development of novel thymidine minetics targeting Pseudomonas aeruginosa Thymidylate kinase: From hit to lead generation", JOURNAL OF MEDICINAL CHEMISTRY., vol. 55, 13 January 2012 (2012-01-13), USAMERICAN CHEMICAL SOCIETY. WASHINGTON., pages 852 - 870, XP002778247, ISSN: 0022-2623
- HYEJU CHOI ETAL.: "The impact of an indeno[1,2-b]thiophene spacer on dye-sensitized solar cell performances of cyclic thiourea functionalized organic sensitizers", JOURNAL OF MATERIALS CHEMISTRY A, vol. 2, 2014, pages 12931 - 12939, XP002778297

## Description

### Field of the Invention

The present invention provides compounds which bind to the ubiquitously expressed E3 ligase protein cereblon (CRBN) and alter the substrate specificity of the CRBN E3 ubiquitin ligase complex, resulting in breakdown of intrinsic downstream proteins. Present compounds are thus useful for the treatment of various cancers.

### Background of the Invention

The field of targeted protein degradation promoted by small molecules has been intensively studied over the last years¹.

Protein degradation plays a role in various cellular functions, i.e. the concentrations of regulatory proteins are adjusted through degradation into small peptides to maintain health and productivity of the cells.

Cereblon is a protein that forms an E3 ubiquitin ligase complex, which ubiquinates various other proteins. Cereblon is known as primary target for anticancer thalidomide analogs. A higher expression of cereblon has been linked to the efficiency of thalidomide analogs in cancer therapy.

In recent years, a few bifunctional compounds have been described as useful modulators of targeted ubiquitination, e.g. WO2013020557 ², WO2013063560 ³ , WO 2013106643 ⁴ , WO2015160845⁵, WO2016011906⁶, WO2016105518⁷, WO2017007612⁸, WO2017024318⁹ and WO2017117473¹⁰.

WO2011035124 ¹¹ , WO02072576 ¹² , WO2010085684 ¹³ , WO2014134240 ¹⁴ , WO2011130628¹⁵, WO2007065518¹⁶, WO2016176460¹⁷, WO2009135651¹⁸, WO2011156245¹⁹, WO2017046318²⁰, WO2010130794²¹, WO2012021382²², WO2005060967²³, WO2012174199²⁴, EP385850 ²⁵ , WO2016176449 ²⁶ , WO2016040508 ²⁷ , WO0020358 ²⁸ , EP1256578 ²⁹ , WO2006058338³⁰, WO2004108133³¹, WO2010108187³², WO2005113489³³, WO2012061708³⁴, WO2010023161³⁵, WO2007041598³⁶, WO2009050232³⁷, Terefenko *et al*.³⁸*,* Smolyar *et al.*³⁹*,* Jun Yon Choi *et al*.⁴⁰*,* Hyeju Choi *et al.*⁴¹*,* Mitsuaki *et al.*⁴²*,* Yu *et al.*⁴³*,* Vincent *et al*.⁴⁴ describe structurally related compounds.

However, there is still an ongoing need for effective treatment of cancers.

### Cited documents

US2010/081643 describes cyclic benzimidazole derivatives.

### Summary of the Invention

The present invention is defined by the appended claims.

The present invention provides dihydrobenzimidazolones, or a pharmaceutically acceptable salt thereof, of formula 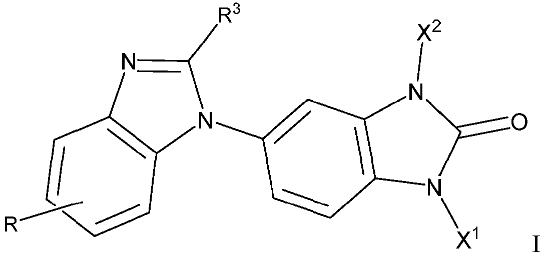 wherein
- X¹: is H or -C₁₋₆alkyl,
- X²: is H or -C₁₋₆alkyl,
- R: is H, -C(O)NHMe, or -C(O)OMe;
- R³: is independently selected from the group consisting of
i) -(CH₂)₀₋₁-(C₃₋₇cycloalkyl)-aryl substituted by R¹⁰,
ii) -(CH₂)₀₋₁-(C₃₋₇cycloalkyl)-aryl,
iii) -(CH₂)₀₋₁-aryl substituted by R¹⁰,
iv) -(CH₂)₀₋₁-aryl,
v) -(CH₂)₀₋₁-C₃₋₇cycloalkyl substituted by R¹⁰,
vi) -(CH₂)₀₋₁-C₃₋₇cycloalkyl,
vii) -(CH₂)₀₋₁-heteroaryl substituted by R⁹,
viii) -(CH₂)₀₋₁-heteroaryl,
ix) -(CH₂)₀₋₁-heterocycloalkyl,
x) -C(=O)C₁₋₆alkyl
xi) -(CH₂)₀₋₂-C(=O)-N(R⁷,R⁸),
xii) -C(=O)OC₁₋₆alkyl,
xiii) -C₁₋₆alkoxy,
xiv) -C₁₋₆alkyl,
xv) -C₁₋₆alkyl-N(R¹¹)-C(=O)-R¹²,
xvi) -CH₂-O-(CH₂)₀₋₁-aryl substituted by R¹⁰,
xvii) -CH₂-O-(CH₂)₀₋₁-aryl,
xviii) -O-(CH₂)₀₋₁-aryl,
xix) -halogen,
xx) -halogen-C₁₋₆alkyl,
xxi) -hydroxy-C₁₋₆alkyl
xxii) -hydroxy-C₁₋₆alkyl-aryl,
xxiii) -N(R⁵,R⁶),
xxiv) -NH-C(=O)C₁₋₆alkyl,
xxv) -NH-C(=O)OC₁₋₆alkyl, and
xxvi) =O;
- R⁵: is each independently selected from H, C₁₋₆alkyl and phenyl,
- R⁶: is each independently selected from H and C₁₋₆alkyl,
- R⁷: is each independently selected from H and C₁₋₆alkyl,
- R⁸: is each independently selected from H and C₁₋₆alkyl,
- or R⁷ and R⁸: form together with the nitrogen they are attached to a heterocycloalkyl,
- R⁹: is each independently selected from the group consisting of
i) C₁₋₆alkoxy,
ii) C₁₋₆alkyl
iii) halogen,
iv) halogen-C₁₋₆alkyl,
v) heteroaryl, and
vi) heteroaryl substituted by C₁₋₆alkyl or C₁₋₆alkoxy,
- R¹⁰: is each independently selected from the group consisting of
i) C₁₋₆alkoxy,
ii) C₁₋₆alkyl
iii) C₁₋₆alkyl-C₁₋₆alkoxy,
iv) halogen, and
v) halogen-C₁₋₆alkyl;
- R¹¹: is each independently selected from H and C₁₋₆alkyl,
- R¹²: is each independently selected from C₁₋₆alkyl and C₃₋₇cycloalkyl,
and their use as a therapeutically active substance.

The present compounds are useful for the therapeutic and/or prophylactic treatment of cancer.

The compounds of present invention can further be used as part of bifunctional compounds that comprise the compounds of present invention as E3 Ubiquitin Ligase moiety that is linked to a moiety that binds to a target protein where the target protein is proximate to the ubiquitin ligase to effect degradation of said protein.

### Detailed Description of the Invention

Described herein a compound of formula I and their pharmaceutically acceptable salts thereof, the preparation of the above mentioned compounds, medicaments containing them and their manufacture as well as the use of the above mentioned compounds in the therapeutic and/or prophylactic treatment of cancer.

The following definitions of the general terms used in the present description apply irrespectively of whether the terms in question appear alone or in combination with other groups.

Unless otherwise stated, the following terms used in this Application, including the specification and claims, have the definitions given below. It must be noted that, as used in the specification and the appended claims, the singular forms "a", "an," and "the" include plural referents unless the context clearly dictates otherwise.

The term "C₁₋₆-alkyl", alone or in combination with other groups, stands for a hydrocarbon radical which may be linear or branched, with single or multiple branching, wherein the alkyl group in general comprises 1 to 6 carbon atoms, for example, methyl (Me), ethyl (Et), propyl, isopropyl (i-propyl), n-butyl, i-butyl (isobutyl), 2-butyl (sec-butyl), t-butyl (tert-butyl), isopentyl, 2-ethyl-propyl (2-methyl-propyl), 1,2-dimethyl-propyl and the like. A specific group is methyl.

The term "halogen-C₁₋₆-alkyl", alone or in combination with other groups, refers to C₁₋₆-alkyl as defined herein, which is substituted by one or multiple halogen, particularly 1-5 halogen, more particularly 1-3 halogen. Particular halogen is fluoro. Particular "halogen-C₁₋₆-alkyl" is fluoro-C₁₋₆-alkyl and a particular "halogen-C₁₋₃-alkyl" is fluoro-C₁₋₃-alkyl. Examples are trifluoromethyl, difluoromethyl, fluoromethyl and the like.

The term "hydroxy-C₁₋₆-alkyl", alone or in combination with other groups, refers to C₁₋₆-alkyl as defined herein, which is substituted by one or multiple hydroxy, particularly by 1 hydroxy. Examples are -CH₂OH, -CH₂CH₂OH and the like.

The term "cycloalkyl" denotes a monovalent saturated monocyclic or bicyclic hydrocarbon group of 3 to 10 ring carbon atoms, particularly a monovalent saturated monocyclic hydrocarbon group of 3 to 8 ring carbon atoms. Bicyclic means consisting of two carbocycles having one or more carbon atoms in common, while one carbocycle is saturated, the other one may be aromatic. Particular cycloalkyl groups are monocyclic. Examples for monocyclic cycloalkyl are "C₃₋₇cycloalkyl" such as cyclopropyl, cyclobutanyl, cyclopentyl, cyclohexyl or cycloheptyl. Examples for saturated bicyclic cycloalkyl are bicyclo[2.2.1]heptanyl, or bicyclo[2.2.2]octanyl. Examples for bicyclic cycloalkyl wherein one ring is aromatic are 1H-indenyl or 1,2,3,4-tetrahydronaphthalenyl.

The term "hydroxy", alone or in combination with other groups, refers to OH.

The term "Bz" stands for benzyl.

The term "halogen", alone or in combination with other groups, denotes chloro (Cl), iodo (I), fluoro (F) and bromo (Br). A specific group is F.

The term "heteroaryl" denotes a monovalent heterocyclic mono- or bicyclic ring system of 5 to 12 ring atoms, comprising 1, 2, 3 or 4 heteroatoms selected from N, O and S, the remaining ring atoms being carbon and in which at least one ring is aromatic. Examples of heteroaryl moieties include pyrrolyl, furanyl, thienyl, imidazolyl, oxazolyl, thiazolyl, triazolyl, oxadiazolyl, thiadiazolyl, tetrazolyl, pyridinyl, pyrazinyl, pyrazolyl, pyridazinyl, pyrimidinyl, triazinyl, azepinyl, diazepinyl, isoxazolyl, benzofuranyl, isothiazolyl, benzothienyl, indolinyl, indolyl, isoindolyl, isobenzofuranyl, benzimidazolyl, benzoxazolyl, benzoisoxazolyl, benzothiazolyl, benzoisothiazolyl, benzooxadiazolyl, benzothiadiazolyl, benzotriazolyl, purinyl, quinolinyl, isoquinolinyl, quinazolinyl, or quinoxalinyl. Specific examples include benzimidazolyl, pyridinyl, thiazolyl, indolinyl, 1,2,3,4-tetrahydroquinolinyl, 3,4-dihydroquinolinyl, benzofuranyl, furanyl, imidazolyl, isoindolyl, and quinolinyl.

The term "heterocycloalkyl" denotes a monovalent saturated or partly unsaturated mono- or bicyclic ring system of 4 to 9 ring atoms, comprising 1, 2, or 3 ring heteroatoms selected from N, O and S, the remaining ring atoms being carbon. Examples for monocyclic saturated heterocycloalkyl are azetidinyl, pyrrolidinyl, tetrahydrofuranyl, tetrahydrothienyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, or oxazepanyl. Examples for bicyclic saturated heterocycloalkyl are 8-aza-bicyclo[3.2.1]octyl, quinuclidinyl, 8-oxa-3-aza-bicyclo[3.2.1]octyl, 9-aza-bicyclo[3.3.1]nonyl, 3-oxa-9-aza-bicyclo[3.3.1]nonyl, or 3-thia-9-aza-bicyclo[3.3.1]nonyl. Examples for partly unsaturated heterocycloalkyl are dihydrofuryl, imidazolinyl, dihydro-oxazolyl, tetrahydro-pyridinyl, or dihydropyranyl. Specific examples include piperazinyl, piperidinyl, pyrrolidinyl and 3,8-diazabicyclo[3.2.1]octanyl.

The term "C₁₋₆-alkoxy", alone or in combination with other groups, stands for an -O-C₁₋₆-alkyl radical which may be linear or branched, with single or multiple branching, wherein the alkyl group in general comprises 1 to 6 carbon atoms, for example, methoxy (OMe, MeO), ethoxy (OEt), propoxy, isopropoxy (i-propoxy), n-butoxy, i-butoxy (iso-butoxy), 2-butoxy (sec-butoxy), t-butoxy (*tert*-butoxy), isopentyloxy (i-pentyloxy) and the like. Particular "C₁₋₆-alkoxy" are groups with 1 to 4 carbon atoms. A specific group is methoxy.

The term "aryl" denotes a monovalent aromatic carbocyclic mono- or bicyclic ring system comprising 6 to 10 carbon ring atoms. Examples of aryl moieties include phenyl (Ph), Benzyl (Bz) and naphthyl. Specific "aryl" is phenyl.

Terms like "a-b-x substituted by R" means that the "x" portion of the moiety is substituted by R.

The term "pharmaceutically acceptable" denotes an attribute of a material which is useful in preparing a pharmaceutical composition that is generally safe, non-toxic, and neither biologically nor otherwise undesirable and is acceptable for veterinary as well as human pharmaceutical use.

The term "a pharmaceutically acceptable salt" refers to a salt that is suitable for use in contact with the tissues of humans and animals. Examples of suitable salts with inorganic and organic acids are, but are not limited to acetic acid, citric acid, formic acid, fumaric acid, hydrochloric acid, lactic acid, maleic acid, malic acid, methane-sulfonic acid, nitric acid, phosphoric acid, p-toluenesulphonic acid, succinic acid, sulfuric acid (sulphuric acid), tartaric acid, trifluoroacetic acid and the like. Particular acids are formic acid, trifluoroacetic acid and hydrochloric acid. Specific acids are hydrochloric acid, trifluoroacetic acid and fumaric acid.

The terms "pharmaceutically acceptable auxiliary substance" refer to carriers and auxiliary substances such as diluents or excipients that are compatible with the other ingredients of the formulation.

The term "pharmaceutical composition" encompasses a product comprising specified ingredients in pre-determined amounts or proportions, as well as any product that results, directly or indirectly, from combining specified ingredients in specified amounts. Particularly it encompasses a product comprising one or more active ingredients, and an optional carrier comprising inert ingredients, as well as any product that results, directly or indirectly, from combination, complexation or aggregation of any two or more of the ingredients, or from dissociation of one or more of the ingredients, or from other types of reactions or interactions of one or more of the ingredients.

"Therapeutically effective amount" means an amount of a compound that, when administered to a subject for treating a disease state, is sufficient to effect such treatment for the disease state. The "therapeutically effective amount" will vary depending on the compound, disease state being treated, the severity or the disease treated, the age and relative health of the subject, the route and form of administration, the judgment of the attending medical or veterinary practitioner, and other factors.

The terms "treating", "contacting" and "reacting" when referring to a chemical reaction means adding or mixing two or more reagents under appropriate conditions to produce the indicated and/or the desired product. It should be appreciated that the reaction which produces the indicated and/or the desired product may not necessarily result directly from the combination of two reagents which were initially added, i.e., there may be one or more intermediates which are produced in the mixture which ultimately leads to the formation of the indicated and/or the desired product.

The term "aromatic" denotes the conventional idea of aromaticity as defined in the literature, in particular in IUPAC - Compendium of Chemical Terminology, 2nd Edition, A. D. McNaught & A. Wilkinson (Eds). Blackwell Scientific Publications, Oxford (1997).

The term "pharmaceutically acceptable excipient" denotes any ingredient having no therapeutic activity and being non-toxic such as disintegrators, binders, fillers, solvents, buffers, tonicity agents, stabilizers, antioxidants, surfactants or lubricants used in formulating pharmaceutical products.

Whenever a chiral carbon is present in a chemical structure, it is intended that all stereoisomers associated with that chiral carbon are encompassed by the structure as pure stereoisomers as well as mixtures thereof.

The invention also provides pharmaceutical compositions, methods of using, and methods of preparing the aforementioned compounds.

All separate embodiments may be combined.

Furthermore, the invention includes all optical isomers, i.e. diastereoisomers, diastereomeric mixtures, racemic mixtures, all their corresponding enantiomers and/or tautomers as well as their solvates of the compounds of formula I.

The compounds of formula I may contain one or more asymmetric centers and can therefore occur as racemates, racemic mixtures, single enantiomers, diastereomeric mixtures and individual diastereomers. Additional asymmetric centers may be present depending upon the nature of the various substituents on the molecule. Each such asymmetric center will independently produce two optical isomers and it is intended that all of the possible optical isomers and diastereomers in mixtures and as pure or partially purified compounds are included within this invention. The present invention is meant to encompass all such isomeric forms of these compounds. The independent syntheses of these diastereomers or their chromatographic separations may be achieved as known in the art by appropriate modification of the methodology disclosed herein. Their absolute stereochemistry may be determined by the x-ray crystallography of crystalline products or crystalline intermediates which are derivatized, if necessary, with a reagent containing an asymmetric center of known absolute configuration. If desired, racemic mixtures of the compounds may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography.

In the embodiments, where optically pure enantiomers are provided, optically pure enantiomer means that the compound contains > 90% of the desired isomer by weight, particularly > 95% of the desired isomer by weight, or more particularly > 99% of the desired isomer by weight, said weight percent based upon the total weight of the isomer(s) of the compound. Chirally pure or chirally enriched compounds may be prepared by chirally selective synthesis or by separation of enantiomers. The separation of enantiomers may be carried out on the final product or alternatively on a suitable intermediate.

The compounds of formula I may be prepared in accordance with the schemes described in the examples. The starting material is commercially available or may be prepared in accordance with known methods.

The preparation of compounds of formula I is further described in more detail in the schemes below.

The preparation of compounds of formula I may be carried out in sequential or convergent synthetic routes. Syntheses of the compounds of the invention are shown in the following schemes and in the description of the specific examples. The skills required for carrying out the reaction and purification of the resulting products are known to those skilled in the art. The substituents and indices used in the following description of the processes have the significance given herein before unless indicated to the contrary.

In more detail, the compounds of formula I can be manufactured by the methods given below, by the methods given in the examples or by analogous methods. Appropriate reaction conditions for the individual reaction steps are known to a person skilled in the art. The reaction sequence is not limited to the one displayed in the schemes, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art.

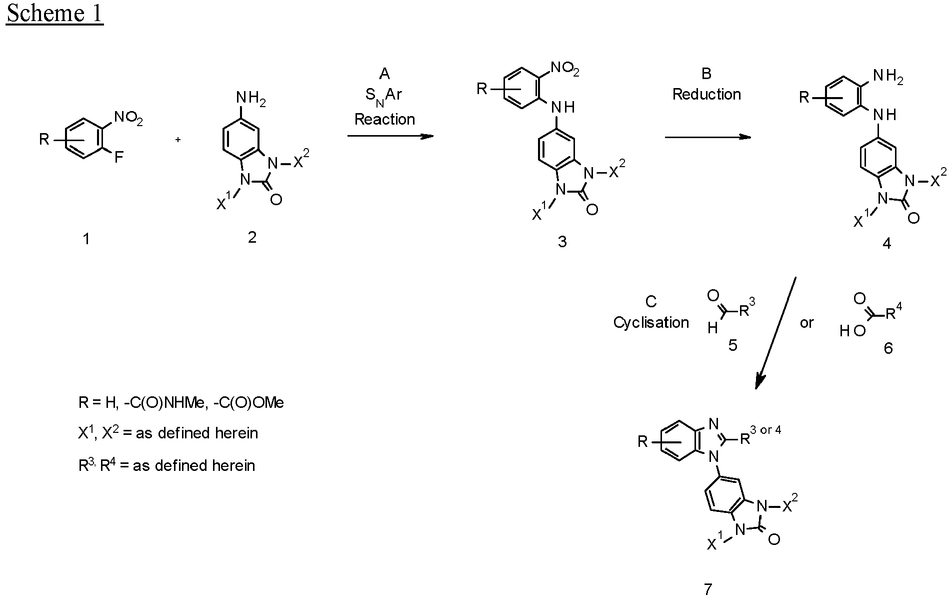

***Step A**:* Nitro-amino-arene compound **3** can be obtained by an aromatic nucleophilic substitution (S_{N}Ar) reaction between a 1,2-fluoro-nitro-arene **1** and an aromatic amine compound **2**.

Examples of suitable 1,2-fluoro-nitro-arenes **1** include, but are not limited to, 1-fluoro-2-nitrobenzene⁴⁵, 4-fluoro-*N*-methyl-3-nitrobenzamide⁴⁶, 3-fluoro-*N*-methyl-4-nitrobenzamide⁴⁷, methyl 4-fluoro-3-nitrobenzoate⁴⁸, methyl 3-fluoro-4-nitrobenzoate⁴⁹.

Examples of suitable aromatic amine compounds **2** include, but are not limited to, 5-amino-1*H*-benzo[*d*]imidazol-2(3*H*)-one⁵⁰, 5-amino-1-methyl-1,3-dihydro-2*H*-benzo[d]imidazol-2-one⁵¹, 6-amino-1-methyl-1,3-dihydro-2*H*-benzo[d]imidazol-2-one ⁵² , 5-amino-1,3-dimethyl-1*H-*benzo[d]imidazol-2(3*H*)-one⁵³.

The S_{N}Ar reaction is carried out in the presence of an organic base such as *N,N-*diisopropylethylamine, triethylamine or *N*-methylmorpholine in a polar aprotic organic solvent such as *N*-methyl-2-pyrrolidinone or *N,N-*dimethylformamide at elevated temperatures. Preferred conditions are *N,N-*diisopropylethylamine in *N*-methyl-2-pyrrolidinone at 120 °C overnight.

***Step B:*** Reduction of nitro-amino-arene compound **3** to diamino-arene **4** can be effected by hydrogenation with hydrogen under normal or elevated pressure in the presence of a catalyst such as PtO₂, Pd-C or Raney nickel in polar solvents such as MeOH, EtOH, dioxane, THF, or mixtures thereof.

Preferred conditions are 1 atm of hydrogen in the presence of 10% palladium on charcoal in a mixture of methanol and THF at room temperature overnight.

***Step C*:** Benzimidazole **7** can be obtained by cyclisation of diamino-arene **4** with an aldehyde **5** in the presence of sodium metabisulfite. The cyclisation reaction is carried out in a polar aprotic organic solvent such as *N*-methyl-2-pyrrolidinone or *N*,*N*-dimethylformamide at elevated temperatures. Preferred conditions are *N*-methyl-2-pyrrolidinone at 120 °C for 3-18 hours.

Benzimidazole **7** can also be obtained by cyclisation of diamino-arene **4** with a carboxylic acid **6** in the presence of another acid. The cyclisation reaction is carried out in an aqueous solution of a strong acid such as HCl or H₂SO₄. Preferred conditions are 6 M aq. HCl at 120 °C for 12-48 hours. 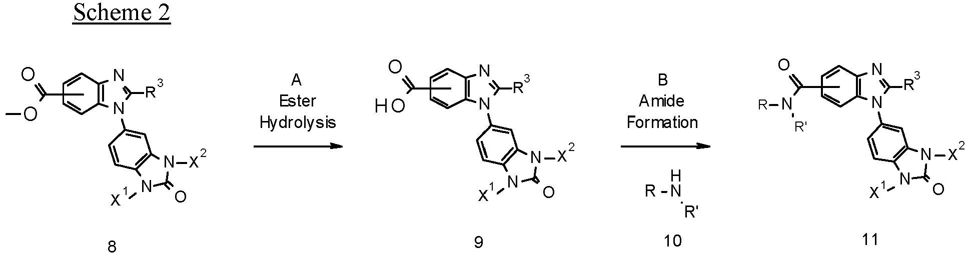
R, R' = H, Me independently of one another
X¹,X² = as defined herein
R³ = as defined herein

***Step A*:** Ester compound **8** can be hydrolysed to carboxylic acid **9** by treatment with an aqueous base such as lithium hydroxide, sodium hydroxide or potassium hydroxide. The hydrolysis reaction is carried out in a mixture of water and a polar ethereal solvent such as dioxane, THF, DME or TBME at room temperature or at elevated temperatures for 2-18 hours. Preferred conditions are 1 M aq. LiOH solution in 1,4-dioxane at room temperature for 3 h.

***Step B***: Amide bond formation can be accomplished by a coupling reaction between carboxylic acid **9** and a primary or secondary amine **10** in the presence of a coupling reagent such as DMTMM, DCC, EDC, TBTU or HATU in the presence of an organic base such as triethylamine, *N,N-*diisopropylethylamine or *N*-methylmorpholine in halogenated solvents such as dichloromethane or 1,2-dichloroethane or ethereal solvents such as diethyl ether, dioxane, THF, DME or TBME or polar non-protic organic solvent such as *N*,*N*-dimethylformamide at room temperature or at elevated temperatures for 2-18 hours.

Preferred conditions are HATU with *N*,*N*-diisopropylethylamine in a mixture of THF and *N*,*N*-dimethylformamide at room temperature for 3 hours.

### Isolation and purification of the compounds

Isolation and purification of the compounds and intermediates described herein can be effected, if desired, by any suitable separation or purification procedure such as, for example, filtration, extraction, crystallization, column chromatography, thin-layer chromatography, thick-layer chromatography, preparative low or high-pressure liquid chromatography or a combination of these procedures. Specific illustrations of suitable separation and isolation procedures can be had by reference to the preparations and examples herein below. However, other equivalent separation or isolation procedures could, of course, also be used. Racemic mixtures of chiral compounds of formula I can be separated using chiral HPLC. Racemic mixtures of chiral synthetic intermediates may also be separated using chiral HPLC.

In cases where the compounds of formula I are basic they may be converted to a corresponding acid addition salt. The conversion is accomplished by treatment with at least a stoichiometric amount of an appropriate acid, such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid and the like. Typically, the free base is dissolved in an inert organic solvent such as diethyl ether, ethyl acetate, chloroform, ethanol or methanol and the like, and the acid added in a similar solvent. The temperature is maintained between 0 °C and 50 °C. The resulting salt precipitates spontaneously or may be brought out of solution with a less polar solvent.

Insofar as their preparation is not described in the examples, the compounds of formula I as well as all intermediate products can be prepared according to analogous methods or according to the methods set forth herein. Starting materials are commercially available, known in the art or can be prepared by methods known in the art or in analogy thereto.

It will be appreciated that the compounds of general formula I may be derivatised at functional groups to provide derivatives which are capable of conversion back to the parent compound *in vivo.*

### Pharmacological Tests

The compounds of formula I and their pharmaceutically acceptable salts possess valuable pharmacological properties. The compounds were investigated in accordance with the test given hereinafter.

### Dual Fluorescent Reporter Assay

In order to measure BRD4 protein abundance in a mammalian cell system at medium throughput, a dual fluorescent reporter system was developed based on a principle described in ¹. Transient expression vectors were designed that contain the BRD4 coding sequence (NM_058243.2) fused to a fluorescent tag. Vectors were synthesized at ATUM (Newark, CA, USA) using the pD2610 CMV backbone and were built up as follows: c-terminal version BRD4_eGFP - IRES - FresnoRFP_NLS, n-terminal version eGFP_BRD4 - IRES - FresnoRFP_NLS, empty vector control eGFP - IRES - FresnoRFP_NLS. The c-terminal version was used for the reporter assays, as it presented with the best assay window. HEK293A cells (Invitrogen, Cat. No. R705-07) were cultured in Dulbecco's Modified Eagle Medium (DMEM), 10% fetal calf serum, 2mM L-Glutamine, 1% Penicillin/Streptomycin. Transfections of the plasmids were performed with Lipofectamine 2000 according to the manufacturer's protocol (Invitrogen, Carlsbad, CA, USA). 40 hours after transfection, cells were seeded at a density of 40'000 / 100ul / 96well flat-bottom and 8 hours later treated with compounds (stocks 10mM in DMSO) at a 10-point dilution ranging from 0 - 25 µM. After 16 hours of treatment, cells were washed with PBS, resuspended in Accumax solution⁵⁷ and analyzed by flow-cytometry (CytoFlex S, BeckmanCoulter). Single cells were gated based on their forward and side-scatter profiles and pulse-width was used to exclude doublets. A minimum of 20'000 cells was acquired per sample. Analysis was performed with the program Flow Jo V10.1 on BRD4-eGFP low/medium cells (< 10⁶ FL1-A Mean Fluorescence Intensity (MFI)). A factor was derived to normalize BRD4-eGFP values to the RFP protein abundance control (20 x FL1A-GFP / FL11A-RFP), then Median and Mode values were calculated and used for comparisons between treatment conditions.

### Capillary-based Immunoassays to measure endogenous BRD4 levels

The biological activity of selected compounds (cut-off > 20% reduction in BRD4-eGFP levels) was confirmed in an additional assay which allowed the quantification of endogenous BRD4 levels. To this end, HEK293A cells (origin and culture conditions see above) were seeded at 400'000 / 300ul / 48 well and were treated 6 hours later with compound concentrations as indicated for. 16 hours after the treatment, the cells were washed with PBS and lysed in 50ul of UREA lysis buffer (10 mM Tris-HCl pH 8, 2% CHAPS, 7M UREA, 0,4% DTT), supplemented with 1 × protease inhibitor cocktail (Complete Mini, Roche) and 1 x phosphatase inhibitor cocktail (PhosSTOP, Sigma-Aldrich). Samples were then analyzed by Peggy Sue or WES capillary-based immunoassay systems according to the manufacturer's protocol (Protein Simple / Bio-Techne, San Jose, California, 95134 USA). Antibodies used were anti- BRD4 (Cell signaling, CST 13440 1:50) and anti-Vinculin (Sigma, V9131, 1:4000). To quantify BRD4 protein levels, the peak signal areas were normalized to the vinculin loading control and to the DMSO condition.

Further, please see Yen *et al.*⁵⁸.

### Fluorescence Direct Binding Protocol

### Principle

Determination of the affinities of compounds to protein containing one or more tryptophan is measurable by monitoring the fluorescence emission in direct mode. The measurements depending on the protein available amounts are performed either manually in a cuvette on ISS-PC1 photon counting spectrofluorometer or automatically in well plates on a fluorescence plate reader device. Fluorescence titrations are performed at 20°C in the chosen binding assay buffer by using a defined constant protein concentration against ligand concentration variations. Small aliquots of known ligand concentration solubilized in DMSO were added and the fluorescence, excited at 280 nm, was recorded at 340 nm. The fluorescence intensity was corrected for protein dilution and for the filter effect (Birdsall *et al*.⁵⁹)*.* The corrected fluorescence intensity was plotted against the ligand concentration and fitted using a four-parameter sigmoidal function, from which the equilibrium dissociation constant Kd was computed using the law of mass action assuming a 1:1 protein-ligand complex (Eftink, 1997⁶⁰).

### Process

1) Optimization of measurement parameters to minimize protein consumption and to minimize the dilution effect and the DMSO content
2) Titration measurements of the protein against ligand by at least 12 titration steps to obtain a good s-curve fit
3) Repeat the same titration measurements with the ligand alone to enable correction
4) Check the stability of the protein once by titration against DMSO alone
5) Determination of the molar extinction coefficients of the ligand at 280 and 340 nm with help of an UV- spectrophotometer
6) Use Excel template for the correction of the measured raw data
7) Use GraphPad Prism software for the quadratic binding fit and the KD evaluation.

### Experimental Details

**Table 1: Protein - buffers, Reference compound: thalidomide, Contergan, Softenon**

| **Protein Batch #** | **Cereblon_17_13** |
|---|---|
| Construct name | hCereblon(M1-L442)_hDDB1(M1-H1140) |
| Concentration | 2.54 mg/ml |
| MW | 180180 Da |
| Molar extinction coefficient | ε₂₈₀ = 165045 M⁻¹.cm⁻¹ |
| Storage buffer | 20 mM MES pH 6.5 200 mM NaCl 1 mM TCEP |
| Assay buffer | 50 mM Hepes 7.4 200 mM NaCl |

**Table 2: Settings**

| | |
|---|---|
| Device | ISS-PC1 |
| Excitation wavelength [nm] | 280 |
| Emission wavelength [nm] | 340 |
| Cuvette | Hellma 115F-QS |
| Volume [µL] | 500 |

### Protein preparation:

**Table 3: Protein preparation**

| Volume Protein [µL] | Volume buffer [µL] | Protein concentration [M] |
|---|---|---|
| 1.8 @ 2.54 mg/ml | 498.2 | 5.0 E-8 |

**Table 4: Titration steps**

| **C Lig [ M ]** | **C Aliquot [ M ]** | **V Aliquot [ µL ]** | **C Prot[ M ]** | **Dilution factor** |
|---|---|---|---|---|
| 1E-10 | 1.0E-07 | 0.5 | 4.995E-08 | 1.001 |
| 1.1E-09 | 1.0E-06 | 0.5 | 4.990E-08 | 1.002 |
| 3.1E-09 | 1.0E-06 | 1 | 4.980E-08 | 1.004 |
| 5.1E-09 | 1.0E-06 | 1 | 4.970E-08 | 1.006 |
| 1.51E-08 | 1.0E-05 | 0.5 | 4.965E-08 | 1.007 |
| 2.51E-08 | 1.0E-05 | 0.5 | 4.960E-08 | 1.008 |
| 4.51E-08 | 1.0E-05 | 1 | 4.950E-08 | 1.01 |
| 6.51E-08 | 1.0E-05 | 1 | 4.941E-08 | 1.012 |
| 1.651E-07 | 1.0E-04 | 0.5 | 4.936E-08 | 1.013 |
| 3.651E-07 | 1.0E-04 | 1 | 4.926E-08 | 1.015 |
| 5.651E-07 | 1.0E-04 | 1 | 4.916E-08 | 1.017 |
| 7.651E-07 | 1.0E-04 | 1 | 4.907E-08 | 1.019 |
| 9.651E-07 | 1.0E-04 | 1 | 4.897E-08 | 1.021 |
| 1.9651E-06 | 1.0E-03 | 0.5 | 4.892E-08 | 1.022 |
| 2.9651E-06 | 1.0E-03 | 0.5 | 4.888E-08 | 1.023 |
| 1.29651E-05 | 1.0E-02 | 0.5 | 4.883E-08 | 1.024 |
| 2.29651E-05 | 1.0E-02 | 0.5 | 4.878E-08 | 1.025 |
| 4.29651E-05 | 1.0E-02 | 1 | 4.869E-08 | 1.027 |
| 6.29651E-05 | 1.0E-02 | 1 | 4.859E-08 | 1.029 |
| 8.29651E-05 | 1.0E-02 | 1 | 4.850E-08 | 1.031 |

Wherein examples 1, 6, 9, 12, 18-27, 29, 32, 33, 36-44, 49-56, 58, 59, 62, 63, 67-78, 83, 86-101, 104-106, 109-113, 116-130, 132-156, A-D are provided for reference.

**Table 5: affinities of examples to protein**

| **Example** | **Name** | **Fluorescence h-Cereblon_DDB1 Mean K_{d_}EQ (µM)** |
|---|---|---|
| 1 | 3-amino-*N*-methyl-4-[(2-oxo-1,3-dihydrobenzimidazol-5-yl)amino]benzamide | 0.587 |
| 2 | *N*-methyl-2-[2-(3-methylimidazol-4-yl)-4-quinolyl]-1-(2-oxo-1,3-dihydrobenzimidazol-5-yl)benzimidazole-5-carboxamide | 0.028 |
| 3 | *N*-methyl-1-(2-oxo-1,3-dihydrobenzimidazol-5-yl)-2-(4-quinolyl)benzimidazole-5-carboxamide | 0.039 |
| 4 | 2-ethyl-*N*-methyl-1-(2-oxo-1,3-dihydrobenzimidazol-5-yl)benzimidazole-5-carboxamide | 0.036 |
| 5 | *N*,2-dimethyl-1-(2-oxo-1,3-dihydrobenzimidazol-5-yl)benzimidazole-5-carboxamide | 0.180 |
| 6 | 5-(2-aminoanilino)-1,3-dihydrobenzimidazol-2-one | 0.054 |
| 7 | 5-[2-(4-quinolyl)benzimidazol-1-yl]-1,3-dihydrobenzimidazol-2-one | 0.027 |
| 8 | 5-[2-[2-(3-methylimidazol-4-yl)-4-quinolyl]benzimidazol-1-yl]-1,3-dihydrobenzimidazol-2-one | 0.006 |
| 9 | methyl 3-amino-4-[(2-oxo-1,3-dihydrobenzimidazol-5-yl)amino]benzoate | 0.029 |
| 10 | *N*-methyl-1-(2-oxo-1,3-dihydrobenzimidazol-5-yl)-2-phenyl-benzimidazole-5-carboxamide | 0.088 |
| 11 | *N*-methyl-1-(2-oxo-1,3-dihydrobenzimidazol-5-yl)-2-(4-pyridyl)benzimidazole-5-carboxamide | 0.008 |
| 12 | 5-[2-(4-quinolyl)imidazol-1-yl]-1,3-dihydrobenzimidazol-2-one | 0.001 |
| 13 | *N*-methyl-1-(2-oxo-1,3-dihydrobenzimidazol-5-yl)-2-(trifluoromethyl)benzimidazole-5-carboxamide | 0.007 |
| 14 | *N*-methyl-1-(2-oxo-1,3-dihydrobenzimidazol-5-yl)-2-propyl-benzimidazole-5-carboxamide | 0.321 |
| 15 | 2-(1*H*-imidazol-5-yl)-*N*-methyl-1-(2-oxo-1,3-dihydrobenzimidazol-5-yl)benzimidazole-5-carboxamide | 0.049 |
| 16 | 2-isopropyl-*N*-methyl-1-(2-oxo-1,3-dihydrobenzimidazol-5-yl)benzimidazole-5-carboxamide | 0.011 |
| 17 | 1-(1,3-dimethyl-2-oxo-benzimidazol-5-yl)-*N*-methyl-2-[2-(3-methylimidazol-4-yl)-4-quinolyl]benzimidazole-5-carboxamide | 0.042 |
| 18 | *N*,*N*-dimethyl-2-[2-(3-methylimidazol-4-yl)-4-quinolyl]-1-(2-oxo-1,3-dihydrobenzimidazol-5-yl)benzimidazole-5-carboxamide | 0.016 |
| 19 | 5-(3-pyridyl)-1,3-dihydrobenzimidazol-2-one | 0.007 |
| 20 | 5-(2-chloro-4-pyridyl)-1,3-dihydrobenzimidazol-2-one | 0.068 |
| 21 | 5-(2-methylthiazol-5-yl)-1,3-dihydrobenzimidazol-2-one | 0.005 |
| 22 | 5-(4-acetylpiperazine-1-carbonyl)-1,3-dihydrobenzimidazol-2-one | 0.103 |
| 23 | *N*-(1-acetyl-4-piperidyl)-2-oxo-1,3-dihydrobenzimidazole-5-carboxamide | 0.003 |
| 24 | 5-(indoline-1-carbonyl)-1,3-dihydrobenzimidazol-2-one | 0.001 |
| 25 | *N*-(1,3-dimethyl-2-oxo-benzimidazol-5-yl)furan-2-carboxamide | 0.111 |
| 26 | 5-(3-phenyl-propyl)-1,3-dihydro-imidazo[4,5-b]pyridin-2-one | 0.041 |
| 27 | 1-methyl-2-oxo-*N*-phenyl-3*H*-imidazo[4,5-b]pyridine-6-carboxamide | 0.001 |
| 28 | 2-(3-methoxybenzyl)-*N*-methyl-2'-oxo-2',3'-dihydro-1'*H-*[1,5'-bibenzo[d]imidazole]-5-carboxamide | 0.012 |
| 29 | *N-*(1-(2-oxo-2,3-dihydro-1*H*-benzo[d]imidazole-5-carbonyl)piperidin-4-yl)acetamide | 0.020 |
| 30 | 2-isopropyl-*N-*methyl-2'-oxo-2',3'-dihydro-1*'H-*[1*,*5'-bibenzo[d]imidazole]-6-carboxamide | 0.001 |
| 31 | 1',3'-dimethyl-2-(2-(1-methyl-1*H*-imidazol-5-yl)quinolin-4-yl)-1',3'-dihydro-2'*H*-[1,5'-bibenzo[d]imidazol]-2'-one | 0.007 |
| 32 | 5-(2-isopropyl-1*H*-imidazol-1-yl)-1,3-dihydro-2*H-*benzo[d]imidazol-2-one | 0.006 |
| 33 | 5-(2-oxopyrrolidin-1-yl)-1,3-dihydro-2*H-*benzo[d]imidazol-2-one | 0.024 |
| 34 | 2-isopropyl-1'-methyl-1',3'-dihydro-2'*H*-[1,5'-bibenzo[d]imidazol]-2'-one | 0.019 |
| 35 | 2-isopropyl-3'-methyl-1',3'-dihydro-2'*H*-[1,5'-bibenzo[d]imidazol]-2'-one | 0.024 |
| 36 | 6-(4-fluorophenyl)-1,3-dihydroimidazo[4,5-c]pyridin-2-one | 0.077 |
| 37 | 5-(2,5-difluoro-phenyl)-1,3-dihydro-benzoimidazol-2-one | 0.010 |
| 38 | 5-(2,4-difluoro-phenyl)-1,3-dihydro-benzoimidazol-2-one | 0.082 |
| 39 | 5-[(4-methoxyphenyl)methyl]-1,3-dihydrobenzimidazol-2-one | 0.006 |
| 40 | 5-[4-(2-pyridyl)piperazin-1-yl]-1,3-dihydroimidazo[4,5-b]pyridin-2-one | 0.007 |
| 41 | 5-(4-pyrrol-1-yl-phenyl)-1,3-dihydro-benzoimidazol-2-one | 0.018 |
| 42 | 5-quinolin-5-yl-1,3-dihydro-benzoimidazol-2-one | 0.336 |
| 43 | ethyl 4-((2-oxo-2,3-dihydro-1H-benzo[d]imidazole)-5-sulfonamido)benzoate | 0.100 |
| 44 | 1-methyl-5-phenyl-3H-imidazo[4, 5-b]pyridin-2-one | < 0.001 |
| 45 | 2-(2-methoxybenzyl)-*N*-methyl-2'-oxo-2',3'-dihydro-1'*H-*[1,5'-bibenzo[d]imidazole]-5-carboxamide | 0.022 |
| 46 | 2-(*tert-*butyl)-*N-*methyl-2'-oxo-2',3'-dihydro-1*'H*-[1,5*'-*bibenzo[d]imidazole]-5-carboxamide | 0.010 |
| 47 | methyl 2-isopropyl-2'-oxo-2',3'-dihydro-1'*H*-[1,5'-bibenzo[d]imidazole]-4-carboxylate | 0.020 |
| 48 | methyl 2-isopropyl-2'-oxo-2',3'-dihydro-1'*H*-[1,5'-bibenzo[d]imidazole]-6-carboxylate | 0.011 |
| 49 | 1,3-dimethyl-5-((2-nitrophenyl)amino)-1,3-dihydro-2*H-*benzo[d]imidazol-2-one | 0.023 |
| 50 | 5-((2-aminophenyl)amino)-1,3-dimethyl-1,3-dihydro-2*H-*benzo[d]imidazol-2-one | 0.011 |
| 51 | 2-oxo-*N*-(4-piperidyl)-1,3-dihydrobenzimidazole-5-carboxamide | 0.138 |
| 52 | 6-((2-aminophenyl)amino)-1-methyl-1,3-dihydro-2*H-*benzo[d]imidazol-2-one | 0.006 |
| 53 | 5-((2-aminophenyl)amino)-1-methyl-1,3-dihydro-2*H-*benzo[d]imidazol-2-one | 0.049 |
| 54 | methyl 1-(2-oxo-2,3-dihydro-1*H*-benzo[d]imidazole-5-carbonyl)indoline-4-carboxylate | 0.025 |
| 55 | 6-(indoline-1-carbonyl)-1,3-dihydro-2*H*-imidazo[4,5-b]pyridin-2-one | < 0.001 |
| 56 | *N*-(1-acetylpiperidin-4-yl)-2-oxo-2,3-dihydro-1*H-*imidazo[4,5-b]pyridine-6-carboxamide | 0.002 |
| 57 | 2-(3-(methoxymethyl)phenyl)-*N*-methyl-2'-oxo-2',3'-dihydro-1'*H*-[1,5'-bibenzo[d]imidazole]-5-carboxamide | 0.007 |
| 58 | 5-(piperazin-1-yl)-1,3-dihydro-2*H*-benzo[d]imidazol-2-one | 0.012 |
| 59 | 5-[*rac*-(1*S*,5*R*)-3,8-diazabicyclo[3.2.1]octan-3-yl]-1,3-dihydrobenzimidazol-2-one | 0.006 |
| 60 | 2-isopropyl*-N-*methyl-2'-oxo-2',3'-dihydro-1*'H-*[1,5'-bibenzo[d]imidazole]-4-carboxamide | 0.047 |
| 61 | 5-[2-(hydroxymethyl)benzimidazol-1-yl]-1,3-dihydrobenzimidazol-2-one | 0.029 |
| 62 | 5-(4-acetylpiperazin-1-yl)-1,3-dihydro-2*H-*benzo[d]imidazol-2-one | < 0.001 |
| 63 | 5-[*rac*-(1*S*,5*R*)-8-acetyl-3,8-diazabicyclo[3.2.1]octan-3-yl]-1,3-dihydrobenzimidazol-2-one | 0.079 |
| 64 | *N*-methyl-2'-oxo-2-(tetrahydro-2*H*-pyran-4-yl)-2',3'-dihydro-1'*H*-[1,5'-bibenzo[d]imidazole]-5-carboxamide | 0.045 |
| 65 | *N*-methyl-2-(1-methyl-1*H*-imidazol-4-yl)-2'-oxo-2',3'-dihydro-1'*H*-[1,5'-bibenzo[d]imidazole]-5-carboxamide | 0.009 |
| 66 | *N-*methyl-2'-oxo-2-(1*H*-pyrrol-3*-*yl)-2',3'-dihydro-1'*H-*[1,5'-bibenzo[d]imidazole]-5-carboxamide | 0.051 |
| 67 | 2-oxo-*N*-phenyl-2,3-dihydro-1*H*-imidazo[4,5-b]pyridine-6-carboxamide | 0.005 |
| 68 | *N*-(2-oxo-2,3-dihydro-1*H*-benzo[d]imidazol-5-yl)benzofuran-3-carboxamide | 0.009 |
| 69 | *N*-(2-oxo-2,3-dihydro-1*H*-benzo[d]imidazol-5-yl)-2,3-dihydro-1*H*-indene-1-carboxamide | 0.029 |
| 70 | 2-(4-aminophenyl)-*N*-(2-oxo-2,3-dihydro-1*H-*benzo[d]imidazol-5-yl)acetamide | 0.001 |
| 71 | (*S*)-*N*-(2-oxo-2,3-dihydro-1*H*-benzo[d]imidazol-5-yl)-1,2,3,4-tetrahydronaphthalene-1-carboxamide | 0.016 |
| 72 | (*R*)-*N*-(2-oxo-2,3-dihydro-1*H*-benzo[d]imidazol-5-yl)-1,2,3,4-tetrahydronaphthalene-1-carboxamide | 0.014 |
| 73 | *N*-(2-oxo-2,3-dihydro-1*H*-benzo[d]imidazol-5-yl)benzamide | 0.011 |
| 74 | 4-acetamido-*N*-(2-oxo-2,3-dihydro-1*H*-benzo[d]imidazol-5-yl)benzamide | 0.002 |
| 75 | 3-acetamido-*N*-(2-oxo-2,3-dihydro-1*H*-benzo[d]imidazol-5-yl)benzamide | 0.007 |
| 76 | *N*-methyl-2-oxo-*N*-phenyl-2,3-dihydro-1*H*-imidazo[4,5-b]pyridine-6-carboxamide | 0.041 |
| 77 | 2-oxo-*N*-phenyl-2,3-dihydro-1*H*-benzo[d]imidazole-5-carboxamide | 0.042 |
| 78 | 2-(4-acetamidophenyl)-*N*-(2-oxo-2,3-dihydro-1*H-*benzo[d]imidazol-5-yl)acetamide | 0.023 |
| 79 | 2-isopropyl-*N*-methyl-1-(2-oxo-1,3-dihydroimidazo[4,5-b]pyridin-6-yl)benzimidazole-5-carboxamide | 0.004 |
| 80 | *N*-[2-[1-(2-oxo-1,3-dihydrobenzimidazol-5-yl)benzimidazol-2-yl]ethyl]acetamide | 0.015 |
| 81 | *N*-methyl-3-[1-(2-oxo-1,3-dihydrobenzimidazol-5-yl)benzimidazol-2-yl]propanamide | 0.004 |
| 82 | methyl 1-(2-oxo-1,3-dihydrobenzimidazol-5-yl)benzimidazole-2-carboxylate | 0.003 |
| 83 | 5-(benzimidazol-1-yl)-1,3-dihydrobenzimidazol-2-one | 0.003 |
| 84 | 5-[2-(1-hydroxyethyl)benzimidazol-1-yl]-1,3-dihydrobenzimidazol-2-one | 0.003 |
| 85 | 5-[2-[hydroxy(phenyl)methyl]benzimidazol-1-yl]-1,3-dihydrobenzimidazol-2-one | 0.003 |
| 86 | *N*-(1-acetylpiperidin-4-yl)-*N*-methyl-2-oxo-2,3-dihydro-1*H*-benzo[d]imidazole-5-carboxamide | 0.004 |
| 87 | 1-(2-oxo-2,3-dihydro-1*H*-benzo[d]imidazol-5-yl)-3-(4-(phenylamino)phenyl)urea | 0.027 |
| 88 | 1-(2-oxo-2,3-dihydro-1*H*-benzo[d]imidazol-5-yl)-3-phenylurea | 0.017 |
| 89 | methyl 1-((2-oxo-2,3-dihydro-1*H*-benzo[d]imidazol-5-yl)carbamoyl)indoline-4-carboxylate | 0.025 |
| 90 | 3-acetamido-*N*-(2-oxo-2,3-dihydro-1*H*-benzo[d]imidazol-5-yl)propanamide | 0.019 |
| 91 | 1-acetyl*-N*-(2-oxo-2,3-dihydro-1*H*-benzo[d]imidazol-5-yl)piperidine-4-carboxamide | 0.005 |
| 92 | methyl 1-((2-oxo-2,3-dihydro-1*H*-benzo[d]imidazol-5-yl)carbamoyl)indoline-5-carboxylate | 0.033 |
| 93 | *N*4-methyl-*N*1-(2-oxo-2,3-dihydro-1*H*-benzo[d]imidazol-5-yl)indoline-1,4-dicarboxamide | 0.010 |
| 94 | 1-benzyl-3-(2-oxo-2,3-dihydro-1*H*-benzo[d]imidazol-5-yl)urea | < 0.001 |
| 95 | 6-(1,2,3,4-tetrahydroquinoline-1-carbonyl)-1,3-dihydro-2*H*-imidazo[4,5-b]pyridin-2-one | 0.020 |
| 96 | *N*-methyl-2-oxo-*N*-phenyl-2,3-dihydro-1*H-*benzo[d]imidazole-5-carboxamide | 0.009 |
| 97 | 5-(1,2,3,4-tetrahydroquinoline-1-carbonyl)-1,3-dihydro-2*H*-benzo[d]imidazol-2-one | 0.009 |
| 98 | methyl 1-((2-oxo-2,3-dihydro-1*H*-benzo[d]imidazol-5-yl)carbamoyl)-1,2,3,4-tetrahydroquinoline-5-carboxylate | 0.086 |
| 99 | *N*-(2-oxo-2,3-dihydro-1*H*-benzo[d]imidazol-5-yl)-2-(4-sulfamoylphenyl)acetamide | 0.046 |
| 100 | *N*-(2-oxo-2,3-dihydro-1*H*-benzo[d]imidazol-5-yl)-4-sulfamoylbenzamide | 0.029 |
| 101 | *N*-(4-(3-(2-oxo-2,3-dihydro-1*H*-benzo[d]imidazol-5-yl)ureido)phenyl)acetamide | 0.014 |
| 102 | *N*-methyl-1-(2-oxo-1,3-dihydrobenzimidazol-5-yl)benzimidazole-2-carboxamide | 0.130 |
| 103 | 5-[2-(piperidine-1-carbonyl)benzimidazol-1-yl]-1,3-dihydrobenzimidazol-2-one | 0.035 |
| 104 | 6-(2-(benzyloxy)phenyl)-1,3-dihydro-2*H*-imidazo[4,5-b]pyridin-2-one | 0.014 |
| 105 | *N*-(2-oxo-2,3-dihydro-1*H*-benzo[d]imidazol-5-yl)indoline-1-carboxamide | 0.004 |
| 106 | 2-(4-(N-methylsulfamoyl)phenyl)-*N*-(2-oxo-2,3-dihydro-1*H*-benzo[d]imidazol-5-yl)acetamide | 0.001 |
| 107 | methyl 2-(2-(1-methyl-1*H*-imidazol-5-yl)quinolin-4-yl)-2'-oxo-2',3'-dihydro-1'*H*-[1,5'-bibenzo[d]imidazole]-5-carboxylate | 0.831 |
| 108 | 2-(2-methoxypyridin-4-yl)-*N*-methyl-2'-oxo-2',3'-dihydro-1'*H*-[1,5'-bibenzo[d]imidazole]-5-carboxamide | 0.107 |
| 109 | *N*5-methyl-*N*1-(2-oxo-2,3-dihydro-1*H*-benzo[d]imidazol-5-yl)-3,4-dihydroquinoline-1,5(2*H*)-dicarboxamide | 0.039 |
| 110 | 5-(5-bromo-1-oxo-isoindolin-2-yl)-1,3-dihydrobenzimidazol-2-one | 0.029 |
| 111 | ethyl 1-oxo-2-(2-oxo-1,3-dihydrobenzimidazol-5-yl)isoindoline-5-carboxylate | 0.018 |
| 112 | methyl 1-oxo-2-(2-oxo-1,3-dihydrobenzimidazol-5-yl)isoindoline-5-carboxylate | 0.057 |
| 113 | 2-(2-(1-methyl-1*H*-imidazol-5-yl)quinolin-4-yl)-2'-oxo-2',3'-dihydro-1'*H*-[1,5'-bibenzo[d]imidazole]-5-carboxamide | 0.899 |
| 114 | *N*-methyl-2'-oxo-2-(1-phenylcyclopropyl)-2',3'-dihydro-1'*H*-[1,5'-bibenzo[d]imidazole]-5-carboxamide | 0.008 |
| 115 | 2-(1-(4-methoxyphenyl)cyclopropyl)-*N*-methyl-2'-oxo-2',3'-dihydro-1'*H*-[1,5'-bibenzo[d]imidazole]-5-carboxamide | 0.096 |
| 116 | 5-(indolin-1-ylsulfonyl)-1,3-dihydro-2*H*-benzo[d]imidazol-2-one | 0.048 |
| 117 | *N*-(1-acetylpiperidin-4-yl)-2-oxo-2,3-dihydro-1*H-*benzo[d]imidazole-5-sulfonamide | 0.008 |
| 118 | methyl 4-(*N*-(2-oxo-2,3-dihydro-1*H*-benzo[d]imidazol-5-yl)sulfamoyl)benzoate | 0.082 |
| 119 | *tert*-butyl (1-((2-oxo-2,3-dihydro-1*H*-benzo[d]imidazol-5-yl)carbamoyl)indolin-5-yl)carbamate | 0.113 |
| 120 | 5-(benzyloxy)-2-isopropyl-1',3'-dihydro-2'*H*-[1,5'-bibenzo[d]imidazol]-2'-one | 0.194 |
| 121 | 5-amino-*N*-(2-oxo-2,3-dihydro-1*H*-benzo[d]imidazol-5-yl)indoline-1-carboxamide hydrochloride | 0.065 |
| 122 | 2'-methyl-*N*-(2-oxo-2,3-dihydro-1*H*-benzo[d]imidazol-5-yl)-[1,1'-biphenyl]-2-carboxamide | 0.730 |
| 123 | 2-benzyl-*N*-(2-oxo-2,3-dihydro-1*H*-benzo[d]imidazol-5-yl)benzamide | 0.022 |
| 124 | 2-isopropyl-5-methoxy-1',3'-dihydro-2'*H*-[1,5'-bibenzo[d]imidazol]-2'-one | 0.013 |
| 125 | *tert*-butyl 4-(*N*-(2-oxo-2,3-dihydro-1*H*-benzo[d]imidazol-5-yl)sulfamoyl)piperidine-1-carboxylate | 0.020 |
| 126 | ethyl 4-(3-(2-oxo-2,3-dihydro-1*H*-benzo[d]imidazol-5-yl)ureido)benzoate | 0.009 |
| 127 | 1-(3-fluorophenyl)-3-(2-oxo-2,3-dihydro-1*H-*benzo[d]imidazol-5-yl)urea | 0.026 |
| 128 | 4-(2-methoxyphenyl)-*N*-(2-oxo-2,3-dihydro-1*H-*benzo[d]imidazol-5-yl)piperazine-1-carboxamide | 0.014 |
| 129 | 6-chloro-*N*-(2-oxo-2,3-dihydro-1*H*-benzo[d]imidazol-5-yl)indoline-1-carboxamide | 0.188 |
| 130 | *N*-(3-(1-methyl-1*H*-pyrazol-3-yl)phenyl)-2-oxo-2,3-dihydro-1*H*-benzo[d]imidazole-5-carboxamide | 0.781 |
| 131 | 2-(4-methoxycyclohexyl)-*N*-methyl-2'-oxo-2',3'-dihydro-1'*H*-[1,5'-bibenzo[d]imidazole]-5-carboxamide | 0.010 |
| 132 | 3-methoxy-*N*-(2-oxo-2,3-dihydro-1*H*-benzo[d]imidazol-5-yl)isonicotinamide | < 0.001 |
| 133 | *N*2-methyl-*N*5-(2-oxo-2,3-dihydro-1*H*-benzo[d]imidazol-5-yl)pyridine-2,5-dicarboxamide | 0.121 |
| 134 | *N*-(2-oxo-2,3-dihydro-1*H*-benzo[d]imidazol-5-yl)piperidine-4-sulfonamide | 0.007 |
| 135 | 4-(4-chlorobenzyl)-*N*-(2-oxo-2,3-dihydro-1*H-*benzo[d]imidazol-5-yl)piperazine-1-carboxamide | 0.007 |
| 136 | *N*-(2-oxo-2,3-dihydro-1*H*-benzo[d]imidazol-5-yl)-2-(pyridin-4-yl)benzamide | 0.079 |
| 137 | *N*-(2-oxo-2,3-dihydro-1*H*-benzo[d]imidazol-5-yl)benzofuran-3-sulfonamide | 0.022 |
| 138 | 2-(3-methoxyphenyl)-*N*-(2-oxo-2,3-dihydro-1*H-*benzo[d]imidazol-5-yl)nicotinamide | 0.109 |
| 139 | *N*-(1-benzylpiperidin-4-yl)-2-oxo-2,3-dihydro-1*H-*benzo[d]imidazole-5-carboxamide | 0.004 |
| 140 | 5-(5-aminoindolin-1-yl)-1,3-dihydro-2*H*-benzo[d]imidazol-2-one | 0.168 |
| 141 | *N*-(1-(2-oxo-2,3-dihydro-1*H*-benzo[d]imidazol-5-yl)indolin-5-yl)acetamide | 0.053 |
| 142 | *N*-(3-(oxazol-5-yl)phenyl)-2-oxo-2,3-dihydro-1*H-*benzo[d]imidazole-5-carboxamide | 0.011 |
| 143 | *tert*-butyl 4-((2-oxo-2,3-dihydro-1*H*-benzo[d]imidazol-5-yl)carbamoyl)indoline-1-carboxylate | 0.159 |
| 144 | *N*-(2-oxo-2,3-dihydro-1*H*-benzo[d]imidazol-5-yl)indoline-4-carboxamide | 0.029 |
| 145 | 1-acetyl-*N*-(2-oxo-2,3-dihydro-1*H*-benzo[d]imidazol-5-yl)indoline-4-carboxamide | 0.084 |
| 146 | *N*-(4-(((2-oxo-2,3-dihydro-1*H*-benzo[d]imidazol-5-yl)methyl)amino)phenyl)acetamide | 0.018 |
| 147 | *N*-(2-(methylcarbamoyl)phenyl)*-*2-oxo-2,3-dihydro-1*H-*benzo[d]imidazole-5-carboxamide | 0.338 |
| 148 | *tert*-butyl (1-((2-oxo-2,3-dihydro-1*H*-benzo[d]imidazol-5-yl)methyl)indolin-5-yl)carbamate | 0.019 |
| 149 | 5-((5-aminoindolin-1-yl)methyl)-1,3-dihydro-2*H-*benzo[d]imidazol-2-one | 0.017 |
| 150 | *N*-(1-((2-oxo-2,3-dihydro-1*H*-benzo[d]imidazol-5-yl)methyl)indolin-5-yl)acetamide | 0.003 |
| 151 | methyl 2-((2-oxo-2,3-dihydro-1*H*-benzo[d]imidazol-5-yl)carbamoyl)benzoate | 0.052 |
| 152 | 5-((1-benzylpiperidin-4-yl)amino)-1,3-dihydro-2*H-*benzo[d]imidazol-2-one | 0.021 |
| 153 | methyl 6-((2-oxo-2,3-dihydro-1*H*-benzo[d]imidazol-5-yl)amino)nicotinate | 0.016 |
| 154 | 5-(benzyloxy)-1,3-dihydro-2*H*-benzo[d]imidazol-2-one | 0.017 |
| 155 | methyl 2-((2-oxo-2,3-dihydro-1*H*-benzo[d]imidazol-5-yl)methyl)isoindoline-5-carboxylate | 0.029 |
| 156 | *N*-methyl-4-((2-oxo-2,3-dihydro-1*H*-benzo[d]imidazol-5-yl)oxy)benzamide | 0.022 |
| A | *N-*[5-[[2-[(9*S*)-7-(4-chlorophenyl)-4,5,13-trimethyl-3-thia-1,8,11,12-tetrazatricyclo[8.3.0.02,6]trideca-2(6),4,7,10,12-pentaen-9-yl]acetyl]amino]pentyl]-2-ethyl-1-(2-oxo-1,3-dihydrobenzimidazol-5-yl)benzimidazole-5-carboxamide | 0.055 |
| B | *N*-[2-[2-[2-[[2-[(9*S*)-7-(4-chlorophenyl)-4,5,13-trimethyl-3-thia-1,8,11,12-tetrazatricyclo[8.3.0.02,6]trideca-2(6),4,7,10,12-pentaen-9-yl]acetyl]amino]ethoxy]ethoxy]ethyl]-2-ethyl-1-(2-oxo-1,3-dihydrobenzimidazol-5-yl)benzimidazole-5-carboxamide | 0.051 |
| C | *N*-[2-[2-[2-[[2-[(9*S*)-7-(4-chlorophenyl)-4,5,13-trimethyl-3-thia-1,8,11,12-tetrazatricyclo[8.3.0.02,6]trideca-2(6),4,7,10,12-pentaen-9-yl]acetyl]amino]ethoxy]ethoxy]ethyl]-1-(2-oxo-1,3-dihydrobenzimidazol-5-yl)-2-(4-quinolyl)benzimidazole-5-carboxamide | 0.019 |
| D | *N*4-[5-[[2-[(9*S*)-7-(4-chlorophenyl)-4,5,13-trimethyl-3-thia-1,8,11,12-tetrazatricyclo[8.3.0.02,6]trideca-2(6),4,7,10,12-pentaen-9-yl]acetyl]amino]pentyl]-N1-(2-oxo-1,3-dihydrobenzimidazol-5-yl)indoline-1,4-dicarboxamide | 0.007 |

### Pharmaceutical Compositions

The compounds of formula I and the pharmaceutically acceptable salts can be used as therapeutically active substances, e.g. in the form of pharmaceutical preparations. The pharmaceutical preparations can be administered orally, e.g. in the form of tablets, coated tablets, dragées, hard and soft gelatin capsules, solutions, emulsions or suspensions. The administration can, however, also be effected rectally, e.g. in the form of suppositories, or parenterally, e.g. in the form of injection solutions.

The compounds of formula I and the pharmaceutically acceptable salts thereof can be processed with pharmaceutically inert, inorganic or organic carriers for the production of pharmaceutical preparations. Lactose, corn starch or derivatives thereof, talc, stearic acids or its salts can be used, for example, as such carriers for tablets, coated tablets, dragées and hard gelatin capsules. Suitable carriers for soft gelatin capsules are, for example, vegetable oils, waxes, fats, semi-solid and liquid polyols. Depending on the nature of the active substance no carriers are however usually required in the case of soft gelatin capsules. Suitable carriers for the production of solutions and syrups are, for example, water, polyols, glycerol, vegetable oil. Suitable carriers for suppositories are, for example, natural or hardened oils, waxes, fats, semi-liquid or liquid polyols.

The pharmaceutical preparations can, moreover, contain pharmaceutically acceptable auxiliary substances such as preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

Medicaments containing a compound of formula I or a pharmaceutically acceptable salt thereof and a therapeutically inert carrier are also provided, as is a process for their production, which comprises bringing one or more compounds of formula I and/or pharmaceutically acceptable salts thereof and, if desired, one or more other therapeutically valuable substances into a galenical administration form together with one or more therapeutically inert carriers.

The dosage can vary within wide limits and will, of course, have to be adjusted to the individual requirements in each particular case. In the case of oral administration the dosage for adults can vary from about 0.01 mg to about 1000 mg per day of a compound of general formula I or of the corresponding amount of a pharmaceutically acceptable salt thereof. The daily dosage may be administered as single dose or in divided doses and, in addition, the upper limit can also be exceeded when this is found to be indicated.

The following examples illustrate the present invention, serving merely as representative thereof. The pharmaceutical preparations conveniently contain about 1-500 mg, particularly 1-100 mg, of a compound of formula I. Examples of compositions according to the invention are:

### Example A

Tablets of the following composition are manufactured in the usual manner:

**Table 6: possible tablet composition**

| ingredient | mg/tablet | | | |
|---|---|---|---|---|
| | 5 | 25 | 100 | 500 |
| Compound of formula I | 5 | 25 | 100 | 500 |
| Lactose Anhydrous DTG | 125 | 105 | 30 | 150 |
| Sta-Rx 1500 | 6 | 6 | 6 | 60 |
| Microcrystalline Cellulose | 30 | 30 | 30 | 450 |
| Magnesium Stearate | 1 | 1 | 1 | 1 |
| Total | 167 | 167 | 167 | 831 |

### Manufacturing Procedure

1. Mix ingredients 1, 2, 3 and 4 and granulate with purified water.
2. Dry the granules at 50 °C.
3. Pass the granules through suitable milling equipment.
4. Add ingredient 5 and mix for three minutes; compress on a suitable press.

### Example B-1

Capsules of the following composition are manufactured:

**Table 7: possible capsule ingredient composition**

| ingredient | mg/capsule | | | |
|---|---|---|---|---|
| | 5 | 25 | 100 | 500 |
| Compound of formula I | 5 | 25 | 100 | 500 |
| Hydrous Lactose | 159 | 123 | 148 | - |
| Corn Starch | 25 | 35 | 40 | 70 |
| Talk | 10 | 15 | 10 | 25 |
| Magnesium Stearate | 1 | 2 | 2 | 5 |
| Total | 200 | 200 | 300 | 600 |

### Manufacturing Procedure

1. Mix ingredients 1, 2 and 3 in a suitable mixer for 30 minutes.
2. Add ingredients 4 and 5 and mix for 3 minutes.
3. Fill into a suitable capsule.

The compound of formula I, lactose and corn starch are firstly mixed in a mixer and then in a comminuting machine. The mixture is returned to the mixer; the talc is added thereto and mixed thoroughly. The mixture is filled by machine into suitable capsules, e.g. hard gelatin capsules.

### Example B-2

Soft Gelatin Capsules of the following composition are manufactured:

**Table 8: possible soft gelatin capsule ingredient composition**

| ingredient | mg/capsule |
|---|---|
| Compound of formula I | 5 |
| Yellow wax | 8 |
| Hydrogenated Soya bean oil | 8 |
| Partially hydrogenated plant oils | 34 |
| Soya bean oil | 110 |
| Total | 165 |

**Table 9: possible soft gelatin capsule composition**

| ingredient | mg/capsule |
|---|---|
| Gelatin | 75 |
| Glycerol 85 % | 32 |
| Karion 83 | 8 (dry matter) |
| Titan dioxide | 0.4 |
| Iron oxide yellow | 1.1 |
| Total | 116.5 |

### Manufacturing Procedure

The compound of formula I is dissolved in a warm melting of the other ingredients and the mixture is filled into soft gelatin capsules of appropriate size. The filled soft gelatin capsules are treated according to the usual procedures.

### Example C

Suppositories of the following composition are manufactured:

**Table 10: possible suppository composition**

| ingredient | mg/supp. |
|---|---|
| Compound of formula I | 15 |
| Suppository mass | 1285 |
| Total | 1300 |

### Manufacturing Procedure

The suppository mass is melted in a glass or steel vessel, mixed thoroughly and cooled to 45 °C. Thereupon, the finely powdered compound of formula I is added thereto and stirred until it has dispersed completely. The mixture is poured into suppository moulds of suitable size, left to cool; the suppositories are then removed from the moulds and packed individually in wax paper or metal foil.

### Example D

Injection solutions of the following composition are manufactured:

**Table 11: possible injection solution composition**

| ingredient | meg/injection solution. |
|---|---|
| Compound of formula I | 3 |
| Polyethylene Glycol 400 | 150 |
| acetic acid | q.s. ad pH 5.0 |
| water for injection solutions | ad 1.0 ml |

### Manufacturing Procedure

The compound of formula I is dissolved in a mixture of Polyethylene Glycol 400 and water for injection (part). The pH is adjusted to 5.0 by acetic acid. The volume is adjusted to 1.0 ml by addition of the residual amount of water. The solution is filtered, filled into vials using an appropriate overage and sterilized.

### Example E

Sachets of the following composition are manufactured:

**Table 12: possible sachet composition**

| ingredient | mg/sachet |
|---|---|
| Compound of formula I | 50 |
| Lactose, fine powder | 1015 |
| Microcrystalline cellulose (AVICEL PH 102) | 1400 |
| Sodium carboxymethyl cellulose | 14 |
| Polyvinylpyrrolidon K 30 | 10 |
| Magnesium stearate | 10 |
| Flavoring additives | 1 |
| Total | 2500 |

### Manufacturing Procedure

The compound of formula I is mixed with lactose, microcrystalline cellulose and sodium carboxymethyl cellulose and granulated with a mixture of polyvinylpyrrolidone in water. The granulate is mixed with magnesium stearate and the flavoring additives and filled into sachets.

### Experimental Part

The following examples are provided for illustration of the invention.

### Example 1 (provided for reference)

### 3-Amino-N-methyl-4-[(2-oxo-1,3-dihydrobenzimidazol-5-yl)amino]benzamide

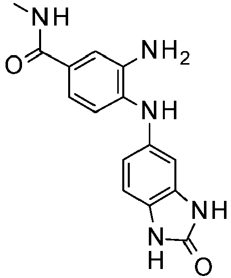

### a) N-Methyl-3-nitro-4-((2-oxo-2,3-dihydro-1H-benzo[d]imidazol-5-yl)amino)benzamide

A stirred solution of 4-fluoro*-N*-methyl-3-nitrobenzamide⁴⁶ (200 mg), 5-amino-1*H-*benzo[d]imidazol-2(3*H*)-one⁵⁰ (151 mg) and *N*,*N*-diisopropylethylamine (264 µl) in*N*-methyl-2-pyrrolidinone (1 ml) was heated at 120 °C overnight. The reaction mixture was then cooled to room temperature and the crude product was collected by filtration, washed with water, and then dried *in vacuo* at 40 °C to afford N-methyl-3-nitro-4-((2-oxo-2,3-dihydro-1H-benzo[d]imidazol-5-yl)amino)benzamide (296 mg, 90%) as a red solid which was used in the next step without further purification. MS (ISP): 328.0 ([M+H]⁺).

### b) 3-Amino-N-methyl-4-[(2-oxo-1,3-dihydrobenzimidazol-5-yl)amino]benzamide

To a stirred suspension of *N*-methyl-3-nitro-4-((2-oxo-2,3-dihydro-1*H*-benzo[d]imidazol-5-yl)amino)benzamide (295 mg) in methanol (158 ml) and THF (158 ml) was added 10% palladium on charcoal (48 mg). The reaction mixture was stirred overnight at room temperature under an atmosphere of hydrogen. The catalyst was collected by filtration, washing with methanol. The filtrate was then concentrated *in vacuo* to afford 3-amino-*N*-methyl-4-[(2-oxo-1,3-dihydrobenzimidazol-5-yl)amino]benzamide (265 mg, 99%) as a brown solid. MS (ISP): 298.0 ([M+H]⁺).

### Example 2

### N-Methyl-2-(2-(1-methyl-1H-imidazol-5-yl)quinolin-4-yl)-2'-oxo-2',3'-dihydro-1'H-[1,5'-bibenzo[d]imidazole]-5-carboxamide

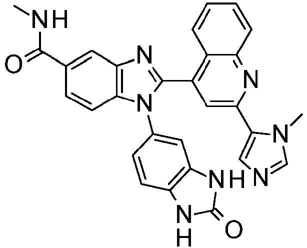

To a stirred solution of 3-amino-*N*-methyl-4-[(2-oxo-1,3-dihydrobenzimidazol-5-yl)amino]benzamide (50 mg, example 1) and 2-(3-methylimidazol-4-yl)quinoline-4-carbaldehyde⁶¹ (39.9 mg) in *N*,*N*-dimethylformamide (1.5 ml) was added sodium metabisulfite (128 mg) at room temperature. The reaction mixture was then heated at 120 °C overnight. Subsequent TLC and LC-MS analysis showed the reaction was complete. The reaction mixture was then cooled to room temperature and poured into EtOAc/THF (30 ml, 1:1 mixture). The resulting mixture was washed sequentially with water and with saturated brine. The organic phase was then separated, dried over MgSO₄, filtered, and concentrated *in vacuo.* The crude material was purified by preparative reversed phase HPLC to afford after lyophilisation *N*-methyl-2-(2-(1-methyl-1*H*-imidazol-5-yl)quinolin-4-yl)-2'-oxo-2',3'-dihydro-1'*H*-[1,5'-bibenzo[d]imidazole]-5-carboxamide (21 mg, 24%) as a white solid. 515.2 ([M+H]⁺).

### Example 3

### N-Methyl-2'-oxo-2-(quinolin-4-yl)-2',3'-dihydro-1'H-[1,5'-bibenzo[d]imidazole]-5-carboxamide

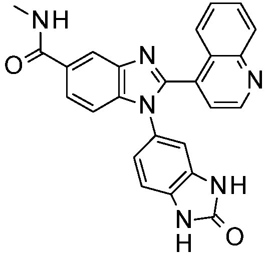

The title compound was obtained in analogy to example 2 using quinoline-4-carbaldehyde⁶² in place of 2-(3-methylimidazol-4-yl)quinoline-4-carbaldehyde. White solid. MS (ISP): 435.2 ([M+H]⁺).

### Example 4

### 2-Ethyl-N-methyl-2'-oxo-2',3'-dihydro-1'H-[1,5'-bibenzo[d]imidazole]-5-carboxamide

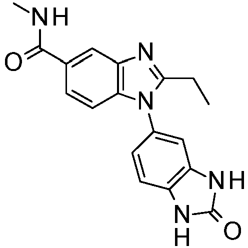

The title compound was obtained in analogy to example 2 using propionaldehyde⁶³ in place of 2-(3-methylimidazol-4-yl)quinoline-4-carbaldehyde. White solid. MS (ISP): 336.1 ([M+H]⁺).

### Example 5

### N,2-Dimethyl-2'-oxo-2',3'-dihydro-1'H-[1,5'-bibenzo[d]imidazole]-5-carboxamide

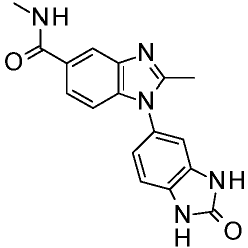

The title compound was obtained in analogy to example 2 using acetaldehyde⁶⁴ in place of 2-(3-methylimidazol-4-yl)quinoline-4-carbaldehyde. White solid. MS (ISP): 322.1 ([M+H]⁺).

### Example 6 (provided for reference)

### 5-((2-Aminophenyl)amino)-1H-benzo[d]imidazol-2(3H)-one

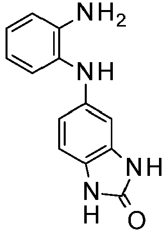

The title compound was obtained in analogy to example 1 using 1-fluoro-2-nitrobenzene⁴⁵ in place of 4-fluoro-*N*-methyl-3-nitrobenzamide in step a. Brown solid. MS (ISP): 240.9 ([M+H]⁺).

### Example 7

### 2-(Quinolin-4-yl)-1'H-[1,5'-bibenzo[d]imidazol]-2'(3'H)-one

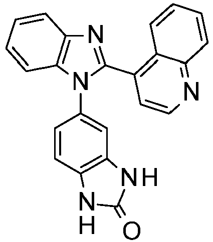

The title compound was obtained in analogy to example 2 using 5-((2-aminophenyl)amino)-1*H*-benzo[d]imidazol-2(3*H*)-one (example 6) in place of 3-amino-*N-*methyl-4-[(2-oxo-1,3-dihydrobenzimidazol-5-yl)amino]benzamide and quinoline-4-carbaldehyde⁶² in place of 2-(3-methylimidazol-4-yl)quinoline-4-carbaldehyde. White solid. MS (ISP): 378.1 ([M+H]⁺).

### Example 8

### 2-(2-(1-Methyl-1H-imidazol-5-yl)quinolin-4-yl)-1'H-[1,5'-bibenzo[d]imidazol]-2'(3'H)-one

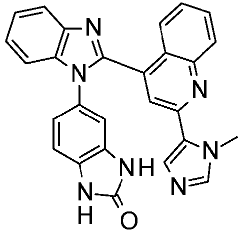

The title compound was obtained in analogy to example 2 using 5-((2-aminophenyl)amino)-1H-benzo[d]imidazol-2(3*H*)-one (example 6) in place of 3-amino-*N-*methyl-4-[(2-oxo-1,3-dihydrobenzimidazol-5-yl)amino]benzamide. White solid. MS (ISP): 458.2 ([M+H]⁺).

### Example 9 (provided for reference)

### Methyl 3-amino-4-((2-oxo-2,3-dihydro-1H-benzo[d]imidazol-5-yl)amino)benzoate

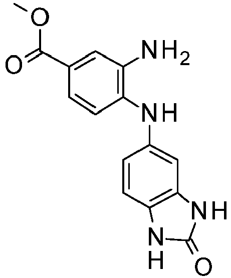

The title compound was obtained in analogy to example 1 using methyl 4-fluoro-3-nitrobenzoate⁴⁸ in place of 4-fluoro-N-methyl-3-nitrobenzamide in step a. Grey solid. MS (ISP): 299.2 ([M+H]⁺).

### Example 10

### N-Methyl-2'-oxo-2-phenyl-2',3'-dihydro-1'H-[1,5'-bibenzo[d]imidazole]-5-carboxamide

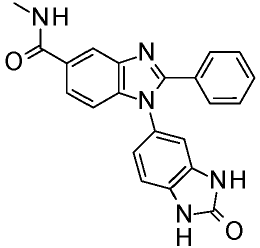

The title compound was obtained in analogy to example 2 using benzaldehyde⁶⁵ in place of 2-(3-methylimidazol-4-yl)quinoline-4-carbaldehyde. White solid. MS (ISP): 384.1 ([M+H]⁺).

### Example 11

### N-Methyl-2'-oxo-2-(pyridin-4-yl)-2',3'-dihydro-1'H-[1,5'-bibenzo[d]imidazole]-5-carboxamide

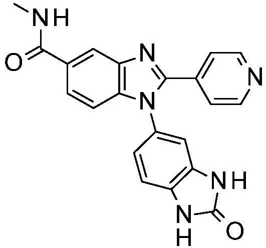

The title compound was obtained in analogy to example 2 using isonicotinaldehyde⁶⁶ in place of 2-(3-methylimidazol-4-yl)quinoline-4-carbaldehyde. White solid. MS (ISP): 385.1 ([M+H]⁺).

### Example 12 (provided for reference)

### 5-(2-(Quinolin-4-yl)-1H-imidazol-1-yl)-1H-benzo[d]imidazol-2(3H)-one

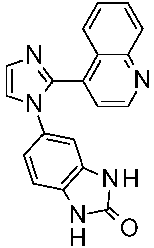

### a) 4-(1H-Imidazol-2-yl)quinoline

To a stirred solution of quinoline-4-carbaldehyde⁶² (1 g) in methanol (55 ml) at 0 °C was added 25 % aq. ammonium hydroxide solution (4.96 ml) followed by dropwise addition of oxalaldehyde (7.3 ml, 40% solution in water). The reaction mixture was stirred at room temperature for 72 hours. The solvent was then evaporated and the residue was partitioned between ethyl acetate (20 ml) and water (20 ml). The phases were separated and the organic phase was dried over MgSO₄, filtered, and concentrated *in vacuo.* The crude material was purified by flash chromatography (silica gel, eluent: 0 to 10% of methanol in dichloromethane) to afford 4-(1H-imidazol-2-yl)quinoline (320mg, 26%) as an off-white solid. MS (ISP): 196.4 ([M+H]⁺).

### b) 2-Nitro-5-(2-(quinolin-4-yl)-1H-imidazol-1-yl)aniline

To a stirred solution of 4-(1*H*-imidazol-2-yl)quinoline (190 mg) in DMF (3 ml) was added sodium hydride (38.9 mg) and the mixture was stirred at room temperature for 15min (gas evolution). 5-Fluoro-2-nitroaniline (152 mg) was added and the reaction mixture was heated at 60 °C overnight. After cooling, the reaction mixture was diluted with ethyl acetate and washed with 1 M aq. sodium bicarbonate solution. The organic phase was dried over MgSO₄, filtered, and concentrated *in vacuo.* The crude material was purified by flash chromatography (silica gel, eluent: 0 to 10% of methanol in dichloromethane) to afford 2-nitro-5-(2-(quinolin-4-yl)-1H-imidazol-1-yl)aniline (44 mg, 14%) as a yellow solid. MS (ISP): 332.1 ([M+H]⁺).

### c) 4-(2-(Quinolin-4-yl)-1H-imidazol-1-yl)benzene-1,2-diamine

To a stirred solution of 2-nitro-5-(2-(quinolin-4-yl)-1H-imidazol-1-yl)aniline (44 mg) in a mixture of methanol (5 ml) and THF (2 ml) was added 10% palladium on charcoal (14.1 mg). The reaction mixture was hydrogenated under an atmosphere of hydrogen. After stirring vigorously for 1 hour, the catalyst was collected by filtration, washing with methanol. The filtrate was then concentrated *in vacuo* to afford 4-(2-(quinolin-4-yl)-1H-imidazol-1-yl)benzene-1,2-diamine as a green solid (38 mg, 95%), which was used in the next step without further purification. MS (ISP): 302.2 ([M+H]⁺).

### d) 5-(2-(Quinolin-4-yl)-1H-imidazol-1-yl)-1H-benzo[d]imidazol-2(3H)-one

To a stirred solution of 4-(2-(quinolin-4-yl)-1*H*-imidazol-1-yl)benzene-1,2-diamine (38 mg) in THF (1 ml) was added dropwise a solution of di(1*H*-imidazol-1-yl)methanone (20.4 mg) in THF (1 ml). The reaction mixture was stirred at room temperature overnight. TLC analysis showed that the reaction was not finished. A solution of di(1*H*-imidazol-1-yl)methanone (20.4 mg) in tetrahydrofuran (0.5 ml) was added dropwise and the reaction was stirred at room temperature for 2 h. The reaction mixture was then partitioned between ethyl acetate (5 ml) and water (5 ml). The phases were separated and the organic phase was dried over MgSO₄, filtered, and concentrated *in vacuo.* The crude material was purified by flash chromatography (silica gel, eluent: 0 to 10% of methanol in dichloromethane) to afford 5-(2-(quinolin-4-yl)-1H-imidazol-1-yl)-1H-benzo[d]imidazol-2(3H)-one (10 mg, 24%) as a white solid. MS (ISP): 328.1 ([M+H]⁺).

### Example 13

### N-Methyl-2'-oxo-2-(trifluoromethyl)-2',3'-dihydro-1'H-[1,5'-bibenzo[d]imidazole]-5-carboxamide

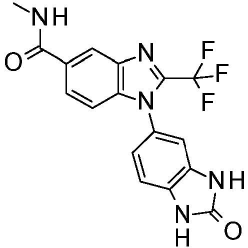

A stirred solution of 3-amino-*N*-methyl-4-[(2-oxo-1,3-dihydrobenzimidazol-5-yl)amino]benzamide (39 mg, example 1) in 2,2,2-trifluoroacetic acid (299 mg, 201 µl) was heated at 120 °C overnight in a sealed tube. The reaction mixture was then cooled to room temperature and concentrated *in vacuo.* The crude product was dissolved in DMSO, and then 25% aq. ammonia was added to adjust the pH to 14. The mixture was poured into THF/EtOAc (2:1) and extracted sequentially with water and with saturated brine. The organic layer was then separated, dried over MgSO₄ and concentrated *in vacuo.* The product was purified by reversed phase HPLC followed by lyophilisation to afford *N*-methyl-2'-oxo-2-(trifluoromethyl)-2',3'-dihydro-1'*H*-[1,5'-bibenzo[d]imidazole]-5-carboxamide (10mg, 20%) as a white solid. MS (ISP): 376.1 ([M+H]⁺).

### Example 14

### N-Methyl-2'-oxo-2-propyl-2',3'-dihydro-1'H-[1,5'-bibenzo[d]imidazole]-5-carboxamide

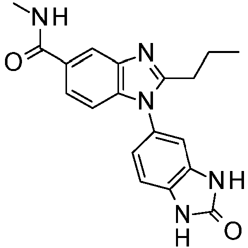

The title compound was obtained in analogy to example 2 using butyraldehyde⁶⁷ in place of 2-(3-methylimidazol-4-yl)quinoline-4-carbaldehyde. White solid. MS (ISP): 350.2 ([M+H]⁺).

### Example 15

### 2-(1H-Imidazol-4-yl)-N-methyl-2'-oxo-2',3'-dihydro-1'H-[1,5'-bibenzo[d]imidazole]-5-carboxamide

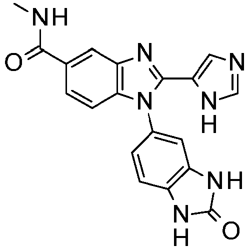

The title compound was obtained in analogy to example 2 using 1*H*-imidazole-4-carbaldehyde⁶⁸ in place of 2-(3-methylimidazol-4-yl)quinoline-4-carbaldehyde. White solid. MS (ISP): 374.1 ([M+H]⁺).

### Example 16

### 2-Isopropyl-N-methyl-2'-oxo-2',3'-dihydro-1'H-[1,5'-bibenzo[d]imidazole]-5-carboxamide

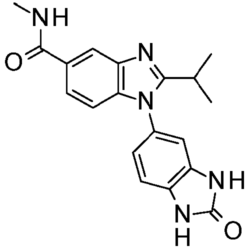

The title compound was obtained in analogy to example 2 using isobutyraldehyde⁶⁹ in place of 2-(3-methylimidazol-4-yl)quinoline-4-carbaldehyde. White solid. MS (ISP): 350.2 ([M+H]⁺).

### Example 17

### N,1',3'-Trimethyl-2-(2-(1-methyl-1H-imidazol-5-yl)quinolin-4-yl)-2'-oxo-2',3'-dihydro-1'H-[1,5'-bibenzo[d]imidazole]-5-carboxamide

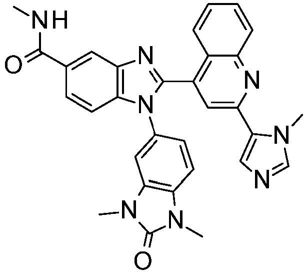

### a) 3-Amino-4-[(1,3-dimethyl-2-oxobenzimidazol-5-yl)amino]-N-methylbenzamide

The title compound was obtained in analogy to example 1 using 5-amino-1,3-dimethyl-1*H-*benzo[d]imidazol-2(3*H*)-one⁵³ in place of 5-amino-1*H*-benzo[d]imidazol-2(3*H*)-one in step a. Grey solid. MS (ISP): 326.1 ([M+H]⁺).

### b) N,1',3'-trimethyl-2-(2-(1-methyl-1H-imidazol-5-yl)quinolin-4-yl)-2'-oxo-2',3'-dihydro-1'H-[1,5'-bibenzo[d]imidazole]-5-carboxamide

The title compound was obtained in analogy to example 2 using 3-amino-4-[(1,3-dimethyl-2-oxobenzimidazol-5-yl)amino]- *N*-methylbenzamide in place of 3-amino-*N*-methyl-4-[(2-oxo-1,3-dihydrobenzimidazol-5-yl)amino]benzamide. White solid. MS (ISP): 543.2 ([M+H]⁺).

### Example 18 (provided for reference)

### N,N-Dimethyl-2-(2-(1-methyl-1H-imidazol-5-yl)quinolin-4-yl)-2'-oxo-2',3'-dihydro-1'H-[1,5'-bibenzo[d]imidazole]-5-carboxamide

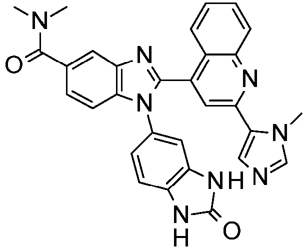

### a) Methyl 3-nitro-4-[(2-oxo-1,3-dihydrobenzimidazol-5-yl)amino]benzoate

To a stirred solution of methyl 4-fluoro-3-nitrobenzoate⁴⁸ (1.5 g) and 5-amino-1*H-*benzo[d]imidazol-2(3*H*)-one⁵⁰ (1.12 g) in *N*-methyl-2-pyrrolidinone (1 ml) was added dropwise *N*,*N*-diisopropylethylamine (1.97 ml). The reaction mixture was heated at 120 °C overnight. The reaction mixture was then cooled to room temperature and filtered through sintered glass to afford methyl 3-nitro-4-[(2-oxo-1,3-dihydrobenzimidazol-5-yl)amino]benzoate (2.48 g, quant.) as a red solid. MS (ISP): 329.1 ([M+H]⁺).

### b) Methyl 3-amino-4-[(2-oxo-1,3-dihydrobenzimidazol-5-yl)amino]benzoate

To a stirred suspension of methyl 3-nitro-4-[(2-oxo-1,3-dihydrobenzimidazol-5-yl)amino]benzoate (2.48 g) in methanol (30 ml) and THF (30 ml) was added 10% palladium on charcoal (402 mg). The reaction mixture was stirred at room temperature overnight under an atmosphere of hydrogen. The reaction mixture was filtered through celite, washing with methanol. The filtrate was then concentrated *in vacuo* to afford methyl 3-amino-4-[(2-oxo-1,3-dihydrobenzimidazol-5-yl)amino]benzoate (2.2 g, 98%) as a grey solid. MS (ISP): 299.2 ([M+H]⁺).

### c) Methyl 2-(2-(1-methyl-1H-imidazol-5-yl)quinolin-4-yl)-2'-oxo-2',3'-dihydro-1'H-[1,5'-bibenzo[d]imidazole]-5-carboxylate

To a stirred solution of methyl 3-amino-4-[(2-oxo-1,3-dihydrobenzimidazol-5-yl)amino]benzoate (200 mg) and 2-(1-methyl-1*H*-imidazol-5-yl)quinoline-4-carbaldehyde⁶¹ (159 mg) in *N,N*-dimethylformamide (2.2 ml) was added sodium metabisulfite (510 mg) at room temperature. The reaction mixture was then heated at 120 °C for 3 h. LC-MS analysis showed the reaction was complete. The reaction mixture was then cooled to room temperature and partitioned between ethyl acetate/THF (1: 1, 50 ml) and 1 M aqueous sodium hydroxide solution (30 ml). The layers were separated and the aqueous layer was extracted twice with ethyl acetate (2 x 30 ml). The combined organic layers were washed with saturated brine (30 ml), dried over anhydrous Na₂SO₄, filtered, and concentrated *in vacuo.* The residue was purified by preparative reverse phase HPLC on a Gemini NX 3u 50 x 4.6 mm column to afford methyl 2-(2-(1-methyl-1*H*-imidazol-5-yl)quinolin-4-yl)-2'-oxo-2',3'-dihydro-1*'H-*[1,5'-bibenzo[d]imidazole]-5-carboxylate (166 mg, 48%) as a light brown solid. MS (ISP): 516.1 ([M+H]⁺).

### d) 2-(2-(1-Methyl-1H-imidazol-5-yl)quinolin-4-yl)-2'-oxo-2',3'-dihydro-1'H-[1,5'-bibenzo[d]imidazole]-5-carboxylic acid

To a stirred solution of methyl 2-(2-(1-methyl-1*H*-imidazol-5-yl)quinolin-4-yl)-2'-oxo-2',3'-dihydro-1'*H*-[1,5'-bibenzo[d]imidazole]-5-carboxylate (166 mg) in 1,4-dioxane (4 ml) was added dropwise 1 M aq. LiOH solution (2 ml). The reaction mixture was stirred at room temperature for 3 h. Subsequent LC-MS showed the reaction was complete. The reaction mixture was partitioned between ethyl acetate/THF (1:1, 50 ml) and 1 M aqueous HCl (5 ml). The layers were separated. The aqueous layer was extracted three timesd with ethyl acetate (3 x 20 ml). The combined organic layers were dried over anhydrous sodium sulfate and concentrated *in vacuo.* Most product was detected in aqueous layer. The aqueous layer was concentrated *in vacuo* and the residue was lyophilised to afford 2-(2-(1-methyl-1*H*-imidazol-5-yl)quinolin-4-yl)-2'-oxo-2',3'-dihydro-1'*H*-[1,5'-bibenzo[d]imidazole]-5-carboxylic acid as a yellow solid (451mg, 100%, 36% purity) which was used in the next step without further purification. MS (ISP): 500.1 ([M-H]⁻).

### e) N,N-Dimethyl-2-(2-(1-methyl-1H-imidazol-5-yl)quinolin-4-yl)-2'-oxo-2',3'-dihydro-1'H-[1,5'-bibenzo[d]imidazole]-5-carboxamide

To a solution of 2-(2-(1-methyl-1*H*-imidazol-5-yl)quinolin-4-yl)-2'-oxo-2',3'-dihydro-1'*H-*[1,5'-bibenzo[d]imidazole]-5-carboxylic acid (451 mg), N,N-diisopropylethylamine (188 µl) and dimethylamine (202 µl, 2 M solution in THF) in N,Ndimethylformamide (4 ml) was added HATU (226 mg). The reaction mixture was stirred at room temperature for 3 h. The reaction mixture was partitioned between ethyl acetate (50 ml) and water (30 ml). The layers were separated and the organic layer was washed with saturated brine (30 ml), dried over anhydrous sodium sulfate, and concentrated *in vacuo.* The residue was purified by reversed phase HPLC on a Gemini NX 3u 50 x 4.6 mm column to afford N,Ndimethyl-2-(2-(1-methyl-1*H*-imidazol-5-yl)quinolin-4-yl)-2'-oxo-2',3'-dihydro-1*'H*-[1,5'-bibenzo[d]imidazole]-5-carboxamide as a yellow solid (74 mg, 52%). MS (ISP): 527.1 ([M-H]⁻).

### Example 19 (provided for reference)

### 5-(Pyridin-3-yl)-1H-benzo[d]imidazol-2(3H)-one

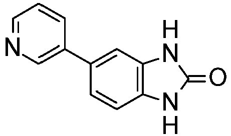

To a solution of 5-bromo-1*H*-benzo[d]imidazol-2(3*H*)-one⁷⁰ (50 mg) in dioxane (1 ml) were added pyridin-3-ylboronic acid ⁷¹ (43.3 mg), [1,1-bis(diphenyl-phosphino)ferrocene]dichloropalladium(II) (17.2 mg) and 2 M aq. sodium carbonate solution (235 µl). The reaction mixture was heated at 100 °C for 18 hours. The reaction mixture was then cooled to room temperature, diluted with water (50 ml). and extracted twice with ethyl acetate (2 x 30ml). The combined organic layers were dried over Na₂SO₄, filtered and concentrated *in vacuo.* The residue was purified by chromatography (silica gel; 12 g; eluent: dichloromethane/methanol = 100/0 to 95/05 in 10 minutes) to afford 5-(pyridin-3-yl)-1*H*-benzo[d]imidazol-2(3*H*)-one (12 mg, 23%) as a red solid. MS (ISP): 212.1 ([M+H]⁺).

### Example 20 (provided for reference)

### 5-(2-Chloropyridin-4-yl)-1H-benzo[d]imidazol-2(3H)-one

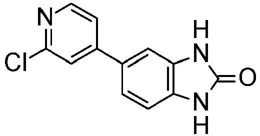

The title compound was obtained in analogy to example 19 using (2-chloropyridin-4-yl)boronic acid⁷² in place of pyridin-3-ylboronic acid. Light yellow solid. MS (ISP): 246.1 ({³⁵Cl}[M+H]⁺), 248.1 (³⁷Cl}[M+H]⁺).

### Example 21 (provided for reference)

### 5-(2-Methylthiazol-5-yl)-1H-benzo[d]imidazol-2(3H)-one

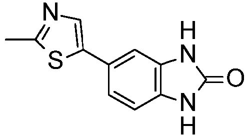

The title compound was obtained in analogy to example 19 using 2-methyl-5-(4,4,5,5-tetramethyl-1,3,2-dioxaborolan-2-yl)thiazole⁷³ in place of pyridin-3-ylboronic acid. Light brown solid. MS (ISP): 232.0 ([M+H]⁺).

### Example 22 (provided for reference)

### 5-(4-Acetylpiperazine-1-carbonyl)-1H-benzo[d]imidazol-2(3H)-one

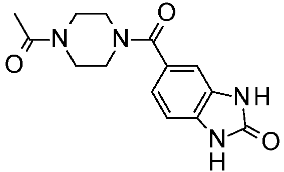

To a stirred solution of 2-oxo-2,3-dihydro-1*H*-benzo[d]imidazole-5-carboxylic acid**^{Error! Bookmark not defined.}** (70 mg), 1-(piperazin-1-yl)ethanone⁷⁴ (50.4 mg) and *N,N-*diisopropylethylamine (206 µl) in DMF (1 ml) was added HATU (224 mg). The reaction mixture was stirred at room temperature for 4 h. The reaction mixture was concentrated *in vacuo* and the residue was purified by preparative reverse-phase HPLC (Gemini NX C18, 12 nm, 5µ, 100 x 30 mm, flow rate 40 ml/min, eluant: CH₃CN/H₂O containing formic acid) followed by lyophilisation to afford 5-(4-acetylpiperazine-1-carbonyl)-1H-benzo[d]imidazol-2(3H)-one (50 mg, 44%) as white solid. MS (ISP): 289.1 ([M+H]⁺).

### Example 23 (provided for reference)

### N-(1-Acetylpiperidin-4-yl)-2-oxo-2,3-dihydro-1H-benzo[d]imidazole-5-carboxamide

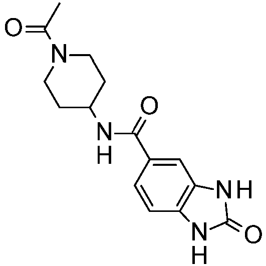

The title compound was obtained in analogy to example 22 using 1-(4-aminopiperidin-1-yl)ethanone⁷⁵ in place of 1-(piperazin-1-yl)ethanone. Brown solid. MS (ISP): 303.1 ([M+H]⁺).

### Example 24 (provided for reference)

### 5-(Indoline-1-carbonyl)-1H-benzo[d]imidazol-2(3H)-one

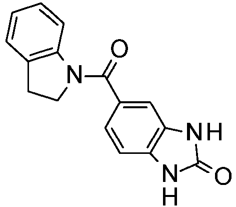

The title compound was obtained in analogy to example 22 using indoline⁷⁶ in place of 1-(piperazin-1-yl)ethanone. Light yellow solid. MS (ISP): 280.1 ([M+H]⁺).

### Example 25 (provided for reference)

### N-(1,3-Dimethyl-2-oxo-benzimidazol-5-yl)furan-2-carboxamide

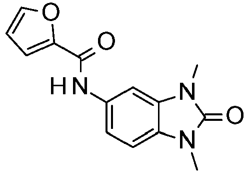

The title compound⁷⁷ can be purchased.

### Example 26 (provided for reference)

### 5-(3-Phenyl-propyl)-1,3-dihydro-imidazo[4,5-b]pyridin-2-one

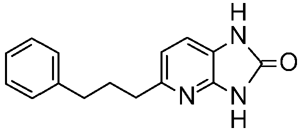

### a) Benzyl N-[2-(benzyloxycarbonylamino)-6-methyl-3-pyridyl]carbamate

To a stirred solution of 6-methylpyridine-2,3-diamine⁷⁸ (1.53 g) in THF (10 ml) were added *N*,*N*-diisopropylethylamine (5.13 ml) and benzyl chloroformiate (4.3 ml). The reaction mixture was stirred at room temperature for 24 hours. Water and ethyl acetate were added. The organic layer was washed with brine, dried over MgSO₄, filtered, and concentrated *in vacuo.* The crude material was purified by flash chromatography (silica gel, 20 g, eluent: 50 % ethyl acetate in heptane) followed by crystallisation in EtOAc/heptane to afford benzyl N-[2-(benzyloxycarbonylamino)-6-methyl-3-pyridyl]carbamate (3.15 g, 65%) as a yellow oil.

¹H NMR (300 MHz, CDCl₃) δ 7.30-7.43 (m, 12H), 7.04 (br d, *J =* 8.07 Hz, 1H), 5.21 (s, 2H), 5.19 (s, 2H), 4.70 (d, *J =* 5.85 Hz, 1H),

### b) Benzyl N-[2-(benzyloxycarbonylamino)-6-(bromomethyl)-3-pyridyl]carbamate

To a stirred solution of benzyl *N*-[2-(benzyloxycarbonylamino)-6-methyl-3-pyridyl]carbamate (3.15 g) in benzene (20 ml) was added *N*-bromosuccinimide (1.6 g) and 2,2'-azobis(2-methylpropionitrile (150 mg). The orange reaction mixture was stirred at 80 °C for 6 hours. Water and ethyl acetate were added. The organic layer was washed with brine, dried over MgSO₄, filtered, and concentrated *in vacuo* to afford benzyl *N*-[2-(benzyloxycarbonylamino)-6-(bromomethyl)-3-pyridyl]carbamate (3.64 g, 96%) as a brown oil which was used in the next step without further purification.

### c) [5,6-Bis(benzyloxycarbonylamino)-2-pyridyl]methyl-triphenyl-phosphonium bromide

To a stirred solution of benzyl *N*-[2-(benzyloxycarbonylamino)-6-(bromomethyl)-3-pyridyl]carbamate (3.64 g) in THF (30 ml) was added triphenylphosphine (6 g). The orange reaction mixture was stirred at 50 °C for 18 hours. The by-product was filtered off and the filtrate was concentrated *in vacuo.* The residue was dissolved in CH₂Cl₂ (20 ml) and added dropwise with stirrring to TBME (300 ml). The resulting suspension was filtered to afford crude [5,6-bis(benzyloxycarbonylamino)-2-pyridyl]methyl-triphenyl-phosphonium bromide (3.2 g, 56%) as a beige solid which was used in the next step without further purification.

### d) Benzyl N-[2-(benzyloxycarbonylamino)-6-[(E)-3-phenylprop-1-enyl]-3-pyridyl]carbamate

To a stirred solution of [5,6-bis(benzyloxycarbonylamino)-2-pyridyl]methyl-triphenylphosphonium bromide (3.20 g) in dichloromethane (25 ml) was added 1,8-diazabicyclo[5.4.0]undec-7-ene (1 ml) and phenylacetaldehyde⁷⁹ (1 ml). The brown reaction mixture was stirred at room temperature for 21 hours. Water and ethyl acetate were added. The pH was adjusted to 5 with 1 M aq. HCl. The organic layer was dried over MgSO₄, filtered, and concentrated *in vacuo* to afford crude benzyl *N*-[2-(benzyloxycarbonylamino)-6-[(*E*)-3-phenylprop-1-enyl]-3-pyridyl]carbamate (3.5 g, >100%) as a yellow oil which was used in the next step without further purification.

### e) 5-(3-Phenyl-propyl)-1,3-dihydro-imidazo[4,5-b]pyridin-2-one

To a stirred suspension of benzyl *N*-[2-(benzyloxycarbonylamino)-6-[(*E*)-3-phenylprop-1-enyl]-3-pyridyl]carbamate (3.5g) in ethanol (100 ml) were added *N*,*N*-diisopropylethylamine (5 ml) and 10% palladium on charcoal (500 mg). The reaction mixture was stirred at room temperature under an atmosphere of hydrogen for 23 h. The reaction mixture was filtered through celite, washing with methanol. The filtrate was then concentrated *in vacuo.* The crude material was purified by flash chromatography (silica gel, 20 g, eluent: 50 % of ethyl acetate in heptane to 20% methanol in ethyl acetate) followed by crystallisation in EtOAc/heptane to afford 5-(3-phenyl-propyl)-1,3-dihydro-imidazo[4,5-b]pyridin-2-one (109 mg, 6%) as a light brown solid. MS (ISP): 254.3([M+H]⁺).

### Example 27 (provided for reference)

### 1-Methyl-2-oxo-N-phenyl-3H-imidazo[4,5-b]pyridine-6-carboxamide

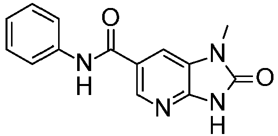

The title compound was obtained in analogy to example 22 using aniline⁸⁰ in place of 1-(piperazin-1-yl)ethanone and 1-methyl-2-oxo-3*H*-imidazo[4,5-b]pyridine-6-carboxylic acid⁸¹ in place of 2-oxo-2,3-dihydro-1*H*-benzo[d]imidazole-5-carboxylic acid. Off-white solid. MS (ISP): 269.1 ([M+H]⁺).

### Example 28

### 2-(3-Methoxybenzyl)-N-methyl-2'-oxo-2',3'-dihydro-1'H-[1,5'-bibenzo[d]imidazole]-5-carboxamide

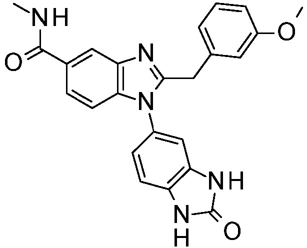

The title compound was obtained in analogy to example 2 using 2-(3-methoxyphenyl)acetaldehyde ⁸² in place of 2-(1-methyl-1*H*-imidazol-5-yl)quinoline-4-carbaldehyde. White solid. MS (ISP): 347.2 ([M+H]⁺).

### Example 29 (provided for reference)

### N-(1-(2-Oxo-2,3-dihydro-1H-benzo[d]imidazole-5-carbonyl)piperidin-4-yl)acetamide

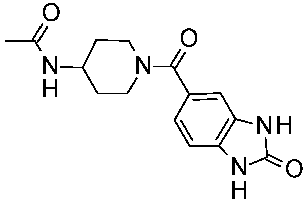

The title compound was obtained in analogy to example 22 using *N*-(piperidin-4-yl)acetamide⁸³ in place of 1-(piperazin-1-yl)ethanone. White solid. MS (ISP): 303.1 ([M+H]⁺).

### Example 30

### 2-Isopropyl-N-methyl-2'-oxo-2',3'-dihydro-1'H-[1,5'-bibenzo[d]imidazole]-6-carboxamide

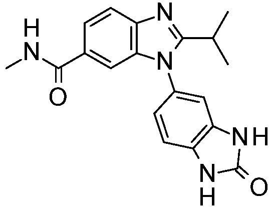

The title compound was obtained in analogy to example 18 using methyl 3-fluoro-4-nitrobenzoate⁴⁹ in place of methyl 4-fluoro-3-nitrobenzoate in step a, isobutyraldehyde⁶⁹ in place of 2-(1-methyl-1*H*-imidazol-5-yl)quinoline-4-carbaldehyde in step c, and methylamine⁸⁴ (2 M solution in THF) in place of dimethylamine (2 M solution in THF) in step e. White solid. MS (ISP): 350.2 ([M+H]⁺).

### Example 31

### 1',3'-Dimethyl-2-(2-(1-methyl-1H-imidazol-5-yl)quinolin-4-yl)-1',3'-dihydro-2'H-[1,5'-bibenzo[d]imidazol]-2'-one

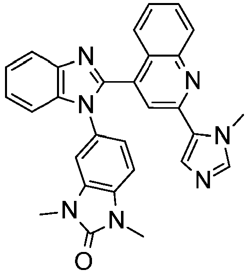

### a) 5-((2-Aminophenyl)amino)-1,3-dimethyl-1,3-dihydro-2H-benzo[d]imidazol-2-one

The title compound was obtained in analogy to example 1 using 1-fluoro-2-nitrobenzene⁴⁵ in place of 4-fluoro-*N*-methyl-3-nitrobenzamide and 5-amino-1,3-dimethyl-1*H*-benzo[d]imidazol-2(3*H*)-one⁵³ in place of 5-amino-1*H*-benzo[d]imidazol-2(3*H*)-one in step a. Amorphous brown solid. MS (ISP): 269.1 ([M+H]⁺).

### b) 1',3'-Dimethyl-2-(2-(1-methyl-1H-imidazol-5-yl)quinolin-4-yl)-1',3'-dihydro-2'H-[1,5'-bibenzo[dlimidazol]-2'-one

The title compound was obtained in analogy to example 2 using 5-((2-aminophenyl)amino)-1,3-dimethyl-1,3-dihydro-2*H*-benzo[d]imidazol-2-one in place of 3-amino-*N*-methyl-4-[(2-oxo-1,3-dihydrobenzimidazol-5-yl)amino]benzamide. White solid. MS (ISP): 486.2 ([M+H]⁺).

### Example 32 (provided for reference)

### 5-(2-Isopropyl-1H-imidazol-1-yl)-1,3-dihydro-2H-benzo[d]imidazol-2-one

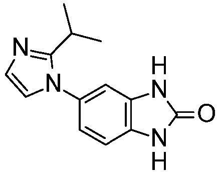

### a) 5-(2-Isopropyl-1H-imidazol-1-yl)-2-nitroaniline

To a solution of 2-isopropyl-1*H*-imidazole⁸⁵ (155 mg) in *N*,*N*-dimethylformamide (4 ml), was added NaH (56.4 mg) portionwise. After stirring for 15 min at room temperature, 5-fluoro-2-nitroaniline**^{Error! Bookmark not defined}**. (200 mg) was added and the reaction mixture was heated at 60 °C for 16 hours. The reaction mixture was diluted with ethyl acetate and washed with aq. sodium bicarbonate. The organic layer was dried over MgSO₄, filtered and concentrated *in vacuo.* The crude material was purified by flash chromatography (silica gel, eluent: 0 to 5% of methanol in dichloromethane) to afford 5-(2-isopropyl-1*H*-imidazol-1-yl)-2-nitroaniline (85mg, 27%) as an orange solid. MS (ISP): 247.2 ([M+H]⁺).

### b) 4-(2-Isopropyl-1H-imidazol-1-yl)benzene-1,2-diamine

To a stirred solution of 5-(2-isopropyl-1*H*-imidazol-1-yl)-2-nitroaniline (85 mg) in methanol (2 ml) was added 10% palladium on charcoal (37 mg). The reaction mixture was hydrogenated under an atmosphere of hydrogen. After stirring vigorously for 1 hour, the catalyst was collected by filtration, washing with methanol. The filtrate was then concentrated *in vacuo* to afford 4-(2-isopropyl-1*H*-imidazol-1-yl)benzene-1,2-diamine as a brown solid ( 61.8 mg, 83%) which was used in the next step without further purification. MS (ISP): 217.1 ([M+H]⁺).

### c) 5-(2-Isopropyl-1H-imidazol-1-yl)-1,3-dihydro-2H-benzo[d]imidazol-2-one

To a solution of 5-(2-isopropyl-1*H*-imidazol-1-yl)-1,3-dihydro-2*H*-benzo[d]imidazol-2-one (27.1 mg) in THF (1 ml) was added dropwise a solution of di(1*H*-imidazol-1-yl)methanone (93 mg) in THF (1 ml). The reaction mixture was stirred at room temperature for 1 hour. The light purple suspension was filtered and the solid was purified by preparative HPLC (Gemini NX, 12 nm, 5 µm, 100 x 30 mm, acetonitrile/water+0.1% triethylamine) to afford 5-(2-isopropyl-1*H-*imidazol-1-yl)-1,3-dihydro-2*H*-benzo[d]imidazol-2-one as a brown solid ( 27.1 mg, 39%). MS (ISP): 243.3 ([M+H]⁺).

### Example 33 (provided for reference)

### 5-(2-Oxopyrrolidin-1-yl)-1,3-dihydro-2H-benzo[d]imidazol-2-one

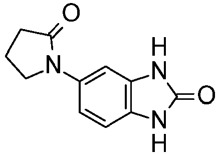

### a) 1-(3-Amino-4-nitro-phenyl)pyrrolidin-2-one

To a solution of 5-bromo-2-nitroaniline (200 mg) in anhydrous dioxane (500 µl) were successively added pyrrolidin-2-one⁸⁶ (94.1 mg), potassium carbonate (255 mg), *trans*-1, 2-cyclohexanediamine (105 mg) and copper (I) iodide (176 mg). The reaction mixture was heated at 110°C for 16 hours. After cooling to room temperature, the mixture was partitioned between water and ethyl acetate. The aqueous layer was extracted with additional ethyl acetate and the combined extracts were dried over anhydrous sodium sulfate, filtered, and concentrated *in vacuo.* The crude material was purified by flash chromatography (silica gel, eluent: 50 to 100% of ethyl acetate in heptane) to afford 1-(3-amino-4-nitro-phenyl)pyrrolidin-2-one (40 mg, 20%) as a yellow solid. MS (ISP): 222.2 ([M+H]⁺).

### b) 1-(3,4-Diaminophenyl)pyrrolidin-2-one

To a stirred solution of 1-(3-amino-4-nitro-phenyl)pyrrolidin-2-one (40 mg) in MeOH (2 ml) was added 10% palladium on charcoal (19.2 mg). The reaction mixture was hydrogenated under an atmosphere of hydrogen. After stirring vigorously for 1 hour, the catalyst was collected by filtration, washing with methanol. The filtrate was then concentrated *in vacuo* to afford 1-(3,4-diaminophenyl)pyrrolidin-2-one as a light yellow solid (34 mg, 98 %) which was used in the next step without further purification. MS (ISP): 192.1 ([M+H]⁺).

### c) 5-(2-Oxopyrrolidin-1-yl)-1,3-dihydro-2H-benzo[d]imidazol-2-one

To a solution of 1-(3,4-diaminophenyl)pyrrolidin-2-one (28.1 mg) in THF (548 µl) was added dropwise a solution of di(1H-imidazol-1-yl)methanone (57.7 mg) in THF (548 µl). The reaction mixture was stirred at room temperature for 16 hours. The suspension was filtered, then the solid was washed with THF and dried under high vacuum to afford 5-(2-oxopyrrolidin-1-yl)-1,3-dihydro-2H-benzo[d]imidazol-2-one as a light brown solid (28.1 mg, 73%). MS (ISP): 218.1 ([M+H]⁺).

### Example 34

### 2-Isopropyl-1'-methyl-1',3'-dihydro-2'H-[1,5'-bibenzo[d]imidazol]-2'-one

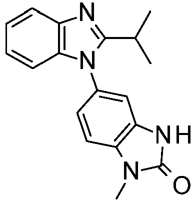

### a) 5-((2-Aminophenyl)amino)-1-methyl-1,3-dihydro-2H-benzo[d]imidazol-2-one

The title compound was obtained in analogy to example 1 using 5-amino-1-methyl-1,3-dihydro-2*H*-benzo[d]imidazol-2-one⁵¹ in place of 5-amino-1*H*-benzo[d]imidazol-2(3*H*)-one and 1-fluoro-2-nitrobenzene⁴⁵ in place of 4-fluoro-*N*-methyl-3-nitrobenzamide in step a. Dark brown solid. MS (ISP): 255.1 ([M+H]⁺).

### b) 2-Isopropyl-3'-methyl-1'3'-dihydro-2'H-[1,5'-bibenzo[d]imidazol]-2'-one

The title compound was obtained in analogy to example 2 using isobutyraldehyde⁶⁹ in place of 2-(1-methyl-1*H*-imidazol-5-yl)quinoline-4-carbaldehyde and 5-((2-aminophenyl)amino)-1-methyl-1,3-dihydro-2*H*-benzo[d]imidazol-2-one instead of 3-amino-*N*-methyl-4-[(2-oxo-1,3-dihydrobenzimidazol-5-yl)amino]benzamide. Orange foam (59.7 mg, 62 %). MS (ISP): 307.1 ([M+H]⁺).

### Example 35

### 2-Isopropyl-3'-methyl-1',3'-dihydro-2'H-[1,5'-bibenzo[d]imidazol]-2'-one

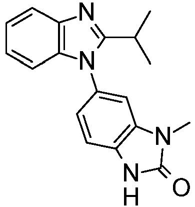

### a) 6-((2-Aminophenyl)amino)-1-methyl-1,3-dihydro-2H-benzo[d]imidazol-2-one

The title compound was obtained in analogy to example 1 using 6-amino-1-methyl-1,3-dihydro-2*H*-benzo[d]imidazol-2-one hydrochloride⁸⁷ in place of 5-amino-1*H*-benzo[d]imidazol-2(3*H*)-one and 1-fluoro-2-nitrobenzene⁴⁵ in place of 4-fluoro-*N*-methyl-3-nitrobenzamide in step a. Dark brown solid. MS (ISP): 255.1 ([M+H]⁺).

### b)2-Isopropyl-1'-methyl-1',3'-dihydro-2'H-[1,5'-bibenzo[d]imidazol]-2'-one

The title compound was obtained in analogy to example 2 using isobutyraldehyde⁶⁹ in place of 2-(1-methyl-1*H*-imidazol-5-yl)quinoline-4-carbaldehyde and 6-((2-aminophenyl)amino)-1-methyl-1,3-dihydro-2*H*-benzo[d]imidazol-2-one instead of 3-amino-*N*-methyl-4-[(2-oxo-1,3-dihydrobenzimidazol-5-yl)amino]benzamide. Orange foam (59.7 mg, 62 %). MS (ISP): 307.1 ([M+H]⁺).

### Example 36 (provided for reference)

### 6-(4-Fluoro-phenyl)-1,3-dihydro-imidazo[4,5-c]pyridin-2-one

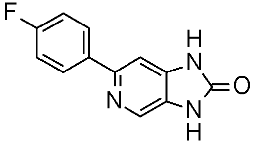

### a) Ethyl 4-azido-6-(4-fluorophenyl)pyridine-3-carboxylate

To a stirred solution of ethyl 4-chloro-6-(4-fluorophenyl)pyridine-3-carboxylate⁸⁸ (11.36 g) in DMF (115 ml) was added sodium azide (7.92 g). The reaction mixture was stirred at 70 °C for 2 hours. TLC analysis indicated that the reaction was finished. The reaction mixture was cooled to room temperature and the white solid was removed by filtration, washing with ethyl acetate. The filtrate was evaporated *in vacuo* and the residue was partitioned between ethyl acetate (100 ml) and water (300 ml). The aqueous phase was extracted twice with ethyl acetate (2 x 100 ml). The combined organic layers were dried over MgSO₄, filtered, and concentrated *in vacuo.* The crude material was purified by chromatography (silica gel, 300 g, eluent: 33 % of ethyl acetate in cyclohexane) to afford ethyl 4-azido-6-(4-fluorophenyl)pyridine-3-carboxylate (10.74 g, 93%) as an orange solid. MS (ISP): 286.1 ([M+H]⁺).

### b) 4-Azido-6-(4-fluorophenyl)pyridine-3-carboxylic acid

To a stirred solution of ethyl 4-chloro-6-(4-fluorophenyl)pyridine-3-carboxylate (10.37g) in ethanol (210 ml) at 0 ° C was added dropwise a solution of KOH (4.8 g) in water (23 ml). The reaction mixture was allowed to reach room temperature and stirring continued for 1 hour. The solvents were evaporated and the residue was dissolved in water (100 ml) and a solution of 1 M aq. HCl (100 ml) was added. The resulting white solid was collected by filtration, washing with water, and dried under high vacuum to afford 4-azido-6-(4-fluorophenyl)pyridine-3-carboxylic acid (8.82 g, 94%) as a white solid. MS (ISP): 257.1 ([M-H]⁻).

### c) tert-Butyl N-[4-azido-6-(4-fluorophenyl)-3-pyridyl]carbamate

To a stirred solution of 4-azido-6-(4-fluorophenyl)pyridine-3-carboxylic acid (8.80 g) and triethylamine (5.2 ml) in THF (215 ml) at -10 °C was added dropwise a solution of ethyl chloroformate (3.6 ml) in THF( 90 ml). After stirring for 30 min, a solution of sodium azide (11 g) in water (80 ml) was added dropwise at -10 ° C. The reaction mixture was allowed to reach room temperature and stirring continued 2.5 hours. The solvents were evaporated and the residue was suspended in water (200 ml). The yellow solid was collected by filtration, washing with water, and dissolved in a mixture of dichloromethane and THF. The organic solution was washed with sat. brine, dried over MgSO₄, filtered, and concentrated *in vacuo* to afford a yellow solid. This solid was dissolved in 1,2-dichloroethane (190 ml) and tert-butanol (80 ml) and the resulting solution was stirred at reflux for 2 hours. The reaction mixture was concentrated in vacuo and the crude material was purified by chromatography (silica gel, 600 g, eluent: 20 % to 33 % of ethyl acetate in cyclohexane) to afford *tert*-butyl *N*-[4-azido-6-(4-fluorophenyl)-3-pyridyl]carbamate (3.45g, 31%) as an orange solid. MS (ISP): 330.3 ([M+H]⁺).

### d) tert-Butyl N-[4-amino-6-(4-fluorophenyl)-3-pyridyl]carbamate

To a stirred solution of *tert*-butyl *N*-[4-azido-6-(4-fluorophenyl)-3-pyridyl]carbamate (3.4 g) in a mixture of MeOH (200 ml) and THF (60 ml) was added 10% palladium on charcoal (360 mg). The reaction mixture was stirred under a hydrogen atmosphere for 4 hours at room temperature. The catalyst was filtered off and the filter cake was washed with methanol. The filtrate was concentrated in *vacuo* to afford *tert*-butyl *N*-[4-amino-6-(4-fluorophenyl)-3-pyridyl]carbamate as a beige solid (3.28 g) which was used in the next step without further purification. MS (ISP): 304.2 ([M+H]⁺).

### e) 6-(4-Fluoro-phenyl)-1,3-dihydro-imidazo[4,5-c]pyridin-2-one

A stirred solution of *tert*-butyl *N*-[4-amino-6-(4-fluorophenyl)-3-pyridyl]carbamate (303 mg) in toluene (4 ml) was refluxed for 48 h. After cooling to room temperature, the resulting solid was collected by filtration, washing with toluene and diethyl ether, to afford 6-(4-fluoro-phenyl)-1,3-dihydro-imidazo[4,5-c]pyridin-2-one (170 mg, 74%) as a yellow solid. MS (ISP): 229.1 ([M+H]⁺).

### Example 37 (provided for reference)

### 5-(2,5-Difluoro-phenyl)-1,3-dihydro-benzoimidazol-2-one

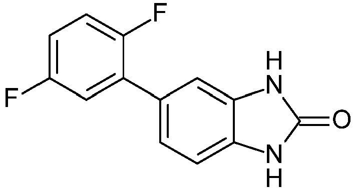

The title compound was obtained in analogy to example 36 step e using *tert*-butyl *N*-[2-amino-4-(2,5-difluorophenyl)phenyl]carbamate ⁸⁹ in place of *tert*-butyl *N*-[4-amino-6-(4-fluorophenyl)-3-pyridyl]carbamate. Beige solid. MS (ISP): 247.2 ([M+H]⁺).

### Example 38 (provided for reference)

### 5-(2,4-Difluoro-phenyl)-1,3-dihydro-benzoimidazol-2-one

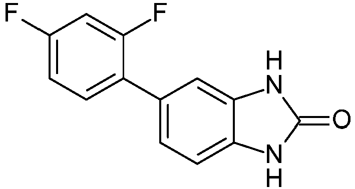

The title compound was obtained in analogy to example 36 step e using *tert*-butyl *N*-[2-amino-4-(2,4-difluorophenyl)phenyl]carbamate ⁹⁰ in place of *tert*-butyl *N*-[4-amino-6-(4-fluorophenyl)-3-pyridyl]carbamate. Beige solid. MS (ISP): 247.2 ([M+H]⁺).

### Example 39 (provided for reference)

### 5-[(4-Methoxyphenyl)methyl]-1,3-dihydrobenzimidazol-2-one

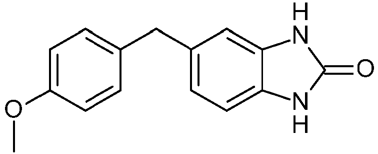

The title compound was obtained in analogy to example 19 using*p*-methoxybenzylboronic acid⁹¹ in place of pyridin-3-ylboronic acid. White solid. MS (ISP): 255.3 ([M+H]⁺).

### Example 40 (provided for reference)

### 5-[4-(2-Pyridyl)piperazin-1-yl]-1,3-dihydroimidazo[4,5-b]pyridin-2-one

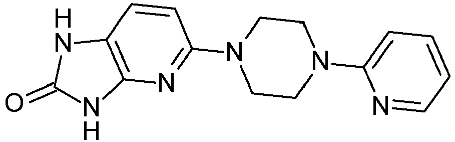

The title compound was obtained in analogy to example 58 step c using 6-[4-(2-pyridyl)piperazin-1-yl]pyridine-2,3-diamine ⁹² in place of *tert-*butyl 4-(3,4-diaminophenyl)piperazine-1-carboxylate. White solid. MS (ISP): 297.3 ([M+H]⁺).

### Example 41 (provided for reference)

### 5-(4-Pyrrol-1-yl-phenyl)-1,3-dihydro-benzoimidazol-2-one

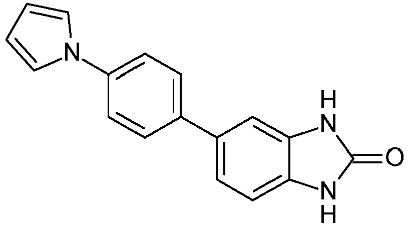

The title compound was obtained in analogy to example 19 using *B*-[4-(1*H*-pyrrol-1-yl)phenyl]boronic acid⁹³ in place of pyridin-3-ylboronic acid. White solid. MS (ISP): 276.1 ([M+H]⁺).

### Example 42 (provided for reference)

### 5-Quinolin-5-yl-1,3-dihydro-benzoimidazol-2-one

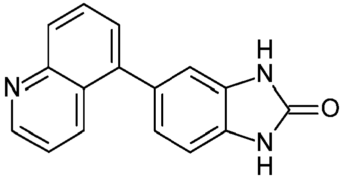

To a stirred solution of 5-bromo-1*H*-benzo[d]imidazol-2(3*H*)-one⁹⁴ (930 mg) in a mixture of dioxane (47 ml) and 1 M aqueous sodium carbonate (24 ml) was added 5-quinolylboronic acid⁹⁵ (906 mg). The reaction mixture was purged three times with Argon before adding 2-dicyclohexylphosphino-2',6'-dimethoxybiphenyl (0.358 g) and palladium(II) acetate (0.098 g). The reaction mixture was refluxed for 4 hours. Dioxane was removed by evaporation and ethyl acetate was added. The solid obtained was filtered, dissolved in dichloromethane and washed with water. The organic layer was dried over MgSO₄, filtered and concentrated *in vacuo.* The crude material was purified by flash chromatography (silica gel, eluent: 0 to 5% of methanol in dichloromethane) to afford 5-(2-isopropyl-1*H*-imidazol-1-yl)-2-nitroaniline (85mg, 27%) as an orange solid. MS (ISP): 247.2 ([M+H]⁺). The organic layer off and the filter cake was washed with methanol. The filtrate was concentrated in *vacuo* to 5-quinolin-5-yl-1,3-dihydro-benzoimidazol-2-one as a dark brown solid (949 mg). MS (ISP): 262.2 ([M+H]⁺).

### Example 43 (provided for reference)

### Ethyl 4-((2-oxo-2,3-dihydro-1H-benzo[d]imidazole)-5-sulfonamido)benzoate

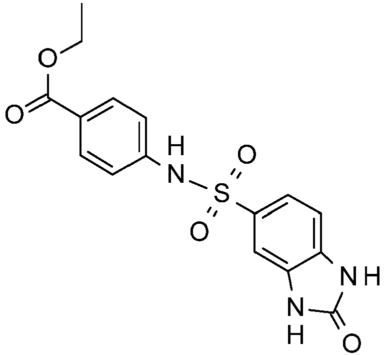

To a solution of ethyl 4-aminobenzoate⁹⁶ (35.5 mg) in pyridine (0.5 ml) was added 2-oxo-2,3-dihydro-1*H*-benzo[d]imidazole-5-sulfonyl chloride**^{Error! Bookmark not defined}**. (50 mg) and the mixture was stirred at room temperature for 2 h. LC-MS analysis indicated that the reaction was finished. The reaction mixture was partitioned between ethyl acetate/THF (1:3, 30 ml) and 2 M aqueous HCl (20 ml). The layers were separated. The aqueous layer was extracted with ethyl acetate/THF (1:1, 20 ml). The combined organic layers were washed with saturated brine (15 ml), dried over anhydrous sodium sulfate, and concentrated *in vacuo.* The crude material was purified by flash chromatography (SiO₂, eluant: dichloromethane/methanol 100:0 to 90:10) to afford ethyl 4-((2-oxo-2,3-dihydro-1*H*-benzo[d]imidazole)-5-sulfonamido)benzoate as an off-white solid. MS (ISP): 360.1 ([M-H]⁻).

### Example 44 (provided for reference)

### 1-Methyl-5-phenyl-3H-imidazo[4,5-b]pyridin-2-one

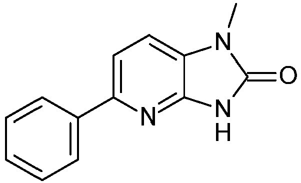

The title compound was obtained in analogy to example 19 using phenylboronic acid⁹⁷ in place of pyridin-3-ylboronic acid and 5-chloro-1,3-dihydro-1-methyl-2*H*-imidazo[4,5-b]pyridin-2-one⁹⁸ in place of 5-bromo-1*H*-benzo[d]imidazol-2(3*H*)-one. White solid. MS (ISP): 226.2 ([M+H]⁺).

### Example 45

### 2-(2-Methoxybenzyl)-N-methyl-2'-oxo-2',3'-dihydro-1'H-[1,5'-bibenzo[d]imidazole]-5-carboxamide

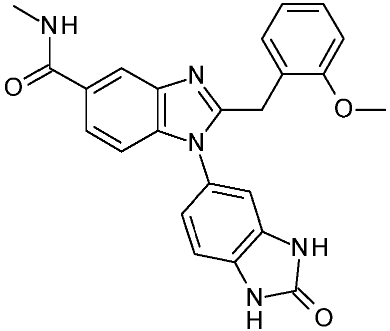

The title compound was obtained in analogy to example 2 using 2-(2-methoxyphenyl)acetaldehyde⁹⁹ in place of 2-(3-methylimidazol-4-yl)quinoline-4-carbaldehyde. Off-white solid. MS (ISP): 428.4 ([M+H]⁺).

### Example 46

### 2-(tert-Butyl)-N-methyl-2'-oxo-2',3'-dihydro-1'H-[1,5'-bibenzo[d]imidazole]-5-carboxamide

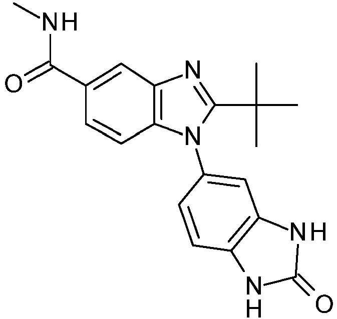

The title compound was obtained in analogy to example 2 using pivaldehyde¹⁰⁰ in place of 2-(3-methylimidazol-4-yl)quinoline-4-carbaldehyde. White solid. MS (ISP): 364.2 ([M+H]⁺).

### Example 47

### Methyl 2-isopropyl-2'-oxo-2',3'-dihydro-1'H-[1,5'-bibenzo[d]imidazole]-4-carboxylate

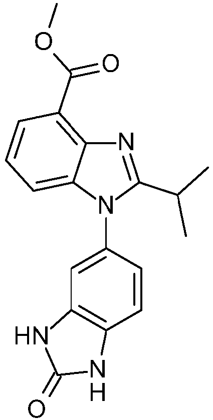

### a) Methyl 2-amino-3-((2-oxo-2,3-dihydro-1H-benzo[d]imidazol-5-yl)amino)benzoate

The title compound was obtained in analogy to example 1 using methyl 3-fluoro-2-nitrobenzoate¹⁰¹ in place of 4-fluoro-N-methyl-3-nitrobenzamide in step a. Yellow solid. MS (ISP): 299.1 ([M+H]⁺).

### b) Methyl 2-isopropyl-2'-oxo-2',3'-dihydro-1'H-[1,5'-bibenzo[d]imidazole]-4-carboxylate

The title compound was obtained in analogy to example 2 using isobutyraldehyde⁶⁹ in place of 2-(3-methylimidazol-4-yl)quinoline-4-carbaldehyde and methyl 2-amino-3-((2-oxo-2,3-dihydro-1*H*-benzo[d]imidazol-5-yl)amino)benzoate in place of 3-amino-*N*-methyl-4-[(2-oxo-1,3-dihydrobenzimidazol-5-yl)amino]benzamide. White solid. MS (ISP): 351.1 ([M+H]⁺).

### Example 48

### Methyl 2-isopropyl-2'-oxo-2',3'-dihydro-1'H-[1,5'-bibenzo[d]imidazole]-6-carboxylate

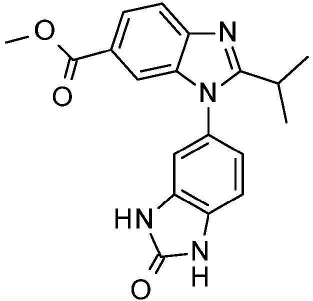

### a) Methyl 4-amino-3-((2-oxo-2,3-dihydro-1H-benzo[d]imidazol-5-yl)amino)benzoate

The title compound was obtained in analogy to example 1 using methyl 3-fluoro-4-nitrobenzoate⁴⁹ in place of 4-fluoro-*N*-methyl-3-nitrobenzamide in step a. Brown solid. MS (ISP): 299.1 ([M+H]⁺).

### b) Methyl 2-isopropyl-2'-oxo-2',3'-dihydro-1'H-[1,5'-bibenzo[d]imidazole]-6-carboxylate

The title compound was obtained in analogy to example 2 using isobutyraldehyde⁶⁹ in place of 2-(3-methylimidazol-4-yl)quinoline-4-carbaldehyde and methyl 4-amino-3-((2-oxo-2,3-dihydro-1*H*-benzo[d]imidazol-5-yl)amino)benzoate in place of 3-amino-*N*-methyl-4-[(2-oxo-1,3-dihydrobenzimidazol-5-yl)amino]benzamide. White solid. MS (ISP): 351.1 ([M+H]⁺).

### Example 49 (provided for reference)

### 1,3-Dimethyl-5-((2-nitrophenyl)amino)-1,3-dihydro-2H-benzo[d]imidazol-2-one

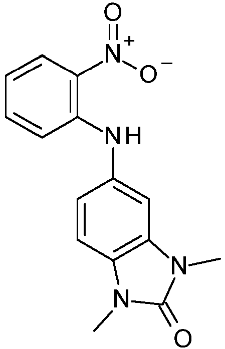

The title compound was obtained in analogy to example 1 step a using 1-fluoro-2-nitrobenzene⁴⁵ in place of 4-fluoro-N-methyl-3-nitrobenzamide and 5-amino-1,3-dimethyl-1,3-dihydro-2*H*-benzo[d]imidazol-2-one⁵³ in place of 5-amino-1*H*-benzo[d]imidazol-2(3*H*)-one. Red solid. MS (ISP): 299.1 ([M+H]⁺).

### Example 50 (provided for reference)

### 5-((2-Aminophenyl)amino)-1,3-dimethyl-1,3-dihydro-2H-benzo[d]imidazol-2-one

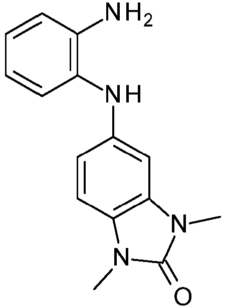

The title compound was obtained in analogy to example 1 step b using 1,3-dimethyl-5-((2-nitrophenyl)amino)-1,3-dihydro-2*H*-benzo[d]imidazol-2-one in place of *N*-methyl-3-nitro-4-((2-oxo-2,3-dihydro-1*H*-benzo[d]imidazol-5-yl)amino)benzamide. Amorphous brown solid. MS (ISP): 269.1 ([M+H]⁺).

### Example 51 (provided for reference)

### 2-Oxo-N-(4-piperidyl)-1,3-dihydrobenzimidazole-5-carboxamide

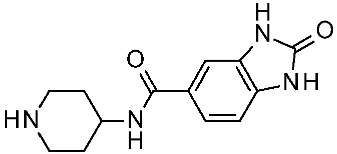

### a) tert-Butyl 4-[(2-oxo-1,3-dihydrobenzimidazole-5-carbonyl)amino]piperidine-1-carboxylate

The title compound was obtained in analogy to example 22 using tert-butyl *4-*aminopiperidine-1-carboxylate¹⁰² in place of 1-(piperazin-1-yl)ethanone. White solid. MS (ISP): 305.1 ([M+H-C₄H₈]⁺).

### b) 2-Oxo-N-(4-piperidyl)-1,3-dihydrobenzimidazole-5-carboxamide

To a stirred suspension of *tert-*butyl 4-[(2-oxo-1,3-dihydrobenzimidazole-5-carbonyl)amino]piperidine-1-carboxylate (54 mg) in dichloromethane (2 ml) was added dropwise a solution of 4 M HCl in dioxane (1.1 ml). The reaction mixture was stirred at room temperature overnight. The reaction mixture was then concentrated *in vacuo.* Diethyl ether was added and the resulting mixture was ultrasonicated. The mixture was then filtered through sintered glass, and the filter cake was dried *in vacuo* to afford 2-oxo-*N*-(4-piperidyl)-1,3-dihydrobenzimidazole-5-carboxamide as its hydrochloride salt (39 mg, 88%) as a white solid. MS (ISP): 261.1 ([M+H-C₄H₈]⁺).

### Example 52 (provided for reference)

### 6-((2-Aminophenyl)amino)-1-methyl-1,3-dihydro-2H-benzo[d]imidazol-2-one

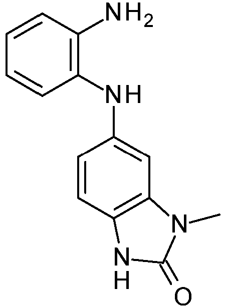

The title compound was obtained in analogy to example 1 using 1-fluoro-2-nitrobenzene⁴⁵ in place of 4-fluoro-*N*-methyl-3-nitrobenzamide and 6-amino-1-methyl-1,3-dihydro-2*H-*benzo[d]imidazol-2-one⁸⁷ instead of 5-amino-1*H*-benzo[d]imidazol-2(3*H*)-one in step a. Brown solid. MS (ISP): 285.2 ([M+H]⁺).

### Example 53 (provided for reference)

### 5-((2-Aminophenyl)amino)-1-methyl-1,3-dihydro-2H-benzo[d]imidazol-2-one

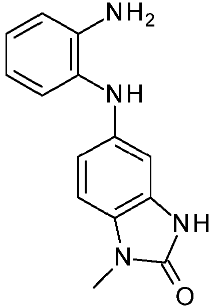

The title compound was obtained in analogy to example 1 using 1-fluoro-2-nitrobenzene⁴⁵ in place of 4-fluoro-*N*-methyl-3-nitrobenzamide and 5-amino-1-methyl-1,3-dihydro-2*H-*benzo[d]imidazol-2-one hydrochloride¹⁰³ instead of 5-amino-1*H*-benzo[*d*]imidazol-2(3*H*)-one in step a. Brown solid. MS (ISP): 255.3 ([M+H]⁺).

### Example 54 (provided for reference)

### Methyl 1-(2-oxo-2,3-dihydro-1H-benzo[d]imidazole-5-carbonyl)indoline-4-carboxylate

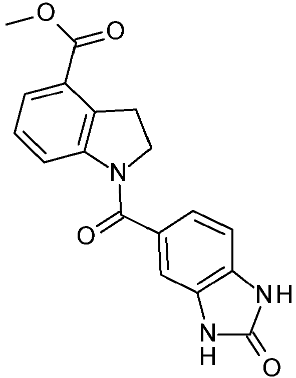

To a stirred solution of 2-oxo-2,3-dihydro-1*H*-benzo[d]imidazole-5-carboxylic acid**^{Error! Bookmark not defined}.** (150 mg) and methyl indoline-4-carboxylate¹⁰⁴ (149 mg) in methanol (5 ml) was added 4-(4,6-dimethoxy-1,3,5-triazin-2-yl)-4-methylmorpholin-4-ium chloride ¹⁰⁵ (280 mg, DMTMM). The reaction muxture was stirred for 4 h at room temperature. LC/MS analysis indicated that the reaction was complete. The reaction mixture was concentrated *in vacuo* and the residue was purified by preparative reverse-phase HPLC (Gemini NX C18, 12 nm, 5µ, 100 x 30 mm, flow rate 40 ml/min, eluant: CH₃CN/H₂O containing formic acid) followed by lyophilisation *in vacuo* to afford methyl 1-(2-oxo-2,3-dihydro-1*H*-benzo[d]imidazole-5-carbonyl)indoline-4-carboxylate (60 mg, 21%) as a white solid. MS (ISP): 338.1 ([M+H]⁺).

### Example 55 (provided for reference)

### 6-(Indoline-1-carbonyl)-1,3-dihydro-2H-imidazo[4,5-b]pyridin-2-one

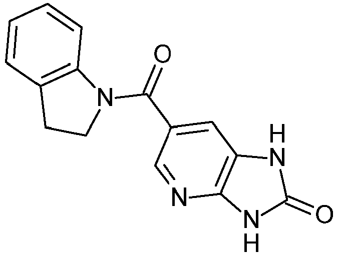

The title compound was obtained in analogy to example 22 using indoline⁷⁶ in place of 1-(piperazin-1-yl)ethanone and 2-oxo-2,3-dihydro-1*H*-imidazo[4,5-b]pyridine-6-carboxylic acid¹⁰⁶ in place of 2-oxo-2,3-dihydro-1*H*-benzo[d]imidazole-5-carboxylic acid. White solid. MS (ISP): 281.1 ([M+H]⁺).

### Example 56 (provided for reference)

### N-(1-Acetylpiperidin-4-yl)-2-oxo-2,3-dihydro-1H-imidazo[4,5-b]pyridine-6-carboxamide

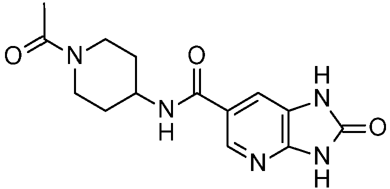

The title compound was obtained in analogy to example 22 using 1-(4-aminopiperidin-1-yl)ethan-1-one⁷⁵ in place of 1-(piperazin-1-yl)ethanone and 2-oxo-2,3-dihydro-1*H*-imidazo[4,5-b]pyridine-6-carboxylic acid¹⁰⁶ in place of 2-oxo-2,3-dihydro-1*H*-benzo[d]imidazole-5-carboxylic acid. White solid. MS (ISP): 304.1 ([M+H]⁺).

### Example 57

### 2-(3-(Methoxymethyl)phenyl)-N-methyl-2'-oxo-2',3'-dihydro-1'H-[1,5'-bibenzo[d]imidazole]-5-carboxamide

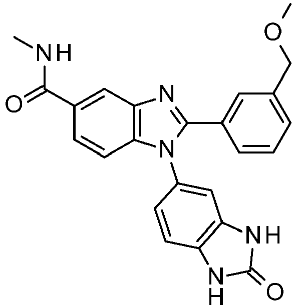

The title compound was obtained in analogy to example 2 using 3-(methoxymethyl)benzaldehyde¹⁰⁷ in place of 2-(3-methylimidazol-4-yl)quinoline-4-carbaldehyde. White solid. MS (ISP): 428.2 ([M+H]⁺).

### Example 58 (provided for reference)

### 5-(Piperazin-1-yl)-1,3-dihydro-2H-benzo[d]imidazol-2-one

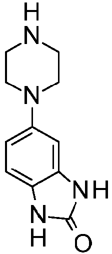

### a) tert-Butyl 4-(3,4-dinitrophenyl)piperazine-1-carboxylate

A mixture of 4-fluoro-1,2-dinitrobenzene**^{Error! Bookmark not defined}.** (250 mg), *tert*-butyl piperazine-1-carboxylate¹⁰⁸ (275 mg) and *N*,*N*-diisopropylethylamine (351 µl) in acetonitrile (2 ml) was stirred at room temperature overnight. Pentane (1 ml) was added and the suspension was filtered. The filter cake was dried *in vacuo* to afford *tert*-butyl 4-(3,4-dinitrophenyl)piperazine-1-carboxylate (385 mg, 81%) as a yellow solid. MS (ISP): 353.3 ([M+H]⁺).

### b) tert-Butyl 4-(3,4-diaminophenyl)piperazine-1-carboxylate

A mixture of *tert*-butyl 4-(3,4-dinitrophenyl)piperazine-1-carboxylate (385 mg) and 10% Pd/C (116 mg) in THF (1 ml) and Ethanol (1 ml) was stirred at room temperature overnight under a hydrogen atmosphere. The mixture was then filtered through a membrane filter and the filtrate was concentrated *in vacuo* to afford tert-butyl 4-(3,4-diaminophenyl)piperazine-1-carboxylate (237 mg, 74%) as an off-white solid. MS (ISP): 293.3 ([M+H]⁺).

### c) tert-Butyl 4-(2-oxo-2,3-dihydro-1H-benzo[d]imidazol-5-yl)piperazine-1-carboxylate

To a stirred solution of *tert*-butyl 4-(3,4-diaminophenyl)piperazine-1-carboxylate (230 mg) in THF (2 ml) was added CDI (140 mg). The reaction mixture was stirred at room temperature for 1 hour. LC-MS analysis indicated that no starting material was left. The reaction mixture was diluted with water (40 ml) and extracted twice with ethyl acetate (2 x 30 ml). The combined organic layers were dried over Na₂SO₄, filtered, and concentrated *in vacuo.* The residue was purified by preparative HPLC to afford *tert-butyl* 4-(2-oxo-2,3-dihydro-1*H*-benzo[d]imidazol-5-yl)piperazine-1-carboxylate (57.7 mg, 23%) as an off-white solid. MS (ISP): 319.3 ([M+H]⁺).

### d) 5-(Piperazin-1-yl)-1,3-dihydro-2H-benzo[d]imidazol-2-one

To a suspension of *tert*-butyl 4-(2-oxo-2,3-dihydro-1*H*-benzo[d]imidazol-5-yl)piperazine-1-carboxylate (65 mg) in 1,4-dioxane (1.5 ml) at room temperature was added a 4 M solution of hydrogen chloride in 1,4-dioxane (1 ml). The reaction mixture was stirred at room temperature for 3 h. LC-MS analysis indicated that the reaction was finished. The solvent was concentrated *in vacuo* to afford 5-(piperazin-1-yl)-1,3-dihydro-2*H*-benzo[d]imidazol-2-one (52 mg, 100 %) as its hydrochloride salt as a white solid. MS (ISP): 219.1 ([M+H]⁺).

### Example 59 (provided for reference)

### 5-[rac-(1S,5R)-3,8-diazabicyclo[3.2.1]octan-3-yl]-1,3-dihydrobenzimidazol-2-one

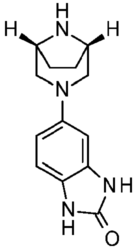

The title compound was obtained as its hydrochloride salt in analogy to example 58 using *tert*-butyl *rac*-(1*S*,5*R*)-3,8-diazabicyclo[3.2.1]octane-8-carboxylate ¹⁰⁹ in place of *tert-*butyl piperazine-1-carboxylate in step a. White solid. MS (ISP): 245.1 ([M+H]⁺).

### Example 60

### 2-Isopropyl-N-methyl-2'-oxo-2',3'-dihydro-1'H-[1,5'-bibenzo[d]imidazole]-4-carboxamide

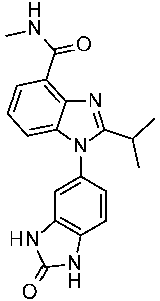

### a) 2-Isopropyl-2'-oxo-2',3'-dihydro-1'H-[1,5'-bibenzo[d]imidazole]-4-carboxylic acid

The title compound was obtained in analogy to example 18 step d using methyl 2-isopropyl-2'-oxo-2',3'-dihydro-1'*H-*[1,5'-bibenzo[d]imidazole]-4-carboxylate (example 47) in place of methyl 2-(2-(1-methyl-1*H*-imidazol-5-yl)quinolin-4-yl)-2'-oxo-2',3'-dihydro-1'*H*-[1,5'-bibenzo[d]imidazole]-5-carboxylate and THF in place of 1,4-dioxane. White solid. MS (ISP): 337.1 ([M+H]⁺).

### b) 2-Isopropyl-N-methyl-2'-oxo-2'3'-dihydro-1'H-[1,5'-bibenzor[d]imidazole]-4-carboxamide

The title compound was obtained in analogy to example 18 step e using 2-isopropyl-2'-oxo-2',3'-dihydro-1'*H*-[1,5'-bibenzo[d]imidazole]-4-carboxylic acid in place of 2-(2-(1-methyl-1*H*-imidazol-5-yl)quinolin-4-yl)-2'-oxo-2',3'-dihydro-1'*H*-[1,5'-bibenzo[d]imidazole]-5-carboxylic acid and methylamine in place of dimethylamine. White solid. MS (ISP): 350.2 ([M+H]⁺).

### Example 61

### 5-[2-(Hydroxymethyl)benzimidazol-1-yl]-1,3-dihydrobenzimidazol-2-one

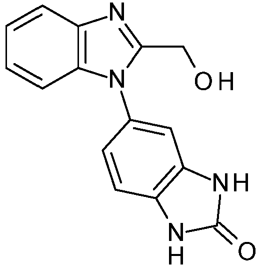

### a) 5-(2-Aminoanilino)-1,3-dihydrobenzimidazol-2-one

The title compound was obtained in analogy to example 1 using 1-fluoro-2-nitrobenzene⁴⁵ in place of 4-fluoro-*N*-methyl-3-nitrobenzamide in step a. Grey solid. MS (ISP): 241.2 ([M+H]⁺).

### b) 5-[2-(Hydroxymethyl)benzimidazol-1-yl]-1,3-dihydrobenzimidazol-2-one

A solution of 5-(2-aminoanilino)-1,3-dihydrobenzimidazol-2-one (250 mg) and glycolic acid¹¹⁰ (158 mg) in a 6 M solution of HCl (8 ml) was stirred at120 °C for 48 hours. According to LC/MS, the reaction was finished. The reaction mixture was concentrated *in vacuo.* The crude material was purified by preparative reversed phase HPLC to afford 5-[2-(hydroxymethyl)benzimidazol-1-yl]-1,3-dihydrobenzimidazol-2-one (170 mg, 57.9%) as a brown solid. MS (ISP): 281.2 ([M+H]⁺).

### Example 62 (provided for reference)

### 5-(4-Acetylpiperazin-1-yl)-1,3-dihydro-2H-benzo[d]imidazol-2-one

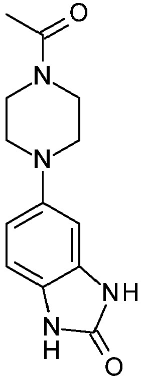

To a stirred solution of 5-(piperazin-1-yl)-1,3-dihydro-2*H*-benzo[d]imidazol-2-one hydrochloride (50 mg, example 59) and triethylamine (82 µl) in DMF (1 ml) at room temperature was added dropwise acetic anhydride (20 mg). The reaction mixture was stirred at room temperature for 2 h. LC/MS analysis indicated that the reaction was complete. The reaction mixture was poured into water (10 ml) and extracted with 1:4 EtOAc/THF (2 x 30 ml). The combined organic phases were dried over Na₂S=4 and concentrated *in vacuo.* The crude material was purified by preparative HPLC followed by lyophilisation to afford 5-(4-acetylpiperazin-1-yl)-1,3-dihydro-2H-benzo[d]imidazol-2-one (23 mg, 45%) as a white solid. MS (ISP): 261.1 ([M+H]⁺).

### Example 63 (provided for reference)

### 5-[rac-(1S,5R)-8-Acetyl-3,8-diazabicyclo[3.2.1]octan-3-yl]-1,3-dihydrobenzimidazol-2-one

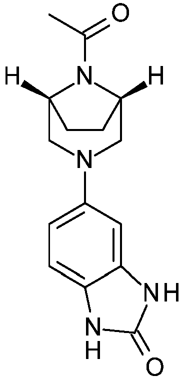

The title compound was obtained in analogy to example 62 using 5-[*rac*-(1*S*,5*R*)-3,8-diazabicyclo[3.2.1]octan-3-yl]-1,3-dihydrobenzimidazol-2-one hydrochloride in place of 5-(piperazin-1-yl)-1,3-dihydro-2*H*-benzo[d]imidazol-2-one hydrochloride. White solid. MS (ISP): 287.2 ([M+H]⁺).

### Example 64

### N-Methyl-2'-oxo-2-(tetrahydro-2H-pyran-4-yl)-2',3'-dihydro-1'H-[1,5'-bibenzo[d]imidazole]-5-carboxamide

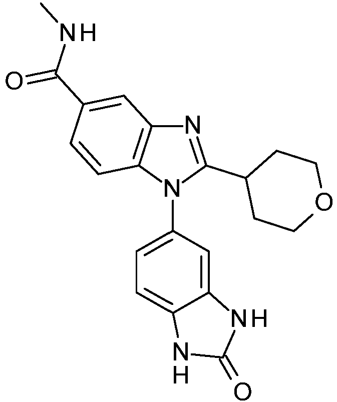

The title compound was obtained in analogy to example 2 using tetrahydro-2*H*-pyran-4-carbaldehyde¹¹¹ in place of 2-(3-methylimidazol-4-yl)quinoline-4-carbaldehyde. White solid. MS (ISP): 392.2 ([M+H]⁺).

### Example 65

### N-Methyl-2-(1-methyl-1H-imidazol-4-yl)-2'-oxo-2',3'-dihydro-1'H-[1,5'-bibenzo[d]imidazole]-5-carboxamide

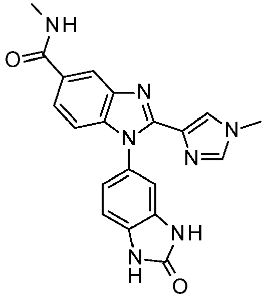

The title compound was obtained in analogy to example 2 using 1-methyl-1*H*-imidazole-4-carbaldehyde¹¹² in place of 2-(3-methylimidazol-4-yl)quinoline-4-carbaldehyde. White solid. MS (ISP): 388.2 ([M+H]⁺).

### Example 66

### N-Methyl-2'-oxo-2-(1H-pyrrol-3-yl)-2',3'-dihydro-1'H-[1,5'-bibenzo[d]imidazole]-5-carboxamide

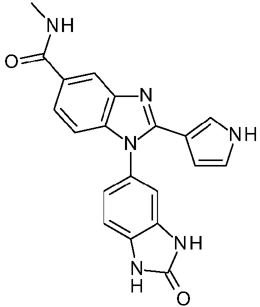

The title compound was obtained in analogy to example 2 using 1*H*-pyrrole-3-carbaldehyde¹¹³ in place of 2-(3-methylimidazol-4-yl)quinoline-4-carbaldehyde. White solid. MS (ISP): 373.1 ([M+H]⁺).

### Example 67 (provided for reference)

### 2-Oxo-N-phenyl-2,3-dihydro-1H-imidazo[4,5-b]pyridine-6-carboxamide

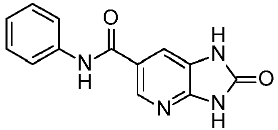

To a mixture of aniline (50 mg) and 2-oxo-2,3-dihydro-1*H*-imidazo[4,5-b]pyridine-6-carboxylic acid¹⁰⁶ (106 mg) were added 0.36 M HATU in *N*,*N*-dimethylformamide (245 mg,1.8 ml) and *N,N*-diisopropylethylamine (276 µl). The reaction mixture was shaken at 25 °C for 2 hours and was then partitioned between water and a 1:1 mixture of ethyl acetate/THF. The organic layer was washed with brine, dried over Na₂SO₄, filtered and concentrated *in vacuo.* The product was suspended in dichloromethane (3 ml) and stirred. The product was collected by filtration, washed with dichloromethane and dried to afford 2-oxo-N-phenyl-2,3-dihydro-1*H*-imidazo[4,5-b]pyridine-6-carboxamide (29.4 mg, 22 %) as an off-white solid. MS (ISP): 255.3 ([M+H]⁺).

### Example 68 (provided for reference)

### N-(2-Oxo-2,3-dihydro-1H-benzo[d]imidazol-5-yl)benzofuran-3-carboxamide

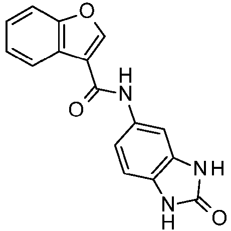

To a stirred solution of benzofuran-3-carboxylic acid¹¹⁴ (40 mg), 5-amino-1,3-dihydro-2*H-*benzo[d]imidazol-2-one⁵⁰ (36.8 mg) and *N*,*N*-diisopropylethylamine (172 µl) in DMF (1 ml) was added HATU (141 mg). The reaction mixture was stirred at room temperature for 2 h. The reaction mixture was then poured into EtOAc/THF (1:1) and extracted with saturated brine. The organic layer was separated, dried over Na₂SO₄, and concentrated *in vacuo.* The crude material was purified by preparative reverse-phase HPLC (Gemini NX C18, 12 nm, 5µ, 100 x 30 mm, flow rate 40 ml/min, eluant: CH₃CN/H₂O containing formic acid) followed by lyophilisation to afford *N*-(2-oxo-2,3-dihydro-1*H*-benzo[d]imidazol-5-yl)benzofuran-3-carboxamide (15 mg, 21%) as an off-white solid. MS (ISP): 294.1 ([M+H]⁺).

### Example 69 (provided for reference)

### N-(2-Oxo-2,3-dihydro-1H-benzo[d]imidazol-5-yl)-2,3-dihydro-1H-indene-1-carboxamide

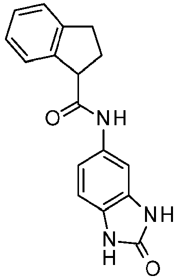

The title compound was obtained in analogy to example 68 using 2,3-dihydro-1*H*-indene-1-carboxylic acid¹¹⁵ in place of benzofuran-3-carboxylic acid. Off-white solid. MS (ISP): 294.1 ([M+H]⁺).

### Example 70 (provided for reference)

### 2-(4-Aminophenyl)-N-(2-oxo-2,3-dihydro-1H-benzo[d]imidazol-5-yl)acetamide

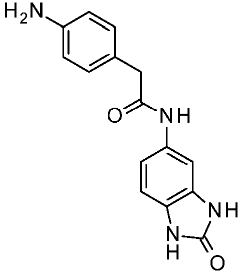

The title compound was obtained in analogy to example 68 using 2-(4-aminophenyl)acetic acid¹¹⁶ in place of benzofuran-3-carboxylic acid. Off-white solid. MS (ISP): 283.1 ([M+H]⁺).

### Example 71 (provided for reference)

### (S)-N-(2-Oxo-2,3-dihydro-1H-benzo[d]imidazol-5-yl)-1,2,3,4-tetrahydronaphthalene-1-carboxamide

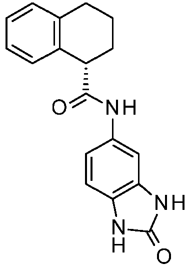

The title compound was obtained in analogy to example 68 using (S)-1,2,3,4-tetrahydronaphthalene-1-carboxylic acid¹¹⁷ in place of benzofuran-3-carboxylic acid. White solid. MS (ISP): 308.1 ([M+H]⁺).

### Example 72 (provided for reference)

### (R)-N-(2-Oxo-2,3-dihydro-1H-benzo[d]imidazol-5-yl)-1,2,3,4-tetrahydronaphthalene-1-carboxamide

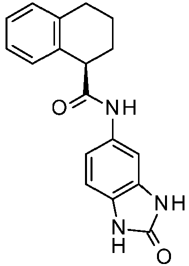

The title compound was obtained in analogy to example 68 using (*R*)-1,2,3,4-tetrahydronaphthalene-1-carboxylic acid¹¹⁸ in place of benzofuran-3-carboxylic acid. White solid. MS (ISP): 308.1 ([M+H]⁺).

### Example 73 (provided for reference)

### N-(2-Oxo-2,3-dihydro-1H-benzo[d]imidazol-5-yl)benzamide

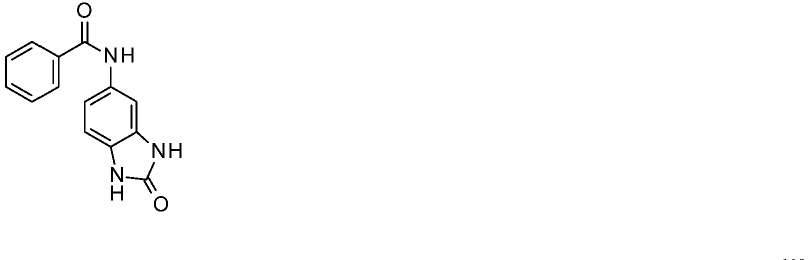

The title compound was obtained in analogy to example 68 using benzoic acid¹¹⁹ in place of benzofuran-3-carboxylic acid. White solid. MS (ISP): 254.1 ([M+H]⁺).

### Example 74 (provided for reference)

### 4-Acetamido-N-(2-oxo-2,3-dihydro-1H-benzo[d]imidazol-5-yl)benzamide

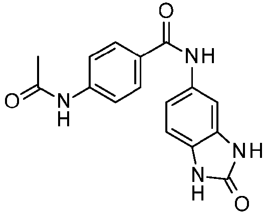

The title compound was obtained in analogy to example 68 using 4-acetamidobenzoic acid¹²⁰ in place of benzofuran-3-carboxylic acid. Off-white solid. MS (ISP): 311.1 ([M+H]⁺).

### Example 75 (provided for reference)

### 3-Acetamido-N-(2-oxo-2,3-dihydro-1H-benzo[d]imidazol-5-yl)benzamide

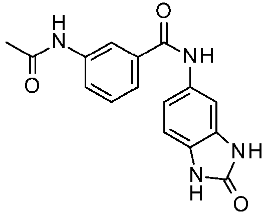

The title compound was obtained in analogy to example 68 using 3-acetamidobenzoic acid¹²¹ in place of benzofuran-3-carboxylic acid. White solid. MS (ISP): 311.1 ([M+H]⁺).

### Example 76 (provided for reference)

### N-Methyl-2-oxo-N-phenyl-2,3-dihydro-1H-imidazo[4,5-b]pyridine-6-carboxamide

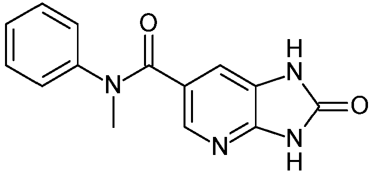

The title compound was obtained in analogy to example 22 using *N*-methylaniline¹²² in place of 1-(piperazin-1-yl)ethanone and 2-oxo-2,3-dihydro-1*H*-imidazo[4,5-b]pyridine-6-carboxylic acid instead of 2-oxo-2,3-dihydro-1*H*-benzo[d]imidazole-5-carboxylic acid. White solid. MS (ISP): 269.3 ([M+H]⁺).

### Example 77¹²³ (provided for reference)

### 2-Oxo-N-phenyl-2,3-dihydro-1H-benzo[d]imidazole-5-carboxamide

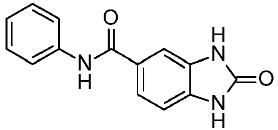

The title compound can be purchased.

### Example 78 (provided for reference)

### 2-(4-Acetamidophenyl)-N-(2-oxo-2,3-dihydro-1H-benzo[d]imidazol-5-yl)acetamide

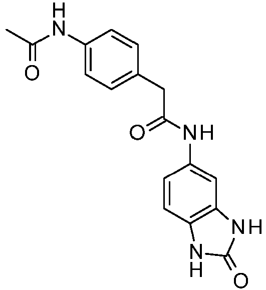

The title compound was obtained in analogy to example 68 using 2-(4-acetamidophenyl)acetic acid¹²⁴ in place of benzofuran-3-carboxylic acid. White solid. MS (ISP): 325.1 ([M+H]⁺).

### Example 79

### 2-Isopropyl-N-methyl-1-(2-oxo-1,3-dihydroimidazo[4,5-b]pyridin-6-yl)benzimidazole-5-carboxamide

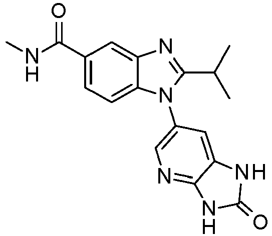

### a) N-Methyl-3-nitro-4-[(2-oxo-1,3-dihydroimidazo[4,5-b]pyridin-6-yl)amino]benzamide

The title compound was obtained in analogy to example 1 using 6-amino-1, 3-dihydroimidazo[4, 5-b]pyridin-2-one¹²⁵ in place of 5-amino-1*H*-benzo[d]imidazol-2(3*H*)-one for step a. Yellow solid. MS (ISP): 297.1 ([M+H]⁺).

### b) 2-Isopropyl-N-methyl-1-(2-oxo-1,3-dihydroimidazo[4,5-b]pyridin-6-yl)benzimidazole-5-carboxamide

To a solution of *N*-methyl-3-nitro-4-[(2-oxo-1,3-dihydroimidazo[4,5-b]pyridin-6-yl)amino]benzamide (180 mg) in ethanol (8 ml) were added isobutyraldehyde (43.5 mg) and 1 M aqueous sodium hydrosulfite (1.64 ml).The reaction mixture was stirred at 70 °C for 12 hours. After cooling, the reaction mixture was concentrated *in vacuo* and the residue was dissolved in ethyl acetate (20 ml).The combined organic layer was washed with brine, dried, filtered and concentrated *in vacuo.* The crude material was purified by preparative reversed phase HPLC to afford 2-isopropyl-*N*-methyl-1-(2-oxo-1,3-dihydroimidazo[4,5-b]pyridin-6-yl)benzimidazole-5-carboxamide (16.3 mg, 8.5%) as a brown solid. MS (ISP): 351.2 ([M+H]⁺).

### Example 80

### N-[2-[1-(2-Oxo-1,3-dihydrobenzimidazol-5-yl)benzimidazol-2-yl]ethyl]acetamide

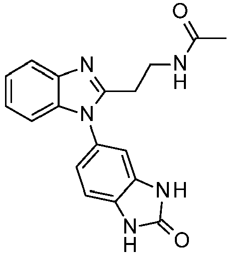

### a) 5-[2-(2-Aminoethyl)benzimidazol-1-yl]-1,3-dihydrobenzimidazol-2-one hydrochloride salt

The title compound was obtained in analogy to example 61 using beta-alanine¹²⁶ in place of glycolic acid in step b. Grey solid. MS (ISP): 294.1 ([M+H]⁺).

### b) N-[2-[1-(2-Oxo-1,3-dihydrobenzimidazol-5-yl)benzimidazol-2-yl]ethyl]acetamide

To a stirred solution of 5-[2-(2-aminoethyl)benzimidazol-1-yl]-1,3-dihydrobenzimidazol-2-one hydrochloride (200 mg) in dichloromethane (10 ml) were added triethylamine (0.25 ml) and acetic anhydride (93 mg). The reaction mixture was stirred at room temperature for 12 hours. According to LC/MS, the reaction was finished. The reaction mixture was concentrated *in vacuo.* The crude material was purified by preparative reversed phase HPLC to afford *N*-[2-[1-(2-oxo-1,3-dihydrobenzimidazol-5-yl)benzimidazol-2-yl]ethyl]acetamide (14.6 mg, 3.6%) as a brown solid. MS (ISP): 336.1 ([M+H]⁺).

### Example 81

### N-Methyl-3-[1-(2-oxo-1,3-dihydrobenzimidazol-5-yl)benzimidazol-2-yl]propanamide

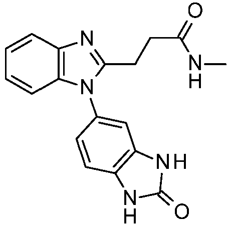

### a) 4-Oxo-4-[2-[(2-oxo-1,3-dihydrobenzimidazol-5-yl)amino]anilino]butanoic acid

To a solution of succinic anhydride¹²⁷ (500 mg) in dichloromethane (15 ml) was added 5-(2-aminoanilino)-1,3-dihydrobenzimidazol-2-one (600 mg). The mixture was stirred at 25 °C for 12 hours. According to LC/MS, the reaction was finished. The reaction mixture was concentrated *in vacuo* to afford crude 4-oxo-4-[2-[(2-oxo-1,3-dihydrobenzimidazol-5-yl)amino]anilino]butanoic acid (800 mg, 94.1%), which was used in the next step without further purification. MS (ISP): 341.2 ([M+H]⁺).

### b) 3-[1-(2-Oxo-1,3-dihydrobenzimidazol-5-yl)benzimidazol-2-yl]propanoic acid

A solution of 4-oxo-4-[2-[(2-oxo-1,3-dihydrobenzimidazol-5-yl)amino]anilino]butanoic acid (800 mg) in a 6 M solution of HCl (15 ml) was stirred at 120 °C for 12 hours. According to LC/MS, the reaction was finished. The reaction mixture was concentrated *in vacuo.* The reaction mixture was concentrated *in vacuo* to afford crude 3-[1-(2-oxo-1,3-dihydrobenzimidazol-5-yl)benzimidazol-2-yl]propanoic acid (550 mg, 72.6%) which was used in the next step without further purification. MS (ISP): 323.1 ([M+H]⁺).

### c) N-Methyl-3-[1-(2-oxo-1,3-dihydrobenzimidazol-5-yl)benzimidazol-2-yl]propanamide

To a solution of 3-[1-(2-oxo-1,3-dihydrobenzimidazol-5-yl)benzimidazol-2-yl]propanoic acid (500 mg) in *N*,*N*-dimethylformamide (10 ml) were added 1-(3-dimethylaminopropyl)-3-ethylcarbodiimide hydrochloride (892 mg),1-hydroxybenzotriazole (629 mg) and methylamine⁸⁴ (3.1 ml). The reaction mixture was stirred at 25 °C for 12 hours. According to LC/MS, the reaction was finished. The mixture was poured into water and extracted with ethyl acetate (3 x 20 ml). The combined organic layers were dried, filtered and concentrated *in vacuo.* The crude material was purified by preparative reversed phase HPLCto afford *N*-methyl-3-[1-(2-oxo-1,3-dihydrobenzimidazol-5-yl)benzimidazol-2-yl]propanamide (270 mg, 51.7%) as a yellow solid. MS (ISP): 336.1 ([M+H]⁺).

### Example 82

### Methyl 1-(2-oxo-1,3-dihydrobenzimidazol-5-yl)benzimidazole-2-carboxylate

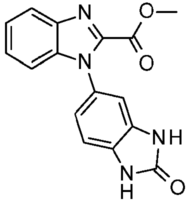

### a) 5-[2-(Trichloromethyl)benzimidazol-1-yl]-1,3-dihydrobenzimidazol-2-one

To a solution of 5-(2-aminoanilino)-1,3-dihydrobenzimidazol-2-one (1000 mg) in acetic acid (15 ml) was added benzyl 2,2,2-trichloroacetimidate¹²⁸ (1.01 ml). The reaction mixture was stirred at 25 °C for 3 hours. According to LC/MS, the reaction was finished. The mixture was concentrated *in vacuo.* The residue was dissolved in ethyl acetate (30 ml) and washed with a aq. solution of sodium carbonate .The organic layer was dried, filtered and concentrated *in vacuo.* The crude material was purified by column chromatography (SiO2, petrol ether/ethylacetate 10:1) to afford 5-[2-(trichloromethyl)benzimidazol-1-yl]-1,3-dihydrobenzimidazol-2-one (1g, 51.5%) as a yellow solid. MS (ISP): 368.8 ([M+H]⁺).

### b) 5-[2-(Trimethoxymethyl)benzimidazol-1-yl]-1,3-dihydrobenzimidazol-2-one

To a solution of 5-[2-(trichloromethyl)benzimidazol-1-yl]-1,3-dihydrobenzimidazol-2-one (200 mg) in methanol (8 ml) was added sodium carbonate (86.5 mg). The reaction mixture was stirred at 80 °C for 12 hours. According to LC/MS, the reaction was finished. The reaction mixture was concentrated *in vacuo* to afford crude 5-[2-(trimethoxymethyl)benzimidazol-1-yl]-1,3-dihydrobenzimidazol-2-one (180 mg, 93.4%) which was used in the next step without further purification. MS (ISP): 355.2 ([M+H]⁺).

### c) Methyl 1-(2-oxo-1,3-dihydrobenzimidazol-5-yl)benzimidazole-2-carboxylate

To a solution of 5-[2-(trimethoxymethyl)benzimidazol-1-yl]-1,3-dihydrobenzimidazol-2-one (180 mg) in methanol (8 ml) was added conc. HCl (2 ml). The reaction mixture was stirred at 25 °C for 12 hours. According to LC/MS, the reaction was finished. The reaction mixture was concentrated *in vacuo.* The crude material was purified by preparative reversed phase HPLC to afford methyl 1-(2-oxo-1,3-dihydrobenzimidazol-5-yl)benzimidazole-2-carboxylate (21.8 mg, 13.7%) as a yellow solid. MS (ISP): 309.1 ([M+H]⁺).

### Example 83 (provided for reference)

### 5-(Benzimidazol-1-yl)-1,3-dihydrobenzimidazol-2-one

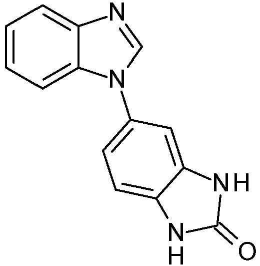

The title compound was obtained in analogy to example 61 using formic acid¹²⁹ in place of glycolic acid for step b and heating for 12 hours instead of 48 hours. Grey solid. MS (ISP): 251.0 ([M+H]⁺).

### Example 84

### 5-[2-(1-Hydroxyethyl)benzimidazol-1-yl]-1,3-dihydrobenzimidazol-2-one

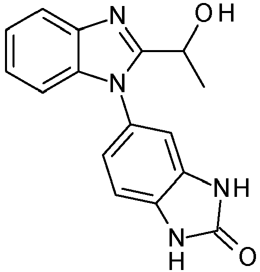

The title compound was obtained in analogy to example 61 using lactic acid¹³⁰ in place of glycolic acid for step b and heating for 12 hours instead of 48 hours. Yellow solid. MS (ISP): 295.1 ([M+H]⁺).

### Example 85

### 5-[2-[Hydroxy(phenyl)methyl]benzimidazol-1-yl]-1,3-dihydrobenzimidazol-2-one

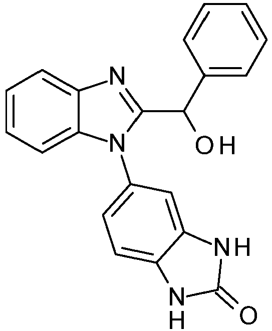

The title compound was obtained in analogy to example 61 using mandelic acid¹³¹ in place of glycolic acid for step b and heating for 12 hours instead of 48 hours. Yellow solid. MS (ISP): 357.1 ([M+H]⁺).

### Example 86 (provided for reference)

### N-(1-Acetylpiperidin-4-yl)-N-methyl-2-oxo-2,3-dihydro-1H-benzo[d]imidazole-5-carboxamide

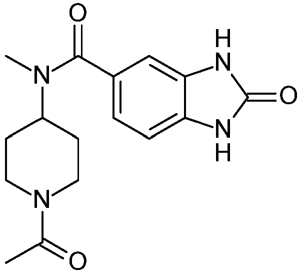

To a solution of 4-dimethylaminopyridine (165 mg) in *N*,*N*-dimethylformamide (5 ml) were added 2-oxo-2,3-dihydro-1*H*-benzimidazole-5-carboxylic acid¹³² (200 mg), HATU (512 mg), *N*,*N*-diisopropylethylamine (0.59 ml) and 1-(4-(methylamino)piperidin-1-yl)ethanone hydrochloride¹³³ (216 mg), then the mixture was stirred at 25 °C for 16 hr. The reaction mixture was diluted with ethyl acetate (5 ml) and water (10 ml). The aqueous layer was extracted twice with ethyl acetate, then the combined organic layers were dried over Na₂SO₄ and concentrated *in vacuo.* The crude material was purified by preparative reversed phase HPLC to afford methyl *N-*(1-acetylpiperidin-4-yl)-N-methyl-2-oxo-2,3-dihydro-1*H*-benzo[d]imidazole-5-carboxamide (30 mg, 8.5 %) as a yellow solid. MS (ISP): 317.2 ([M+H]⁺).

### Example 87 (provided for reference)

### 1-(2-Oxo-2,3-dihydro-1H-benzo[d]imidazol-5-yl)-3-(4-(phenylamino)phenyl)urea

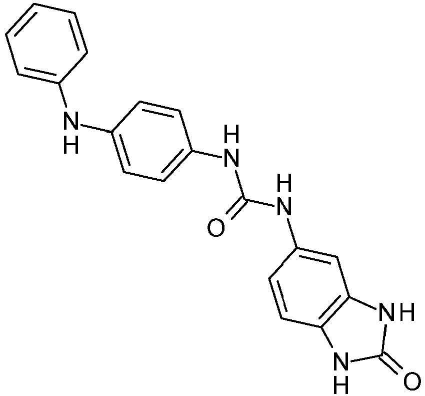

To a stirred suspension of *N*1-phenylbenzene-1,4-diamine¹³⁴ (124 mg) and triethylamine (187 µl) in THF (2 ml) was added triphosgene (73.6 mg) at 0-5 °C. The reaction mixture was stirred at room temperature for 1 h. A solution of 5-amino-1,3-dihydro-2*H*-benzo[d]imidazol-2-one⁵⁰ (100 mg) in DMF (1 ml) added and the reaction mixture was stirred at room temperature for 3 h. Water were added and the mixture was stirred at room temperature for 30 min. The product was collected by filtration, washing with water, and dried *in vacuo* in a drying oven to give 1-(2-oxo-2,3-dihydro-1*H*-benzo[d]imidazol-5-yl)-3-(4-(phenylamino)phenyl)urea (103 mg, 43%) as a grey solid. MS (ISP): 360.1 ([M+H]⁺).

### Example 88 (provided for reference)

### 1-(2-Oxo-2,3-dihydro-1H-benzo[d]imidazol-5-yl)-3-phenylurea

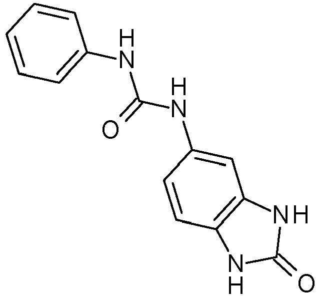

The title compound was obtained in analogy to example 87 using aniline⁸⁰ in place of *N*1-phenylbenzene-1,4-diamine. Off-white solid. MS (ISP): 269.1 ([M+H]⁺).

### Example 89 (provided for reference)

### Methyl 1-((2-oxo-2,3-dihydro-1H-benzo[d]imidazol-5-yl)carbamoyl)indoline-4-carboxylate

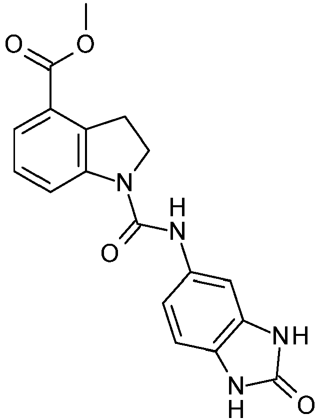

The title compound was obtained in analogy to example 87 using methyl indoline-4-carboxylate¹⁰⁴ in place of *N*1-phenylbenzene-1,4-diamine. Off-white solid. MS (ISP): 353.1 ([M+H]⁺).

### Example 90 (provided for reference)

### 3-Acetamido-N-(2-oxo-2,3-dihydro-1H-benzo[d]imidazol-5-yl)propanamide

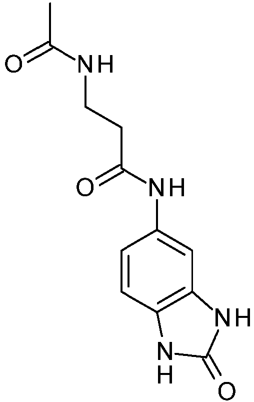

The title compound was obtained in analogy to example 68 using 3-acetamidopropanoic acid¹³⁵ in place of benzofuran-3-carboxylic acid. White solid. MS (ISP): 263.1 ([M+H]⁺).

### Example 91 (provided for reference)

### 1-Acetyl-N-(2-oxo-2,3-dihydro-1H-benzo[d]imidazol-5-yl)piperidine-4-carboxamide

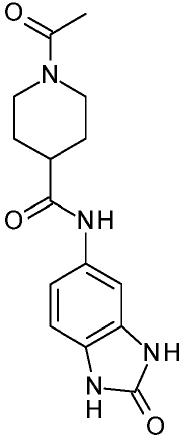

The title compound was obtained in analogy to example 68 using 1-acetylpiperidine-4-carboxylic acid ¹³⁶ in place of benzofuran-3-carboxylic acid. White solid. MS (ISP): 303.1 ([M+H]⁺).

### Example 92 (provided for reference)

### Methyl 1-((2-oxo-2,3-dihydro-1H-benzo[d]imidazol-5-yl)carbamoyl)indoline-5-carboxylate

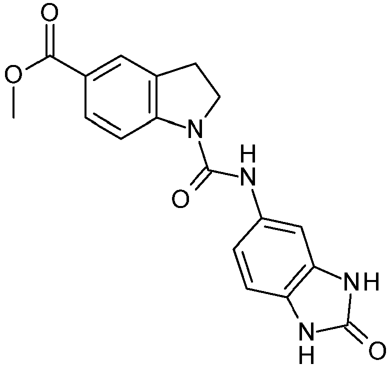

The title compound was obtained in analogy to example 87 using methyl indoline-5-carboxylate ¹³⁷ in place of *N*1-phenylbenzene-1,4-diamine. Off-white solid. MS (ISP): 353.1 ([M+H]⁺).

### Example 93 (provided for reference)

### N4-Methyl-N1-(2-oxo-2,3-dihydro-1H-benzo[d]imidazol-5-yl)indoline-1,4-dicarboxamide

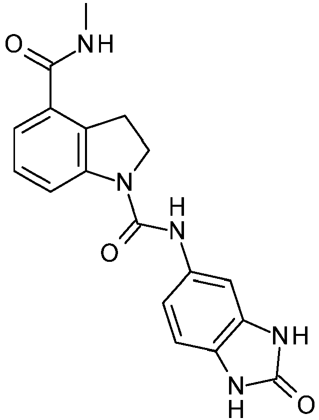

### a) 1-((2-Oxo-2,3-dihydro-1H-benzo[d]imidazol-5-yl)carbamoyl)indoline-4-carboxylic acid

To a stirred suspension of methyl 1-((2-oxo-2,3-dihydro-1*H*-benzo[d]imidazol-5-yl)carbamoyl)indoline-4-carboxylate (120 mg, example 89) in THF (1.5 ml) and MeOH (1.5 ml) was added 1 M aq. LiOH solution (1.36 ml). The reaction mixture was stirred at room temperature overnight to afford a dark brown solution. LC-MS analysis indicated that the reaction was complete. 5 N aq. HCl was added dropwise to adjust the pH to 5 to give a light brown suspension. The reaction mixture was then filtered through a Sartorius funnel. The filter cake was dried *in vacuo* to afford 1-((2-oxo-2,3-dihydro-1*H*-benzo[d]imidazol-5-yl)carbamoyl)indoline-4-carboxylic acid (67 mg, 58%) as an off-white solid. The organic solvents of the filtrate were separated and concentrated *in vacuo.* Water was added to the residue and the resulting solution was lyophilised to afford a further quantity of 1-((2-oxo-2,3-dihydro-1*H*-benzo[d]imidazol-5-yl)carbamoyl)indoline-4-carboxylic acid as an off-white solid. The crude product was used in the next step without further purification.

### b) N4-Methyl-N1-(2-oxo-2,3-dihydro-1H-benzo[d]imidazol-5-yl)indoline-1,4-dicarboxamide

To a stirred suspension of 1-((2-oxo-2,3-dihydro-1*H*-benzo[d]imidazol-5-yl)carbamoyl)indoline-4-carboxylic acid (50 mg), *N*,*N*-diisopropylethylamine (155 µl) and methanamine (148 µl, 2 M solution in THF) in DMF (3 ml) was added HATU (112 mg). The reaction mixture was stirred at room temperature for 2 h. The reaction mixture was then poured into water and extracted with EtOAc/THF (1:2). The organic layer was separated, dried over Na₂SO₄, and concentrated *in vacuo.* The crude material was purified by preparative reverse phase HPLC (YMC-Triart C18, 12 nm, 5 µm, 100 x 30 mm, flow rate 40 ml/min, eluant: CH₃CN/H₂O containing formic acid) followed by lyophilisation to afford *N*4-methyl-*N*1-(2-oxo-2,3-dihydro-1*H*-benzo[d]imidazol-5-yl)indoline-1,4-dicarboxamide (13 mg, 25%) as a white solid. MS (ISP): 352.1 ([M+H]⁺).

### Example 94 (provided for reference)

### 1-Benzyl-3-(2-oxo-2,3-dihydro-1H-benzo[d]imidazol-5-yl)urea

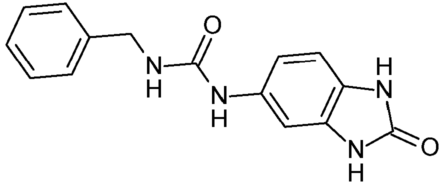

To a stirred suspension of (isocyanatomethyl)benzene¹³⁸ (89.3 mg) in THF (1 ml) at room temperature was added 5-amino-1,3-dihydro-2*H*-benzo[d]imidazol-2-one⁵⁰ (100 mg) and the reaction mixture was stirred for at room temperature for 1 h. The product was collected by filtration, washing with THF (20 ml), and dried *in vacuo* in a drying oven to give 1-benzyl-3-(2-oxo-2,3-dihydro-1*H*-benzo[d]imidazol-5-yl)urea (177 mg, 94%) as a grey solid gry solid. MS (ISP): 283.1 ([M+H]⁺).

### Example 95 (provided for reference)

### 6-(1,2,3,4-Tetrahydroquinoline-1-carbonyl)-1,3-dihydro-2H-imidazo[4,5-b]pyridin-2-one

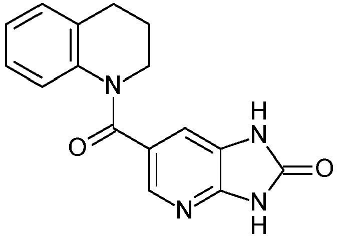

The title compound was obtained in analogy to example 22 using 1,2,3,4-tetrahydroquinoline ¹³⁹ in place of 1-(piperazin-1-yl)ethanone and 2-oxo-2,3-dihydro-1*H-*imidazo[4,5-b]pyridine-6-carboxylic acid instead of 2-oxo-2,3-dihydro-1*H*-benzo[d]imidazole-5-carboxylic acid. White solid. MS (ISP): 295.3 ([M+H]⁺).

### Example 96¹⁴⁰ (provided for reference)

### N-Methyl-2-oxo-N-phenyl-2,3-dihydro-1H-benzo[d]imidazole-5-carboxamide

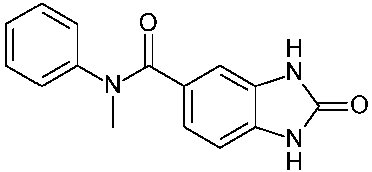

The title compound can be purchased.

### Example 97¹⁴¹ (provided for reference)

### 5-(1,2,3,4-Tetrahydroquinoline-1-carbonyl)-1,3-dihydro-2H-benzo[d]imidazol-2-one

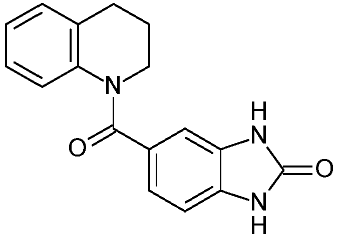

The title compound can be purchased.

### Example 98 (provided for reference)

### Methyl 1-((2-oxo-2,3-dihydro-1H-benzo[d]imidazol-5-yl)carbamoyl)-1,2,3,4-tetrahydroquinoline-5-carboxylate

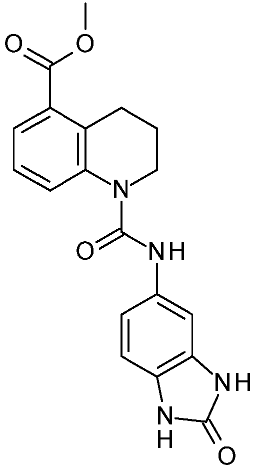

The title compound was obtained in analogy to example 87 using methyl 1,2,3,4-tetrahydroquinoline-5-carboxylate¹⁴² in place of *N*1-phenylbenzene-1,4-diamine. Grey solid. MS (ISP): 367.3 ([M+H]⁺).

### Example 99 (provided for reference)

### N-(2-Oxo-2,3-dihydro-1H-benzo[d]imidazol-5-yl)-2-(4-sulfamoylphenyl)acetamide

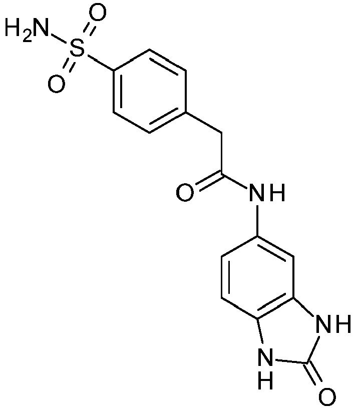

The title compound was obtained in analogy to example 68 using 2-(4-sulfamoylphenyl)acetic acid¹⁴³ in place of benzofuran-3-carboxylic acid. White solid. MS (ISP): 347.1 ([M+H]⁺).

### Example 100 (provided for reference)

### N-(2-Oxo-2,3-dihydro-1H-benzo[d]imidazol-5-yl)-4-sulfamoylbenzamide

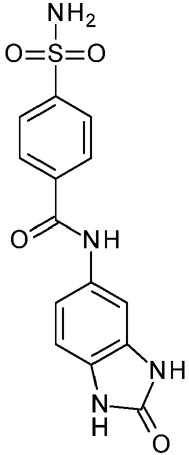

The title compound was obtained in analogy to example 68 using 4-sulfamoylbenzoic acid¹⁴⁴ in place of benzofuran-3-carboxylic acid. White solid. MS (ISP): 333.1 ([M+H]⁺).

### Example 101 (provided for reference)

### N-(2-Oxo-2,3-dihydro-1H-benzo[d]imidazol-5-yl)-4-sulfamoylbenzamide

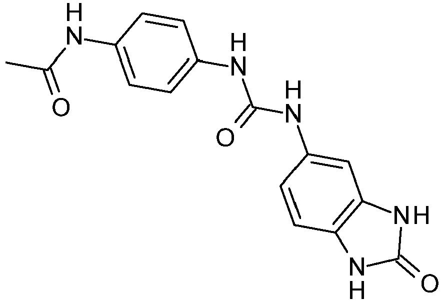

The title compound was obtained in analogy to example 87 using N-(4-aminophenyl)acetamide¹⁴⁵ in place of *N*1-phenylbenzene-1,4-diamine. Brown solid. MS (ISP): 326.1 ([M+H]⁺).

### Example 102

### N-Methyl-1-(2-oxo-1,3-dihydrobenzimidazol-5-yl)benzimidazole-2-carboxamide

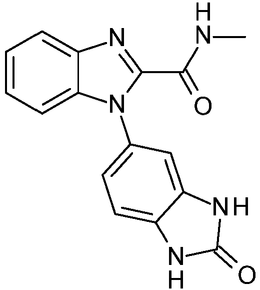

### a) Methyl 1-(2-oxo-1,3-dihydrobenzimidazol-5-yl)benzimidazole-2-carboxylate

To a solution of 5-[2-(trichloromethyl)benzimidazol-1-yl]-1,3-dihydrobenzimidazol-2-one (3.0 g) in methanol (40 ml) was added sodium methoxide (440 mg). The reaction mixture was stirred at 80 °C for 12 hours. According to LC/MS, the reaction was finished. The reaction mixture was concentrated *in vacuo* to afford crude methyl 1-(2-oxo-1,3-dihydrobenzimidazol-5-yl)benzimidazole-2-carboxylate (3 g, 67.9%) which was used in the next step without further purification. MS (ISP): 309.0 ([M+H]⁺).

### b) N-Methyl-1-(2-oxo-1,3-dihydrobenzimidazol-5-yl)benzimidazole-2-carboxamide

To a solution of methyl 1-(2-oxo-1,3-dihydrobenzimidazol-5-yl)benzimidazole-2-carboxylate (300 mg) in methanol (10 ml) were added methylamine (0.73 ml) and triethylamine (0.27 ml The reaction mixture was stirred at 80 °C for 4 hours. According to LC/MS, the reaction was finished ,the reaction mixture was concentrated *in vacuo.* The crude material was purified by preparative reversed phase HPLCto afford N-methyl-1-(2-oxo-1,3-dihydrobenzimidazol-5-yl)benzimidazole-2-carboxamide (85.4 mg, 27.8%) as a yellow solid. MS (ISP): 308.2 ([M+H]⁺).

### Example 103

### 5-[2-(Piperidine-1-carbonyl)benzimidazol-1-yl]-1,3-dihydrobenzimidazol-2-one

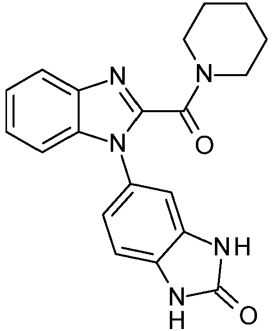

The title compound was obtained in analogy to example 102 using piperidine¹⁴⁶ in place of methylamine for step b. White solid. MS (ISP): 362.6 ([M+H]⁺).

### Example 104 (provided for reference)

### 6-(2-(Benzyloxy)phenyl)-1,3-dihydro-2H-imidazo[4,5-b]pyridin-2-one

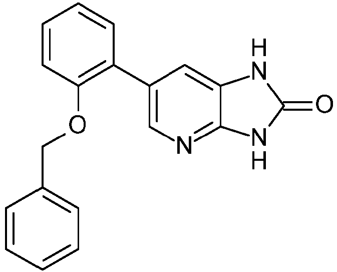

To a stirred solution of (2-(benzyloxy)phenyl)boronic acid¹⁴⁷ (100 mg) and 6-bromo-1,3-dihydro-2*H*-imidazo[4,5-b]pyridin-2-one (94 mg) in water (0.3 ml) and dioxane (1.5 ml) was added 1,1'-bis(diphenylphosphino)ferrocene-palladium(II) dichloride dichloromethane complex (35.8 mg) at room temperature. After degassing for 10 minutes with argon, Cs₂CO₃ (286 mg) was added. The suspension was stirred at 90 °C for 16 hours. The reaction mixture was partitioned between water and ethyl acetate. The organic layer was washed with brine, dried over Na₂SO₄, filtered and concentrated *in vacuo*.The crude material was stirred in a mixture of dichloromethane/methanol 95:5, filtered and dried to afford 6-(2-(benzyloxy)phenyl)-1,3-dihydro-2*H*-imidazo[4,5-b]pyridin-2-one (75.8 mg, 54.5 %) as a light yellow solid MS (ISP): 318.3 ([M+H]⁺).

### Example 105 (provided for reference)

### N-(2-Oxo-2,3-dihydro-1H-benzo[d]imidazol-5-yl)indoline-1-carboxamide

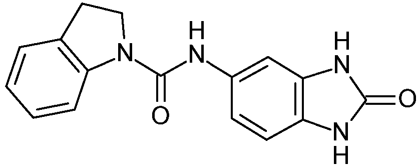

The title compound was obtained in analogy to example 87 using 1*H*-indoline⁷⁶ in place of *N*1-phenylbenzene-1,4-diamine. Purple solid. MS (ISP): 295.1 ([M+H]⁺).

### Example 106 (provided for reference)

### 2-(4-(N-Methylsulfamoyl)phenyl)-N-(2-oxo-2,3-dihydro-1H-benzo[d]imidazol-5-yl)acetamide

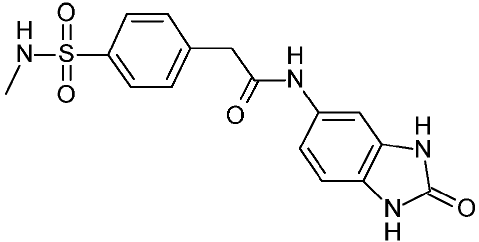

The title compound was obtained in analogy to example 68 using 2-(4-(*N-*methylsulfamoyl)phenyl)acetic acid¹⁴⁸ in place of benzofuran-3-carboxylic acid. White solid. MS (ISP): 361.1 ([M+H]⁺).

### Example 107

### Methyl 2-(2-(1-methyl-1H-imidazol-5-yl)quinolin-4-yl)-2'-oxo-2',3'-dihydro-1'H-[1,5'-bibenzo[d]imidazole]-5-carboxylate

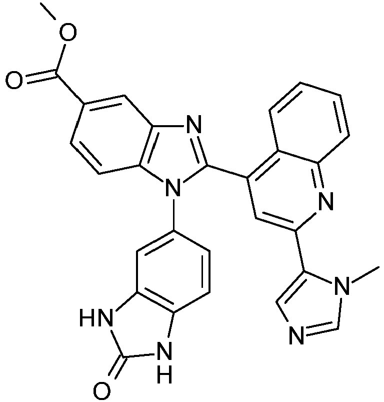

### a) Methyl 3-amino-4-((2-oxo-2,3-dihydro-1H-benzo[d]imidazol-5-yl)amino)benzoate

The title compound was obtained in analogy to example 1 using methyl 4-fluoro-3-nitrobenzoate⁴⁸ in place of 4-fluoro-*N*-methyl-3-nitrobenzamide in step a. Grey solid. MS (ISP): 299.2 ([M+H]⁺).

### b) Methyl 2-(2-(1-methyl-1H-imidazol-5-yl)quinolin-4-yl)-2'-oxo-2',3'-dihydro-1'H-[1,5'-bibenzo[d]imidazole]-5-carboxylate

The title compound was obtained in analogy to example 2 using methyl 3-amino-4-((2-oxo-2,3-dihydro-1*H*-benzo[d]imidazol-5-yl)amino)benzoate in place of 3-amino-*N*-methyl-4-[(2-oxo-1,3-dihydrobenzimidazol-5-yl)amino]benzamide. Light brown solid. MS (ISP): 516.2 ([M+H]⁺).

### Example 108

### 2-(2-Methoxypyridin-4-yl)-N-methyl-2'-oxo-2',3'-dihydro-1'H-[1,5'-bibenzo[d]imidazole]-5-carboxamide

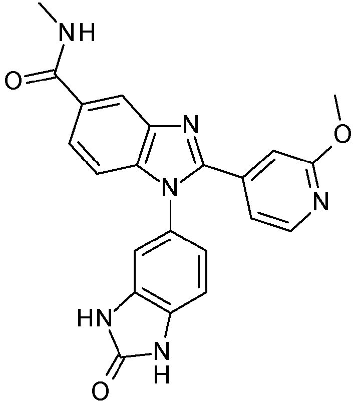

The title compound was obtained in analogy to example 2 using 2-methoxyisonicotinaldehyde ¹⁴⁹ in place of 2-(3-methylimidazol-4-yl)quinoline-4-carbaldehyde. White solid. MS (ISP): 415.2 ([M+H]⁺).

### Example 109 (provided for reference)

### N5-Methyl-N1-(2-oxo-2,3-dihydro-1H-benzo[d]imidazol-5-yl)-3,4-dihydroquinoline-1,5(2H)-dicarboxamide

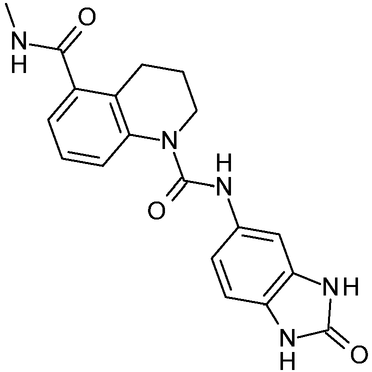

The title compound was obtained in analogy to example 93 using methyl 1-((2-oxo-2,3-dihydro-1*H*-benzo[d]imidazol-5-yl)carbamoyl)-1,2,3,4-tetrahydroquinoline-5-carboxylate (example 98) instead of methyl 1-((2-oxo-2,3-dihydro-1*H*-benzo[d]imidazol-5-yl)carbamoyl)indoline-4-carboxylate. White solid. MS (ISP): 366.2 ([M-H]⁻).

### Example 110 (provided for reference)

### 5-(5-Bromo-1-oxo-isoindolin-2-yl)-1,3-dihydrobenzimidazol-2-one

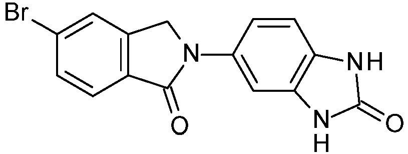

### a) Methyl 4-bromo-2-[[(2-oxo-1,3-dihydrobenzimidazol-5-yl)amino]methyl]benzoate

To a solution of methyl 4-bromo-2-(bromomethyl)benzoate¹⁵⁰ (1.0 g) in methanol (10 ml) was added triethylamine (0.56 ml) and 5-amino-1,3-dihydro-2*H*-benzimidazol-2-one (0.4 g).The reaction mixture was stirred at 25 °C for 12 hours. The yellow suspension obtained was filtered and the solid dried *in vacuo* to afford crude methyl 4-bromo-2-[[(2-oxo-1,3-dihydrobenzimidazol-5-yl)amino]methyl]benzoate (1 g, 98.7%) which was used in the next step without further purification.

### b) Methyl 1-oxo-2-(2-oxo-1,3-dihydrobenzimidazol-5-yl)isoindoline-5-carboxylate

To a solution of methyl 4-bromo-2-[[(2-oxo-1,3-dihydrobenzimidazol-5-yl)amino]methyl]benzoate (1.0 g) in ethanol (5 ml) was added potassium carbonate (551 mg). The reaction mixture was stirred at 80 °C for 4 hours. The resulting yellow suspension was filtered, and the filter cake was washed sequentially with water, ethyl acetate and ethanol and then dried *in vacuo* to afford 5-(5-bromo-1-oxo-isoindolin-2-yl)-1,3-dihydrobenzimidazol-2-one (460 mg, 50.3%). Yellow solid. MS (ISP): MS (IPS): 344.1 [M+H]^{+ 79}Br; 346.1 [M+H]^{+ 81}Br

### Example 111 (provided for reference)

### Ethyl 1-oxo-2-(2-oxo-1,3-dihydrobenzimidazol-5-yl)isoindoline-5-carboxylate

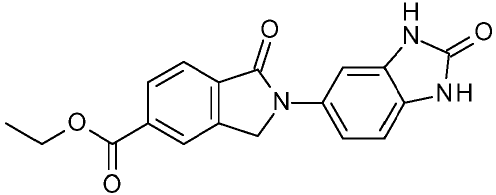

### a) Dimethyl 2-[[(2-oxo-1,3-dihydrobenzimidazol-5-yl)amino]methyl]benzene-1,4-dicarboxylate

To a solution of dimethyl 2-(bromomethyl)benzene-1,4-dicarboxylate**^{Error! Bookmark not defined}**. (1.7 g) in methanol (2 ml) were added triethylamine (1.24 ml) and 5-amino-1,3-dihydro-2*H-*benzimidazol-2-one (883 mg). The reaction mixture was stirred at 25 °C for 12 hours. The resulting suspension was filtered and the filtrate was concentrated *in vacuo.*The residue was diluted with water (20 ml) and ethyl acetate (30 ml). The layers were separated and the aqueous layer was extracted with ethyl acetate (2 x 20 ml). The combined organic layers were washed with brine, dried with Na₂SO₄, filtered and concentrated *in vacuo* to afford dimethyl 2-[[(2-oxo-1,3-dihydrobenzimidazol-5-yl)amino]methyl]benzene-1,4-dicarboxylate in a 1:1 mixture with dimethyl 2-[[[2,5-bis(methoxycarbonyl)phenyl]methyl-(2-oxo-1,3-dihydrobenzimidazol-5-yl)amino]methyl]benzene-1,4-dicarboxylate (1.47g), which was used in the next step without further purification.

### b) Ethyl 1-oxo-2-(2-oxo-1,3-dihydrobenzimidazol-5-yl)isoindoline-5-carboxylate

The title compound was obtained in analogy to example 110 using dimethyl 2-[[(2-oxo-1,3-dihydrobenzimidazol-5-yl)amino]methyl]benzene-1,4-dicarboxylate in place of methyl 4-bromo-2-[[(2-oxo-1,3-dihydrobenzimidazol-5-yl)amino]methyl]benzoate. White solid. MS (ISP): 336.3 ([M-H]⁻).

### Example 112 (provided for reference)

### Methyl 1-oxo-2-(2-oxo-1,3-dihydrobenzimidazol-5-yl)isoindoline-5-carboxylate

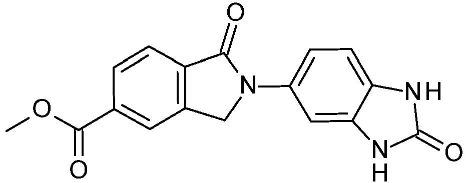

The title compound was obtained in analogy to example 111 using methanol as solvent instead of ethanol in the final step. Light yellow solid. MS (ISP): 322.3 ([M-H]⁻).

### Example 113 (provided for reference)

### 2-(2-(1-Methyl-1H-imidazol-5-yl)quinolin-4-yl)-2'-oxo-2',3'-dihydro-1'H-[1,5'-bibenzo[d]imidazole]-5-carboxamide

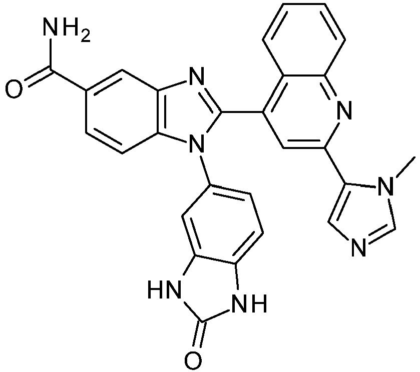

The title compound was obtained in analogy to example 18 step e using aqueous ammonia in place of dimethylamine. Light brown solid. MS (ISP): 501.2 ([M+H]⁺).

### Example 114

### N-Methyl-2'-oxo-2-(1-phenylcyclopropyl)-2',3'-dihydro-1'H-[1,5'-bibenzo[d]imidazole]-5-carboxamide

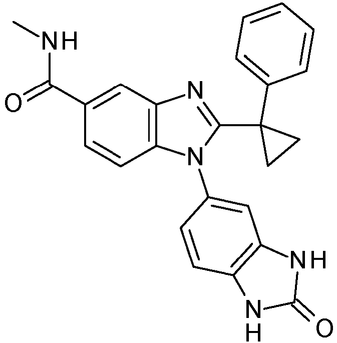

The title compound was obtained in analogy to example 2 using 1-phenylcyclopropane-1-carbaldehyde¹⁵¹ in place of 2-(3-methylimidazol-4-yl)quinoline-4-carbaldehyde. White solid. MS (ISP): 424.2 ([M+H]⁺).

### Example 115

### 2-(1-(4-Methoxyphenyl)cyclopropyl)-N-methyl-2'-oxo-2',3'-dihydro-1'H-[1,5'-bibenzo[d]imidazole]-5-carboxamide

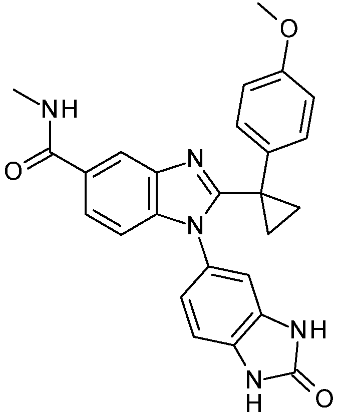

The title compound was obtained in analogy to example 2 using 1-(4-methoxyphenyl)cyclopropane-1-carbaldehyde¹⁵² in place of 2-(3-methylimidazol-4-yl)quinoline-4-carbaldehyde. Light yellow solid. MS (ISP): 454.2 ([M+H]⁺).

### Example 116 (provided for reference)

### 5-(Indolin-1-ylsulfonyl)-1,3-dihydro-2H-benzo[d]imidazol-2-one

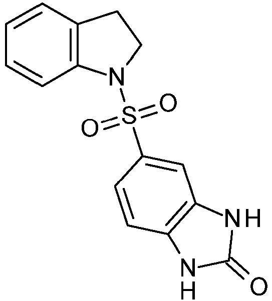

To a stirred solution of indoline⁷⁶ (25.6 mg) in pyridine (0.5 ml) was added 2-oxo-2,3-dihydro-1*H*-benzo[d]imidazole-5-sulfonyl chloride**^{Error! Bookmark not defined}**. (50 mg). The reaction mixture was stirred at room temperature overnight. LC-MS analysis indicated that the reaction was complete. The reaction mixture was poured into EtOAc/THF (1:3) and extracted sequentially with 2 M aq. HCl and with saturated brine. The organic layer was dried over Na₂SO₄ and concentrated *in vacuo.* The crude material was purified by flash chromatography (silica gel, 0% to 10% MeOH in dichloromethane) to afford 5-(indolin-1-ylsulfonyl)-1,3-dihydro-2*H*-benzo[d]imidazol-2-one (44 mg, 65%) as a white solid. MS (ISP): 314.1 ([M-H]⁻).

### Example 117 (provided for reference)

### N-(1-Acetylpiperidin-4-yl)-2-oxo-2,3-dihydro-1H-benzo[d]imidazole-5-sulfonamide

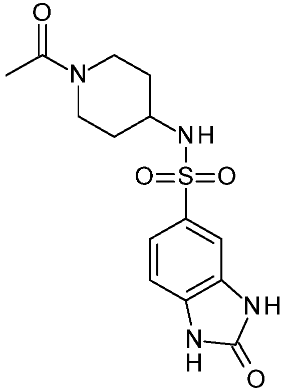

The title compound was obtained in analogy to example 116 using 1-(4-aminopiperidin-1-yl)ethan-1-one⁷⁵ in place of indoline. White solid. MS (ISP): 339.1 ([M+H]⁺).

### Example 118 (provided for reference)

### Methyl 4-(N-(2-oxo-2,3-dihydro-1H-benzo[d]imidazol-5-yl)sulfamoyl)benzoate

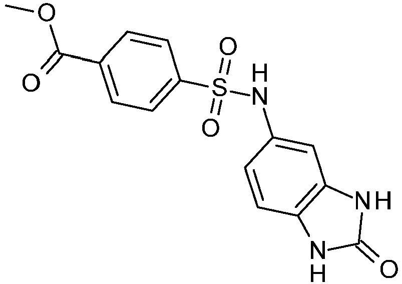

To a stirred solution of 5-amino-1,3-dihydro-2*H*-benzo[d]imidazol-2-one⁵⁰ (50 mg) in pyridine (0.5 ml) was added methyl 4-(chlorosulfonyl)benzoate¹⁵³ (78.7 mg). The reaction mixture was stirred at room temperature for 1 h. LC-MS analysis indicated that the reaction was complete. The reaction mixture was poured into EtOAc/THF (1:3) and extracted sequentially with diluted 2 M aq. HCl and with saturated brine. The organic layer was dried over Na₂SO₄ and concentrated *in vacuo.* The residue was taken up in dichloromethane and the mixture was ultrasonicated at room temperature. The mixture was then filtered through sintered glass, and the solid was dried *in vacuo* to afford methyl 4-(*N*-(2-oxo-2,3-dihydro-1*H*-benzo[d]imidazol-5-yl)sulfamoyl)benzoate (101 mg, 87 %) as an off-white solid. MS (ISP): 348.1 ([M+H]+).

### Example 119 (provided for reference)

### tert-Butyl (1-((2-oxo-2,3-dihydro-1H-benzo[d]imidazol-5-yl)carbamoyl)indolin-5-yl)carbamate

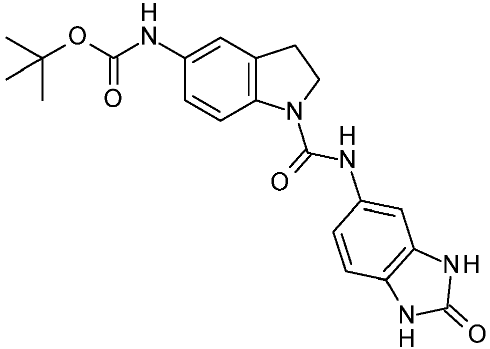

The title compound was obtained in analogy to example 87 using *tert*-butyl indolin-5-ylcarbamate¹⁵⁴ in place of *N*1-phenylbenzene-1,4-diamine. Brown solid. MS (ISP): 408.2 ([M-H]⁻).

### Example 120 (provided for reference)

### 5-(Benzyloxy)-2-isopropyl-1',3'-dihydro-2'H-[1,5'-bibenzo[d]imidazol]-2'-one

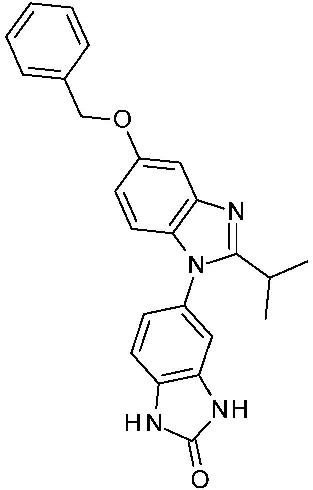

### a) 5-((4-(Benzyloxy)-2-nitrophenyl)amino)-1,3-dihydro-2H-benzo[d]imidazol-2-one

To a stirred solution of 5-amino-1,3-dihydro-2*H*-benzo[d]imidazol-2-one⁵⁰ (200 mg) and 4-(benzyloxy)-1-fluoro-2-nitrobenzene¹⁵⁵ (332 mg) in *N*-methyl-2-pyrrolidinone (2 ml) was added *N*,*N*-diisopropylethylamine (351 µl) at room temperature. The reaction mixture was heated at 120 °C for 15 h. LC-MS analysis indicated that the reaction was complete. The reaction mixture was partitioned between 1:1 ethyl acetate/THF (50-ml) and 1 M aqueous sodium hydroxide solution (30 ml). The layers were separated and the aqueous layer was extracted with three 30 ml portions of ethyl acetate. The combined organic layers were washed with one 25 ml portion of saturated brine, dried over anhydrous sodium sulfate, and concentrated *in vacuo.* The crude material was purified by flash chromatography (SiO₂, eluant: dichloromethane /methanol 100:0 to 95:5) to give 5-((4-(benzyloxy)-2-nitrophenyl)amino)-1,3-dihydro-2*H*-benzo[d]imidazol-2-one (410 mg, 81%) as a red solid. MS (ISP): 377.2 ([M+H]⁺).

### b) 5-((2-Amino-4-(benzyloxy)phenyl)amino)-1,3-dihydro-2H-benzo[d]imidazol-2-one

To a stirred suspension of 5-((4-(benzyloxy)-2-nitrophenyl)amino)-1,3-dihydro-2*H-*benzo[d]imidazol-2-one (410 mg) and iron dust (608 mg) in 1:5 water/EtOH (2 ml) at room temperature was added acetic acid (624 µl). The reaction mixture was then heated at reflux with shaking for 2 h. LC-MS analysis indicated that the reaction was complete. The solids were removed by filtration over HiFlo Decalit and washed with ethyl acetate. The filtrate was partitioned between ethyl acetate (50 ml) and 2 M aq. sodium carbonate (30 ml). The layers were separated. The aqueous layer was extracted with two 20 ml portions of ethyl acetate. The combined organic layers were washed with one 20 ml portion of saturated brine, dried over anhydrous sodium sulfate, and concentrated *in vacuo.* The crude material was purified by flash chromatography (SiO2, eluant: dichloromethane /methanol 100:0 to 90:10) to give 5-((2-amino-4-(benzyloxy)phenyl)amino)-1,3-dihydro-2*H*-benzo[d]imidazol-2-one as a red solid. MS (ISP): 347.2 ([M+H]⁺).

### c) 5-(Benzyloxy)-2-isopropyl-1',3'-dihydro-2'H-[1,5'-bibenzo[d]imidazol]-2'-one

To a stirred solution of 5-((2-amino-4-(benzyloxy)phenyl)amino)-1,3-dihydro-2*H-*benzo[d]imidazol-2-one (95 mg,) and isobutyraldehyde (100 µl) in *N,N*-dimethylformamide (2 ml) at room temperature was added sodium metabisulfite (209 mg). The reaction mixture was then heated at 120 °C for 2 h. LC-MS analysis indicated that the reaction was complete. The reaction mixture was partitioned between 1: 1 THF/ethyl acetate (20 ml) and water (20 ml). The layers were separated. The aqueous layer was extracted with one 15 ml portion of ethyl acetate. The combined organic layers were washed with one 10 ml portion of saturated brine, dried over anhydrous sodium sulfate, and concentrated *in vacuo* to afford 5-(benzyloxy)-2-isopropyl-1',3'-dihydro-2'*H-*[1,5'-bibenzo[d]imidazol]-2'-one ((22 mg, 20%) as a white solid. MS (ISP): 399.2 ([M+H]⁺).

### Example 121 (provided for reference)

### 5-Amino-N-(2-oxo-2,3-dihydro-1H-benzo[d]imidazol-5-yl)indoline-1-carboxamide

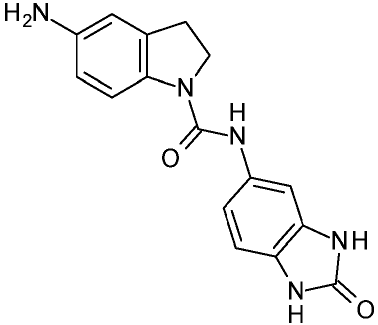

To a stirred suspension of *tert*-butyl (1-((2-oxo-2,3-dihydro-1*H*-benzo[d]imidazol-5-yl)carbamoyl)indolin-5-yl)carbamate (130 mg, example 119) in 1,4-dioxane (0.5 ml) at room temperature was added 4 M hydrogen chloride solution in 1,4-dioxane (1.5 ml). The reaction mixture was stirred at room temperature overnight. LC-MS analysis indicated that the reaction was finished. The resulting solid was collected by filtration, washed with methanol and pentane and dried *in vacuo* to give 5-amino-*N*-(2-oxo-2,3-dihydro-1*H*-benzo[d]imidazol-5-yl)indoline-1-carboxamide (69 mg, 63%) as its hydrochloride salt as a brown solid. MS (ISP): 310.1 ([M+H]⁺).

### Example 122 (provided for reference)

### 2'-Methyl-N-(2-oxo-2,3-dihydro-1H-benzo[d]imidazol-5-yl)-[1,1'-biphenyl]-2-carboxamide

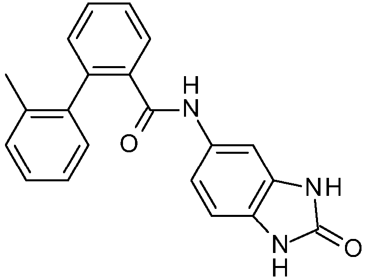

The title compound was obtained in analogy to example 68 using 2'-methyl-[1,1'-biphenyl]-2-carboxylic acid¹⁵⁶ in place of benzofuran-3-carboxylic acid. White solid. MS (ISP): 344.1 ([M+H]⁺).

### Example 123 (provided for reference)

### 2-Benzyl-N-(2-oxo-2,3-dihydro-1H-benzo[d]imidazol-5-yl)benzamide

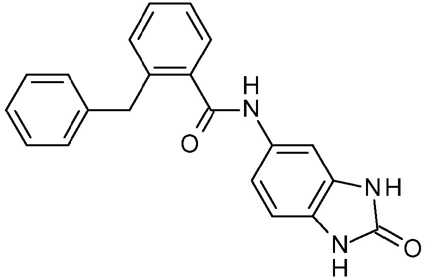

The title compound was obtained in analogy to example 68 using 2-benzylbenzoic acid¹⁵⁷ in place of benzofuran-3-carboxylic acid. White solid. MS (ISP): 344.1 ([M+H]⁺).

### Example 124 (provided for reference)

### 2-Isopropyl-5-methoxy-1',3'-dihydro-2'H-[1,5'-bibenzo[d]imidazol]-2'-one

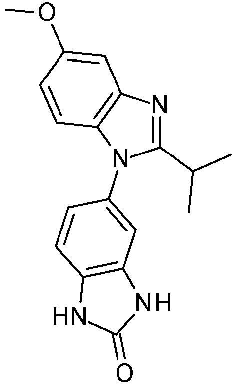

The title compound was obtained in analogy to example 120 using 1-fluoro-4-methoxy-2-nitrobenzene¹⁵⁸ in place of 4-(benzyloxy)-1-fluoro-2-nitrobenzene in step a. Off-white solid. MS (ISP): 323.1 ([M+H]⁺).

### Example 125 (provided for reference)

### tert-Butyl 4-(N-(2-oxo-2,3-dihydro-1H-benzo[d]imidazol-5-yl)sulfamoyl)piperidine-1-carboxylate

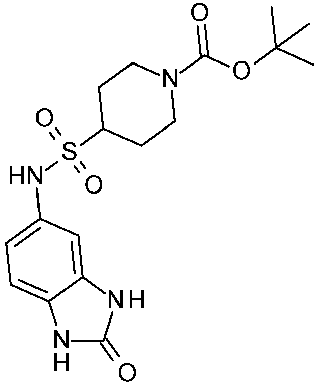

The title compound was obtained in analogy to example 118 using *tert*-butyl 4-(chlorosulfonyl)piperidine-1-carboxylate¹⁵⁹ in place of methyl 4-(chlorosulfonyl)benzoate. White solid. MS (ISP): 395.1 ([M-H]⁻).

### Example 126 (provided for reference)

### Ethyl 4-(3-(2-oxo-2,3-dihydro-1H-benzo[d]imidazol-5-yl)ureido)benzoate

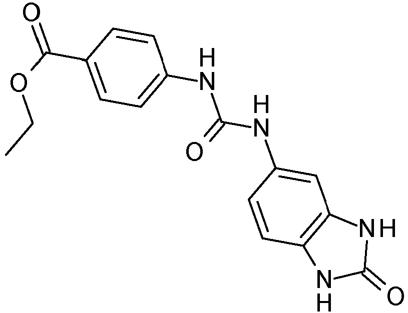

The title compound was obtained in analogy to example 87 using ethyl 4-aminobenzoate⁹⁶ in place of *N*1-phenylbenzene-1,4-diamine. Off-white solid. MS (ISP): 341.1 ([M+H]⁺).

### Example 127 (provided for reference)

### 1-(3-Fluorophenyl)-3-(2-oxo-2,3-dihydro-1H-benzo[d]imidazol-5-yl)urea

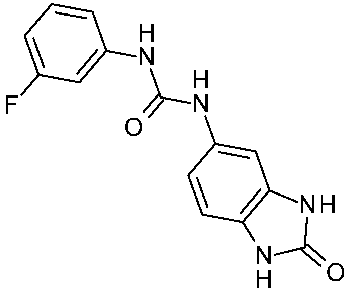

The title compound was obtained in analogy to example 87 using 3-fluoroaniline¹⁶⁰ in place of *N*1-phenylbenzene-1,4-diamine. Off-white solid. MS (ISP): 287.1 ([M+H]⁺).

### Example 128 (provided for reference)

### 4-(2-Methoxyphenyl)-N-(2-oxo-2,3-dihydro-1H-benzo[d]imidazol-5-yl)piperazine-1-carboxamide

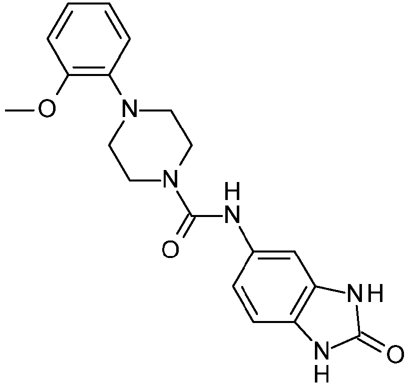

The title compound was obtained in analogy to example 87 using 1-(2-methoxyphenyl)piperazine¹⁶¹ in place of *N*1-phenylbenzene-1,4-diamine. Off-white solid. MS (ISP): 366.1 ([M-H]⁻).

### Example 129 (provided for reference)

### 6-Chloro-N-(2-oxo-2,3-dihydro-1H-benzo[d]imidazol-5-yl)indoline-1-carboxamide

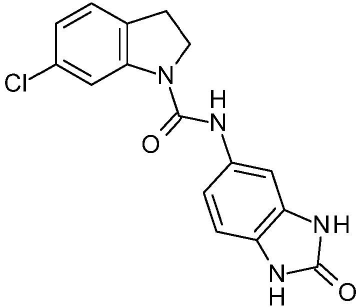

The title compound was obtained in analogy to example 87 using 6-chloroindoline¹⁶² in place of *N*1-phenylbenzene-1,4-diamine. Off-white solid. MS (ISP): 329.1 ([M+H]⁺) 329.1 ({³⁵Cl}[M+H]⁺), 331.1 ({³⁷Cl}[M+H]⁺).

### Example 130 (provided for reference)

### N-(3-(1-Methyl-1H-pyrazol-3-yl)phenyl)-2-oxo-2,3-dihydro-1H-benzo[d]imidazole-5-carboxamide

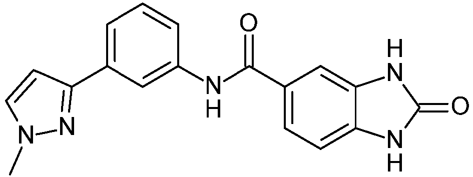

The title compound was obtained in analogy to example 22 using 3-(1-methyl-1H-pyrazol-3-yl)aniline¹⁶³ in place of 1-(piperazin-1-yl)ethanone. Off-white solid. MS (ISP): 334.1 ([M+H]⁺).

### Example 131

### 2-(4-Methoxycyclohexyl)-N-methyl-2'-oxo-2',3'-dihydro-1'H-[1,5'-bibenzo[d]imidazole]-5-carboxamide

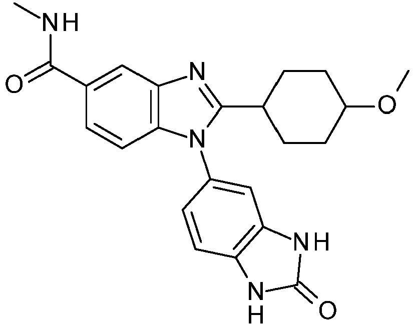

The title compound was obtained in analogy to example 2 using 4-methoxycyclohexane-1-carbaldehyde ¹⁶⁴ in place of 2-(3-methylimidazol-4-yl)quinoline-4-carbaldehyde. Off-white solid. MS (ISP): 420.2 ([M+H]⁺).

### Example 132 (provided for reference)

### 3-Methoxy-N-(2-oxo-2,3-dihydro-1H-benzo[d]imidazol-5-yl)isonicotinamide

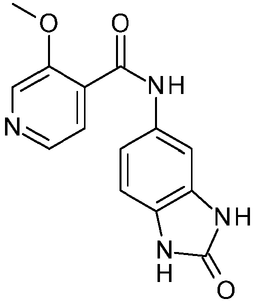

The title compound was obtained in analogy to example 68 using 3-methoxyisonicotinic acid¹⁶⁵ in place of benzofuran-3-carboxylic acid. Off-white solid. MS (ISP): 285.1 ([M+H]⁺).

### Example 133 (provided for reference)

### N2- Methyl-N5-(2-oxo-2,3-dihydro-1H-benzo[d]imidazol-5-yl)pyridine-2,5-dicarboxamide

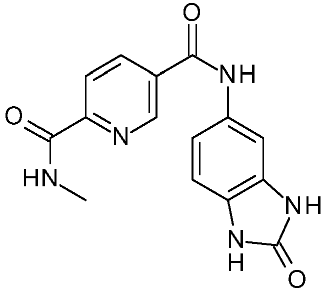

The title compound was obtained in analogy to example 68 using 6-(methylcarbamoyl)nicotinic acid¹⁶⁶ in place of benzofuran-3-carboxylic acid. Light grey solid. MS (ISP): 312.1 ([M+H]⁺).

### Example 134 (provided for reference)

### N-(2-Oxo-2,3-dihydro-1H-benzo[d]imidazol-5-yl)piperidine-4-sulfonamide

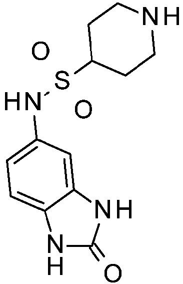

A solution of *tert*-butyl 4-(*N*-(2-oxo-2,3-dihydro-1*H*-benzo[d]imidazol-5-yl)sulfamoyl)piperidine-1-carboxylate (55 mg, example 125) and 4 M hydrogen chloride solution in 1,4-dioxane (1.05 g, 1 ml, 4 mmol, Eq: 28.8) in 1,4-dioxane (1 ml) was stirred at room temperature for 15 h. LC-MS analysis indicated that the reaction was finished. The solid product was collected by filtration, washing with 1,4-dioxane, to afford *N*-(2-oxo-2,3-dihydro-1*H-*benzo[d]imidazol-5-yl)piperidine-4-sulfonamide (36 mg, 78%) as its hydrochloride salt as an off-white solid. MS (ISP): 295.1 ([M-H]⁻).

### Example 135 (provided for reference)

### 4-(4-Chlorobenzyl)-N-(2-oxo-2,3-dihydro-1H-benzo[d]imidazol-5-yl)piperazine-1-carboxamide

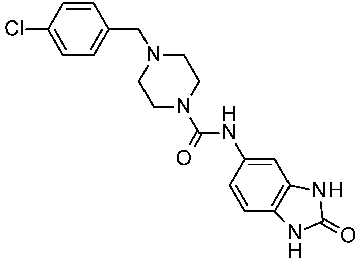

The title compound was obtained in analogy to example 87 using 1-(4-chlorobenzyl)piperazine¹⁶⁷ in place of *N*1-phenylbenzene-1,4-diamine. White solid. MS (ISP): 386.1 ({³5Cl}[M+H]⁺), 388.1 ({³⁷Cl}[M+H]⁺).

### Example 136 (provided for reference)

### N-(2-Oxo-2,3-dihydro-1H-benzo[d]imidazol-5-yl)-2-(pyridin-4-yl)benzamide

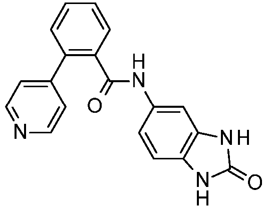

The title compound was obtained in analogy to example 68 using 2-(pyridin-4-yl)benzoic acid¹⁶⁸ in place of benzofuran-3-carboxylic acid. White solid. MS (ISP): 331.1 ([M+H]⁺).

### Example 137 (provided for reference)

### N-(2-Oxo-2,3-dihydro-1H-benzo[d]imidazol-5-yl)benzofuran-3-sulfonamide

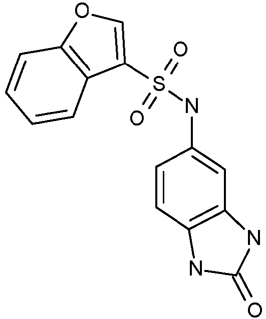

The title compound was obtained in analogy to example 118 using benzofuran-3-sulfonyl chloride ¹⁶⁹ in place of methyl 4-(chlorosulfonyl)benzoate. Off-white solid. MS (ISP): 330.1 ([M+H]⁺).

### Example 138 (provided for reference)

### 2-(3-Methoxyphenyl)-N-(2-oxo-2,3-dihydro-1H-benzo[d]imidazol-5-yl)nicotinamide

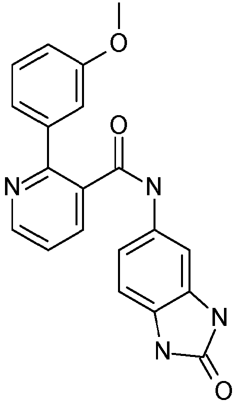

The title compound was obtained in analogy to example 68 using 2-(3-methoxyphenyl)nicotinic acid¹⁷⁰ in place of benzofuran-3-carboxylic acid. White solid. MS (ISP): 361.1 ([M+H]⁺).

### Example 139 (provided for reference)

### N-(1-Benzylpiperidin-4-yl)-2-oxo-2,3-dihydro-1H-benzo[d]imidazole-5-carboxamide

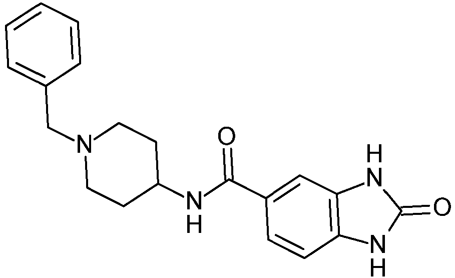

To a stirred suspension of methyl 2-oxo-2,3-dihydro-1*H*-benzo[d]imidazole-5-carboxylate**^{Error! Bookmark not defined}.** (100 mg) and 1-benzylpiperidin-4-amine¹⁷¹ (119 mg) in toluene (2 ml) under argon was added dropwise trimethylaluminum (520 µl, 2 M solution in toluene) to give a brown solution. The reaction mixture was stirred at 110 °C for 2 h. LC-MS analysis indicated that the reaction was complete. The reaction mixture was quenched by dropwise addition to water (10 ml). THF (50 ml) was then added. The resulting mixture was stirred at room temperature for 1 h. The mixture was then washed twice with saturated brine. The organic layer was dried over Na₂SO₄ and concentrated *in vacuo.* The crude material was triturated in EtOAc (4 ml), then filtered through sintered glass, washing with EtOAc (2 x) and then with Et₂O (2 x). The solid crystals were dried *in vacuo* to afford 124 mg of an off-white solid. The impure solid was triturated with methanol (5 ml), then filtered through sintered glass, washing with methanol (1 x), and then with Et₂O (2 x). The crystalline product was dried *in vacuo* to afford *N*-(1-benzylpiperidin-4-yl)-2-oxo-2,3-dihydro-1*H*-benzo[d]imidazole-5-carboxamide (83 mg, 46%) as a white solid. MS (ISP): 351.1 ([M+H]⁺).

### Example 140 (provided for reference)

### 5-(5-Aminoindolin-1-yl)-1,3-dihydro-2H-benzo[d]imidazol-2-one hydrochloride

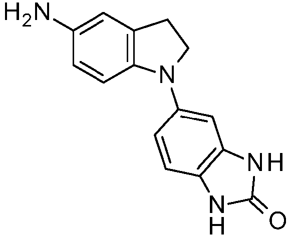

The title compound was obtained as its hydrochloride salt in analogy to example 58 using tert-butyl indolin-5-ylcarbamate¹⁵⁴ in place of tert-butyl piperazine-1-carboxylate in step a. Grey solid. MS (ISP): 267.1 ([M+H]⁺).

### Example 141 (provided for reference)

### N-(1-(2-Oxo-2,3-dihydro-1H-benzo[d]imidazol-5-yl)indolin-5-yl)acetamide

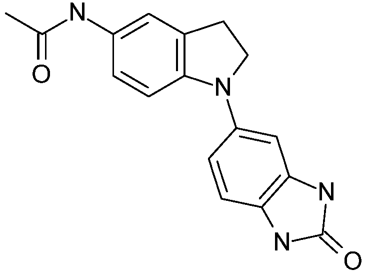

To a stirred suspension of 5-(5-aminoindolin-1-yl)-1,3-dihydro-2*H*-benzo[d]imidazol-2-one dihydrochloride (47 mg) and triethylamine (96.6 µl) in dichloromethane (3 ml) was added dropwise acetic anhydride (19.6 µl). The reaction mixture was stirred at room temperature for 2 h. LC-MS analysis indicated that the reaction was complete. The reaction mixture was poured into THF/EtOAc (3 :1) and extracted sequentially with water and with saturated brine. The organic layer was dried over Na₂SO₄ and concentrated *in vacuo.* The crude material was purified by flash chromatography (silica gel, eluant: 0% to 10% MeOH in dichloromethane) to afford N-(1-(2-oxo-2,3-dihydro-1H-benzo[d]imidazol-5-yl)indolin-5-yl)acetamide (24 mg, 56%) as an off-white solid. MS (ISP): 309.2 ([M+H]⁺).

### Example 142 (provided for reference)

### N-(3-(Oxazol-5-yl)phenyl)-2-oxo-2,3-dihydro-1H-benzo[d]imidazole-5-carboxamide

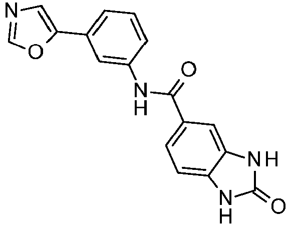

The title compound was obtained in analogy to example 139 using 3-(oxazol-5-yl)aniline¹⁷² in place of 1-benzylpiperidin-4-amine. Off-white solid. MS (ISP): 321.1 ([M+H]⁺).

### Example 143 (provided for reference)

### tert-Butyl 4-((2-oxo-2,3-dihydro-1H-benzo[d]imidazol-5-yl)carbamoyl)indoline-1-carboxylate

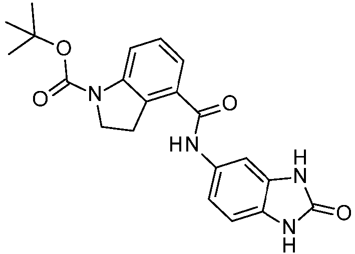

The title compound was obtained in analogy to example 68 using 1-(*tert-*butoxycarbonyl)indoline-4-carboxylic acid¹⁷³ in place of benzofuran-3-carboxylic acid. Off-white solid. MS (ISP): 393.3 ([M-H]⁻).

### Example 144 (provided for reference)

### N-(2-Oxo-2,3-dihydro-1H-benzo[d]imidazol-5-yl)indoline-4-carboxamide

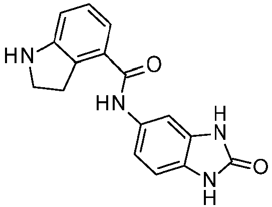

To a stirred suspension of *tert*-butyl 4-((2-oxo-2,3-dihydro-1*H*-benzo[d]imidazol-5-yl)carbamoyl)indoline-1-carboxylate (120 mg) in dichloromethane (5 ml) was added a solution of 4 M HCl in dioxane (1.52 ml). The reaction mixture was stirred at room temperature for 2 h. An additional aliquot of 4 M HCl in dioxane (761 µl) was added and the reaction mixture was stirred at room temperature for a further 2 h. The reaction mixture was filtered through a sintered glass funnel to afford *N*-(2-oxo-2,3-dihydro-1*H*-benzo[d]imidazol-5-yl)indoline-4-carboxamide hydrochloride (95 mg, 94% yield) as its hydrochloride salt as an off-white solid. MS (ISP): 295.2 ([M+H]⁺).

### Example 145 (provided for reference)

### 1-Acetyl-N-(2-oxo-2,3-dihydro-1H-benzo[d]imidazol-5-yl)indoline-4-carboxamide

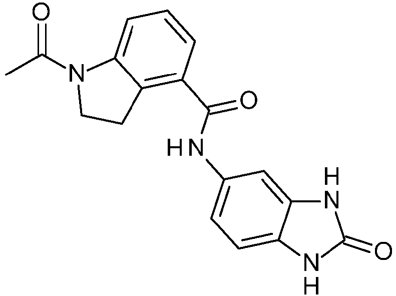

To a stirred suspension of *N*-(2-oxo-2,3-dihydro-1*H*-benzo[d]imidazol-5-yl)indoline-4-carboxamide hydrochloride (95 mg) and triethylamine (160 µl) in dichloromethane (3 ml) was added dropwise acetic anhydride (40.6 µl). The reaction mixture was stirred at room temperature for 2 h. LC-MS analysis indicated that the reaction was complete. The reaction mixture was poured into THF/EtOAc (3: 1) and extracted with water. The aqueous layer was filtered through a sintered glass funnel. The solid was collected and DMSO (2 ml) was added to give a suspension. The suspension was filtered through a sintered glass funnel and washed with water (20 ml). The solid was collected and dried *in vacuo* to afford 1-acetyl-*N*-(2-oxo-2,3-dihydro-1*H*-benzo[d]imidazol-5-yl)indoline-4-carboxamide (35 mg, 36.%) as a white solid. MS (ISP): 337.2 ([M+H]⁺).

### Example 146 (provided for reference)

### N-(4-(((2-Oxo-2,3-dihydro-1H-benzo[d]imidazol-5-yl)methyl)amino)phenyl)acetamide

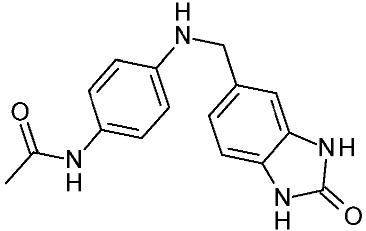

To a stirred suspension of 2-oxo-2,3-dihydro-1*H*-benzo[d]imidazole-5-carbaldehyde**^{Error! Bookmark not defined}**. (100 mg) and *N*-(4-aminophenyl)acetamide¹⁴⁵ (97.5 mg) in methanol (8 ml) were added zinc chloride (336 mg) and sodium cyanoborohydride (116 mg). The reaction mixture was shaken at 60 °C for 3 h. LC-MS analysis indicated that the reaction was complete. The reaction mixture was added to water. THF was added, then 1 N aq. HCl was added to adjust the pH to 3. The reaction mixture was then stirred at room temperature for 10 min. The mixture was extracted with saturated brine. The organic layer was dried over Na₂SO₄ and concentrated *in vacuo.* The crude material was purified by flash chromatography (silica gel, eluant: 0% to 10% MeOH in dichloromethane) to afford *N*-(4-(((2-oxo-2,3-dihydro-1*H*-benzo[d]imidazol-5-yl)methyl)amino)phenyl)acetamide (65 mg, 36%) as a yellow solid. MS (ISP): 297.1 ([M+H]⁺).

### Example 147 (provided for reference)

### N-(2-(Methylcarbamoyl)phenyl)-2-oxo-2,3-dihydro-1H-benzo[d]imidazole-5-carboxamide

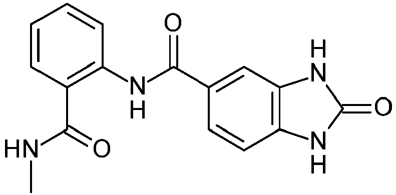

The title compound was obtained in analogy to example 139 using 2-amino-N-methylbenzamide ¹⁷⁴ in place of 1-benzylpiperidin-4-amine. White solid. MS (ISP): 311.1 ([M+H]⁺).

### Example 148 (provided for reference)

### tert-Butyl (1-((2-oxo-2,3-dihydro-1H-benzo[d]imidazol-5-yl)methyl)indolin-5-yl)carbamate

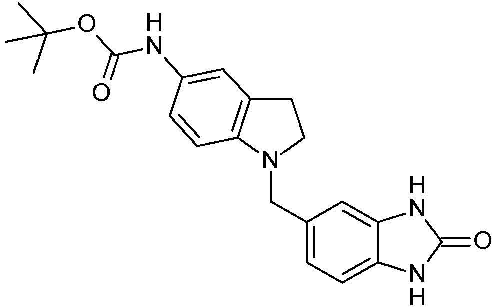

The title compound was obtained in analogy to example 146 using tert-butyl indolin-5-ylcarbamate¹⁵⁴ in place of *N*-(4-aminophenyl)acetamide. White solid. MS (ISP): 381.1 ([M+H]⁺).

### Example 149 (provided for reference)

### 5-((5-Aminoindolin-1-yl)methyl)-1,3-dihydro-2H-benzo[d]imidazol-2-one

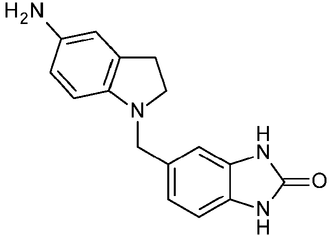

To a stirred suspension of tert-butyl (1-((2-oxo-2,3-dihydro-1*H*-benzo[d]imidazol-5-yl)methyl)indolin-5-yl)carbamate (64 mg) in dichloromethane (5 ml) at room temperature was added a solution of 4 N HCl in dioxane (841 µl). The reaction mixture was stirred at room temperature overnight. The reaction mixture was filtered through a Sartorius funnel, washing twice with diethyl ether), and the solid was dried *in vacuo* to afford 5-((5-aminoindolin-1-yl)methyl)-1,3-dihydro-2H-benzo[d]imidazol-2-one (74 mg, quant.) as its hydrochloride salt as a white solid. MS (ISP): 281.1 ([M+H]⁺).

### Example 150 (provided for reference)

### N-(1-((2-Oxo-2,3-dihydro-1H-benzo[d]imidazol-5-yl)methyl)indolin-5-yl)acetamide

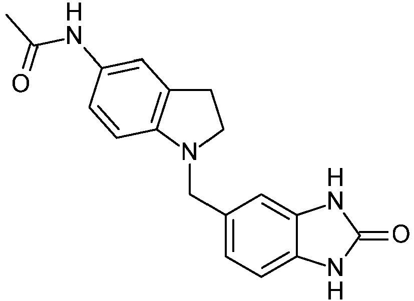

To a stirred suspension of 5-((5-aminoindolin-1-yl)methyl)-1,3-dihydro-2*H-*benzo[d]imidazol-2-one hydrochloride (50 mg) and triethylamine (88 µl) in dichloromethane (3 ml) at room temperature was added dropwise acetic anhydride (22.3 µl). The reaction mixture was stirred at room temperature for 2 h. LC-MS analysis indicated that the reaction was complete. The reaction mixture was poured into THF/EtOAc (4: 1) and extracted with saturated brine. The organic layer was dried over Na₂SO₄ and concentrated *in vacuo.* The crude material was purified by flash chromatography (silica gel, eluant: 0% to 10% MeOH in dichloromethane) to afford *N*-(1-((2-oxo-2,3-dihydro-1*H*-benzo[d]imidazol-5-yl)methyl)indolin-5-yl)acetamide (10 mg, 20%) as an off-white solid. MS (ISP): 323.2 ([M+H]⁺).

### Example 151 (provided for reference)

### Methyl 2-((2-oxo-2,3-dihydro-1H-benzo[d]imidazol-5-yl)carbamoyl)benzoate

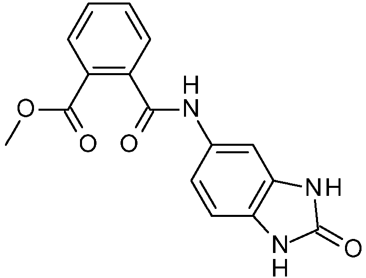

The title compound was obtained in analogy to example 68 using 2-(methoxycarbonyl)benzoic acid¹⁷⁵ in place of benzofuran-3-carboxylic acid. White solid. MS (ISP): 312.1 ([M+H]⁺).

### Example 152 (provided for reference)

### 5-((1-Benzylpiperidin-4-yl)amino)-1,3-dihydro-2H-benzo[d]imidazol-2-one

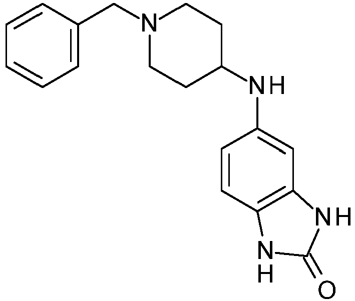

The title compound was obtained in analogy to example 58 steps a-c using 1-benzylpiperidin-4-amine¹⁷¹ in place of tert-butyl piperazine-1-carboxylate in step a. Purple solid. MS (ISP): 323.1 ([M+H]⁺).

### Example 153 (provided for reference)

### Methyl 6-((2-oxo-2,3-dihydro-1H-benzo[d]imidazol-5-yl)amino)nicotinate

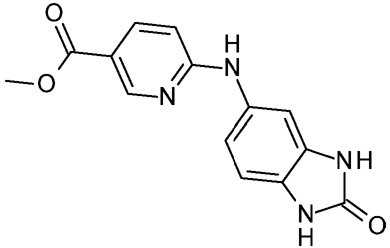

The title compound was obtained in analogy to example 58 steps a-c using methyl 6-aminonicotinate ¹⁷⁶ in place of tert-butyl piperazine-1-carboxylate, potassium tert-butoxide in place of N,Ndiisopropylethylamine, and DMSO in place of acetonitrile in step a. Light yellow solid. MS (ISP): 285.1 ([M+H]⁺).

### Example 154 (provided for reference)

### 5-(Benzyloxy)-1,3-dihydro-2H-benzo[d]imidazol-2-one

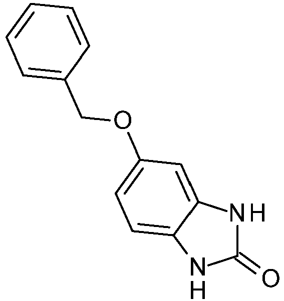

### a) 4-(Benzyloxy)-1,2-dinitro-benzene

To a stirred suspension of benzyl alcohol (1 ml) and potassium hydroxide (49.7 mg) at room temperature was added 4-fluoro-1,2-dinitrobenzene^{**Error! Bookmark not defined**.} (150 mg) and tris[2-(2-methoxyethoxy)ethyl]amine (25.8 µl). The reaction mixture was stirred at room temperature for 15 h. LC-MS analysis indicated that the reaction was complete. The reaction mixture was partitioned between ethyl acetate (75 ml) and water (40 ml). The layers were separated and the aqueous layer was extracted with two 30 ml portions of ethyl acetate. The combined organic layers were washed sequentially with one 40 ml portion of water and one 40 ml portion of saturated brine, then dried over anhydrous sodium sulfate and concentrated *in vacuo.* The crude material was purified by flash chromatography (SiO2, eluant: n-heptane/ethyl acetate: 100:0 to 75:25) to give, after concentration under high vacuum, 4-(benzyloxy)-1,2-dinitro-benzene (242 mg, 93%) as a light yellow oil which was used in the next step without further purification.

### b) 4-(Benzyloxy)benzene-1,2-diamine

To a solution of 4-(benzyloxy)-1,2-dinitrobenzene (247 mg) and 37% aq. HCl (2 ml) in ethanol (2 ml) at room temperature was added tin (II) chloride dihydrate (711 mg). The reaction mixture was stirred at 60 °C for 5 h. LC-MS analysis indicated that the reaction was finished. The reaction mixture was quenched at 0-5 °C with 2 M aqueous sodium hydroxide solution and then stirred for 10 min. The reaction mixture was partitioned between 1:1 ethyl acetate/THF (50 ml) and 1 M aqueous sodium hydroxide solution (30 ml). The layers were separated. The aqueous layer was extracted once with ethyl acetate (40 ml). The combined organic layers were washed once with saturated brine (30 ml), dried over anhydrous sodium sulfate, and concentrated *in vacuo* to afford 4-(benzyloxy)benzene-1,2-diamine (148 mg, 77%) as a yellow solid which was used in the next step without further purification. MS (ISP): 215.0 ([M+H]⁺).

### c) 5-(Benzyloxy)-1,3-dihydro-2H-benzo[d]imidazol-2-one

To a stirred suspension of 4-(benzyloxy)benzene-1,2-diamine (148 mg) in tetrahydrofuran (3 ml) at room temperature was added CDI (168 mg). The reaction mixture was stirred at room temperature for 30 min. LC-MS analysis indicated that the reaction was finished. The reaction mixture was partitioned between 1:1 ethyl acetate/THF (50 ml) and saturated brine (30 ml). The layers were separated and the organic layer was washed with once with saturated brine (20 ml), dried over anhydrous sodium sulfate, and concentrated *in vacuo.* Preparative reverse-phase HPLC (Gemini NX 3 um, 50 mm x 4.6 mm column) afforded 5-(benzyloxy)-1,3-dihydro-2*H-*benzo[d]imidazol-2-one (32 mg, 19%) as a white solid. MS (ISP): 241.1 ([M+H]⁺).

### Example 155 (provided for reference)

### Methyl 2-((2-oxo-2,3-dihydro-1H-benzo[d]imidazol-5-yl)methyl)isoindoline-5-carboxylate

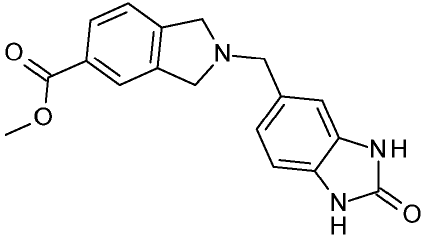

The title compound was obtained in analogy to example 146 using methyl isoindoline-5-carboxylate¹⁷⁷ in place of *N*-(4-aminophenyl)acetamide. Grey solid. MS (ISP): 324.1 ([M+H]⁺).

### Example 156 (provided for reference)

### N-Methyl-4-((2-oxo-2,3-dihydro-1H-benzo[d]imidazol-5-yl)oxy)benzamide

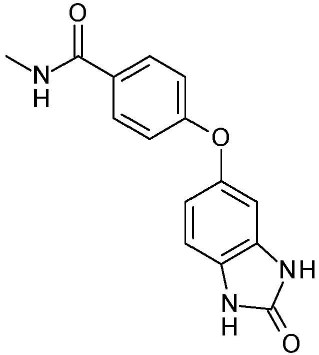

The title compound was obtained in analogy to example 154 using 4-hydroxy-N-methylbenzamide ¹⁷⁸ in place of benzyl alcohol in step a. Off-white solid. MS (ISP): 284.1 ([M+H]⁺).

### Example A (provided for reference)

### N-(5-(2-((6S)-4-(4-chlorophenyl)-2,3,9-trimethyl-6H-thieno[3,2-f][1,2,4]triazolo[4,3-a][1,4]diazepin-6-yl)acetamido)pentyl)-2-ethyl-2'-oxo-2',3'-dihydro-1'H-[1,5'-bibenzo[d]imidazole]-5-carboxamide

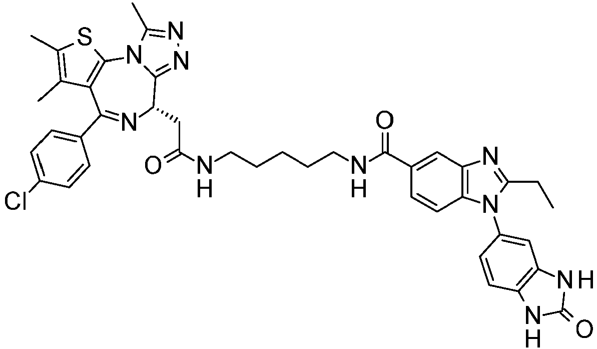

### a) tert-Butyl (5-(2-((6S)-4-(4-chlorophenyl)-2,3,9-trimethyl-6H-thieno[3,2-f][1,2,4]triazolo[4,3-a][1,4]diazepin-6-yl)acetamido)pentyl)carbamate

To a solution of 2-((6*S*)-4-(4-chlorophenyl)-2,3,9-trimethyl-6*H*-thieno[3,2-f][1,2,4]triazolo[4,3-a][1,4]diazepin-6-yl)acetic acid (150 mg) in dichloromethane (15 ml) was added 1-ethyl-3-(3-dimethylaminopropyl)carbodiimide¹⁷⁹ (108 mg) and DMAP (54.9 mg). After stirring at room temperature for 10 minutes, tert-butyl (5-aminopentyl)carbamate¹⁸⁰ (75.7 mg) was added and the resulting solution was stirred at room temperature for 48 h. The reaction mixture was partioned between water and dichloromethane, 1 M aq. HCl was added and the mixture was separated. The organic layer was dried over MgSO₄, filtered and concentrated *in vacuo.* The crude material was purified by flash chromatography (silica gel, eluent: 0 to 5% of methanol in dichloromethane) to afford *tert*-butyl (5-(2-((6*S*)-4-(4-chlorophenyl)-2,3,9-trimethyl-6*H-*thieno[3,2-f][1,2,4]triazolo[4,3-a][1,4]diazepin-6-yl)acetamido)pentyl)carbamate (124mg, 57%) as a light brown oil. MS (ISP): 585.2 ([M+H]⁺).

### b) (S)-N-(5-Aminopentyl)-2-(4-(4-chlorophenyl)-2,3,9-trimethyl-6H-thieno[3,2-f][1,2,4]triazolo[4,3-a][1,4]diazepin-6-yl)acetamide hydrochloride

To a solution of *tert*-butyl (5-(2-((6*S*)-4-(4-chlorophenyl)-2,3,9-trimethyl-6*H*-thieno[3,2-f][1,2,4]triazolo[4,3-a][1,4]diazepin-6-yl)acetamido)pentyl)carbamate (124 mg) in dioxane (2 ml) was added a 4 M solution of HCl in dioxane (795 µl. The reaction mixture was stirred at room temperature for 1 h. The solid was filtered to afford (*S*)-*N*-(5-aminopentyl)-2-(4-(4-chlorophenyl)-2,3,9-trimethyl-6*H*-thieno[3,2-f][1,2,4]triazolo[4,3-a][1,4]diazepin-6-yl)acetamide hydrochloride (98 mg, 89%) which was used in the next step without further purification. MS (ISP): 485.1 ([M+H]⁺).

### c) Methyl 2-ethyl-2'-oxo-2',3'-dihydro-1'H-[1,5'-bibenzo[d]imidazole]-5-carboxylate

The title compound was obtained in analogy to example 1 starting from methyl 3-amino-4-((2-oxo-2,3-dihydro-1*H*-benzo[d]imidazol-5-yl)amino)benzoate (example 9).

### d) 2-Ethyl-2'-oxo-2'3'-dihydro-1'H-[1,5'-bibenzo[d]imidazole]-5-carboxylic acid

To a solution of methyl 2-ethyl-2'-oxo-2',3'-dihydro-1'*H*-[1,5'-bibenzo[d]imidazole]-5-carboxylate (155 mg) in MeOH (1 ml) and THF (2 ml) was added 1 M aq. LiOH (1.38 ml). The reaction mixture was stirred at room temperature for 20 h. LC-MS analysis showed that the reaction was not complete. 1 M aq. LiOH (1 mL) was added and the reaction mixture was stirred overnight. The volatiles were evaporated and a solution of 2 M aq. HCl was added to the remaining aqueous phase until pH= 0. The resulting light yellow suspension was stirred at 0 °C for 15 min and was filtered to give 2-ethyl-2'-oxo-2',3'-dihydro-1'*H*-[1,5'-bibenzo[d]imidazole]-5-carboxylic acid (125 mg, 84%) which was used in the next step without further purification. MS (ISP): 323.1 ([M+H]⁺).

### e) N-(5-(2-((6S)-4-(4-Chlorophenyl)-2,3,9-trimethyl-6H-thieno[3,2-f][1,2,4]triazolo[4,3-a][1,4]diazepin-6-yl)acetamido)pentyl)-2-ethyl-2'-oxo-2',3'-dihydro-1'H-[1,5'-bibenzo[d]imidazole]-5-carboxamide

To 2-ethyl-2'-oxo-2',3'-dihydro-1*'H-*[1,5'-bibenzo[d]imidazole]-5-carboxylic acid (50 mg) was added a solution of HATU in DMF (692 µl, 0.944 g/ml). *N,N*-Diisopropylethylamine (108 µl) and *N*-(5-aminopentyl)-2-((6*S*)-4-(4-chlorophenyl)-2,3,9-trimethyl-6*H*-thieno[3,2-f][1,2,4]triazolo[4,3-a][1,4]diazepin-6-yl)acetamide (75.2 mg) were added. The reaction mixture was shaken at room temperature for 4 hours and then transferred to a separating funnel. Water and ethyl acetate were added to give a sticky solid. After removal of the aqueous and organic layer, the sticky solid was recovered by dissolving in CH₂Cl₂/MeOH (90:10). The organic layer was dried over MgSO4, filtered and concentrated *in vacuo.* The crude material was purified by flash chromatography (silica gel, eluent: 0 to 20% of methanol in dichloromethane) to afford N-(5-(2-((6*S*)-4-(4-chlorophenyl)-2,3,9-trimethyl-6*H*-thieno[3,2-f][1,2,4]triazolo[4,3-a][1,4]diazepin-6-yl)acetamido)pentyl)-2-ethyl-2'-oxo-2',3'-dihydro-1'*H*-[1,5'-bibenzo[d]imidazole]-5-carboxamide (30mg, 25%) as a white solid. MS (ISP): 789.29 ([M+H]⁺).

### Example B (provided for reference)

### (S)-N-(2-(2-(2-(2-(4-(4-Chlorophenyl)-2,3,9-trimethyl-6H-thieno[3,2-f][1,2,4]triazolo[4,3-a][1,4]diazepin-6-yl)acetamido)ethoxy)ethoxy)ethyl)-2-ethyl-2'-oxo-2',3'-dihydro-1'H-[1,5'-bibenzo[d]imidazole ]-5-carboxamide

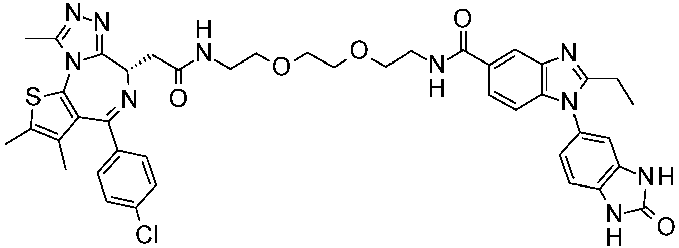

### a) tert-Butyl (2-(2-(2-(2-((6S)-4-(4-chlorophenyl)-2,3,9-trimethyl-6H-thieno[3,2-f][1,2,4]triazolo[4,3-a][1,4]diazepin-6-yl)acetamido)ethoxy)ethoxy)ethyl)carbamate

To a solution of 2-((6*S*)-4-(4-chlorophenyl)-2,3,9-trimethyl-6*H*-thieno[3,2-f][1,2,4]triazolo[4,3-a][1,4]diazepin-6-yl)acetic acid (150 mg) in dichloromethane (15 ml) were added 1-ethyl-3-(3-dimethylaminopropyl)carbodiimide (108 mg) and DMAP (54.9 mg). After stirring at room temperature for 10 minutes, *tert*-butyl (5-aminopentyl)carbamate¹⁸¹ (75.7 mg) was added and the resulting solution was stirred at room temperature for 48 h. The reaction mixture was partioned between water and dichloromethane, then 1 M aq. HCl was added and the mixture was separated. The organic layer was dried over MgSO₄, filtered and concentrated *in vacuo.* The crude material was purified by flash chromatography (silica gel, eluent: 0 to 5% of methanol in dichloromethane) to afford *tert*-butyl (2-(2-(2-(2-((6*S*)-4-(4-chlorophenyl)-2,3,9-trimethyl-6*H-*thieno[3,2-f][1,2,4]triazolo[4,3-a][1,4]diazepin-6-yl)acetamido)ethoxy)ethoxy)ethyl)carbamate (213mg, 90%) as a colorless oil. MS (ISP): 631.4 ([M+H]⁺).

### b) N-(2-(2-(2-Aminoethoxy)ethoxy)ethyl)-2-((6S)-4-(4-chlorophenyl)-2,3,9-trimethyl-6H-thieno[3,2-f][1,2,4]triazolo[4,3-a][1,4]diazepin-6-yl)acetamide hydrochloride

To a solution of *tert*-butyl (2-(2-(2-(2-((6*S*)-4-(4-chlorophenyl)-2,3,9-trimethyl-6*H-*thieno[3,2-f][1,2,4]triazolo[4,3-a][1,4]diazepin-6-yl)acetamido)ethoxy)ethoxy)ethyl)carbamate (213 mg) in dioxane (2 ml) was added a solution of 4 M HCl in dioxane (1.27 ml). The reaction was stirred at room temperature for 1 h. The solvent was evaporated and the residue was dissolved in a minimum of ethanol, then diethyl ether was added dropwise. The solid obtained was filtered to afford *N*-(2-(2-(2-aminoethoxy)ethoxy)ethyl)-2-((6*S*)-4-(4-chlorophenyl)-2,3,9-trimethyl-6*H-*thieno[3,2-f][1,2,4]triazolo[4,3-a][1,4]diazepin-6-yl)acetamide hydrochloride (105 mg, 55%) which was used in the next step without further purification. MS (ISP): 531.3 ([M+H]⁺).

### c) (S)-N-(2-(2-(2-(2-(4-(4-Chlorophenyl)-2,3,9-trimethyl-6H-thieno[3,2-f][1,2,4]triazolo[4,3-a][1,4]diazepin-6-yl)acetamido)ethoxy)ethoxy)ethyl)-2-ethyl-2'-oxo-2',3'-dihydro-1'H-[1,5'-bibenzo[d]imidazole ]-5-carboxamide

To 2-ethyl-2'-oxo-2',3'-dihydro-1'*H*-[1,5'-bibenzo[d]imidazole]-5-carboxylic acid (30 mg) in DMF (1 ml) were added (*S*)*-N*-(2-(2-(2-aminoethoxy)ethoxy)ethyl)-2-(4-(4-chlorophenyl)-2,3,9-trimethyl-6*H*-thieno[3,2-f][1,2,4]triazolo[4,3-a][1,4]diazepin-6-yl)acetamide hydrochloride (52.8 mg), HATU (42.5 mg) and *N*,*N*-diisopropylethylamine (65 µl). The reaction mixture was shaken at room temperature for 4 hours then transferred to a separating funnel. LC/MS analysis indicated that the reaction was finished. Water and ethyl acetate were added to give a sticky solid. After removal of the aqueous and organic layer, the sticky solid was recovered by dissolving in CH₂Cl₂/MeOH (90:10). The organic layer was dried over MgSO₄, filtered and concentrated *in vacuo.* The crude material was purified by flash chromatography (silica gel, eluent: 0 to 20% of methanol in dichloromethane) to afford (*S*)-*N*-(2-(2-(2-(2-(4-(4-chlorophenyl)-2,3,9-trimethyl-6*H*-thieno[3,2-f][1,2,4]triazolo[4,3-a][1,4]diazepin-6-yl)acetamido)ethoxy)ethoxy)ethyl)-2-ethyl-2'-oxo-2',3'-dihydro-1'*H*-[1,5'-bibenzo[d]imidazole ]-5-carboxamide (23.6 mg, 30%) as a white solid. MS (ISP): 835.29 ([M+H]⁺).

### Example C (provided for reference)

### (S)-N-(2-(2-(2-(2-(4-(4-Chlorophenyl)-2,3,9-trimethyl-6H-thieno[3,2-f][1,2,4]triazolo[4,3-a][1,4]diazepin-6-yl)acetamido)ethoxy)ethoxy)ethyl)-2'-oxo-2-(quinolin-4-yl)-2',3'-dihydro-1'H-[1,5'-bibenzo[d ]imidazole]-5-carboxamide

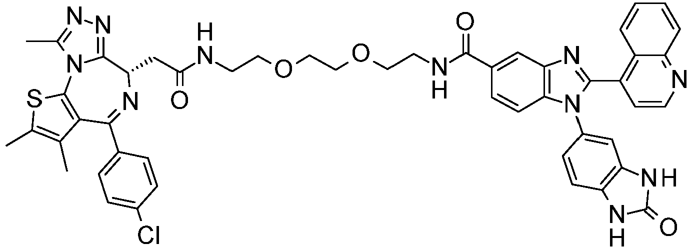

### a) Methyl 2'-oxo-2-(quinolin-4-yl)-2',3'-dihydro-1'H-[1,5'-bibenzo[d]imidazole]-5-carboxylate

The title compound was obtained in analogy to example 1 starting from methyl 3-amino-4-((2-oxo-2,3-dihydro-1H-benzo[d]imidazol-5-yl)amino)benzoate (example 9).

### b) 2'-Oxo-2-(quinolin-4-yl)-2',3'-dihydro-1'H-[1,5'-bibenzo[d]imidazole]-5-carboxylic acid

To a solution of methyl 2'-oxo-2-(quinolin-4-yl)-2',3'-dihydro-1'*H*-[1,5'-bibenzo[d]imidazole]-5-carboxylate (90 mg) in MeOH (1 ml) and THF (2 ml) was added 1 M aq. LiOH (1.41 ml). The reaction mixture was stirred at room temperature for 20 h.The reaction mixture was partioned between ethyl acetate and water. The organic layer was discarded and the aqueous layer was acidified by addition of a solution of 2 M aq. HCl until pH= 0. The aqueous phase was extracted with a 1:1 mixture of ethyl acetate and THF. The organic layer was dried over MgSO₄, filtered and concentrated *in vacuo* to give 2'-oxo-2-(quinolin-4-yl)-2',3'-dihydro-1'*H-*[1,5'-bibenzo[d]imidazole]-5-carboxylic acid (90 mg, 46 %) which was used in the next step without further purification. MS (ISP): 422.2 ([M+H]⁺).

### c) (S)-N-(2-(2-(2-(2-(4-(4-Chlorophenyl)-2,3,9-trimethyl-6H-thieno[3,2-f][1,2,4]triazolo[4,3-a][1,4]diazepin-6-yl)acetamido)ethoxy)ethoxy)ethyl)-2'-oxo-2-(quinolin-4-yl)-2',3'-dihydro-1'H-[1,5'-bibenzo[d]imidazole]-5-carboxamide

To a solution of 2'-oxo-2-(quinolin-4-yl)-2',3'-dihydro-1'*H*-[1,5'-bibenzo[d]imidazole]-5-carboxylic acid (33.7 mg) in DMF (1 ml) were added (*S*)-*N*-(2-(2-(2-aminoethoxy)ethoxy)ethyl)-2-(4-(4-chlorophenyl)-2,3,9-trimethyl-6*H*-thieno[3,2-f][1,2,4]triazolo[4,3-a][1,4]diazepin-6-yl)acetamide hydrochloride (50 mg), HATU (36.5 mg) and *N,N*-diisopropylethylamine (56 µl). The reaction mixture was shaken at room temperature for 4 hours and then transferred to a separating funnel. LC/MS analysis indicated that the reaction was finished. Water and ethyl acetate were added to give a sticky solid. After removal of the aqueous and organic layer, the sticky solid was recovered by dissolving in CH₂Cl₂/MeOH (90:10). The organic layer was dried over MgSO₄, filtered and concentrated *in vacuo.* The crude material was purified by flash chromatography (silica gel, eluent: 0 to 20% of methanol in dichloromethane) to afford (*S*)-*N*-(2-(2-(2-(2-(4-(4-chlorophenyl)-2,3,9-trimethyl-6*H*-thieno[3,2-f][1,2,4]triazolo[4,3-a][1,4]diazepin-6-yl)acetamido)ethoxy)ethoxy)ethyl)-2'-oxo-2-(quinolin-4-yl)-2',3'-dihydro-1'*H*-[1,5'-bibenzo[d]imidazole]-5-carboxamide (37.6mg, 50.3%) as a white solid. MS (ISP): 934.30 ([M+H]⁺).

### Example D (provided for reference)

### N4-[5-[[2-[(9S)-7-(4-Chlorophenyl)-4,5,13-trimethyl-3-thia-1,8,11,12-tetrazatricyclo[8.3.0.02,6]trideca-2(6),4,7,10,12-pentaen-9-yl]acetyl]amino]pentyl]-N1-(2-oxo-1,3-dihydrobenzimidazol-5-yl)indoline-1,4-dicarboxamide

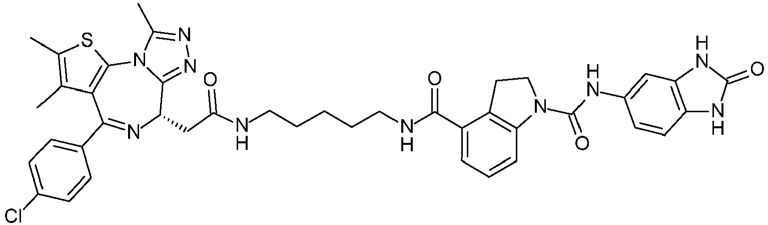

### a) Methyl 1-((2-oxo-2,3-dihydro-1H-benzo[d]imidazol-5-yl)carbamoyl)indoline-4-carboxylate

To a stirred solution of methyl indoline-4-carboxylate (119 mg) and triethylamine (178 µl) in THF (2 ml) was added triphosgene (73.6 mg) at 0-5 ° C. The reaction mixture was stirred at 25 °C for 1 hour then a solution of 5-amino-1,3-dihydrobenzimidazol-2-one (100 mg) in N,N-dimethylformamide (1ml) was added. The reaction mixture was stirred at 25 °C for 1 hour According to LC/MS,the reaction was finished. The reaction was quenched by addition of water (10 ml) and the suspension was stirred for 10 min. The solid was collected by filtration and was suspended in a 1:1 mixture of DMSO/THF. After stirring, the suspension was filtered and the filtercake was washed with water, dried *in vacuo* to afford methyl 1-((2-oxo-2,3-dihydro-1H-benzo[d]imidazol-5-yl)carbamoyl)indoline-4-carboxylate (236 mg,86 %). Off-white solid. MS (ISP): 353.1 ([M+H]⁺).

### b) 1-((2-Oxo-2,3-dihydro-1H-benzo[d]imidazol-5-yl)carbamoyl)indoline-4-carboxylic acid

To a suspension of methyl 1-((2-oxo-2,3-dihydro-1H-benzo[d]imidazol-5-yl)carbamoyl)indoline-4-carboxylate (120 mg) in a 1:1 mixture of THF/methanol (3 ml) was added a 1 m solution of LiOH (1.36 ml). The reaction mixture was stirred at 25 °C overnight. A solution of 5N HCl was slowly added to the dark brown solution until precipitation. The solid was filtered and the solid was dried *in vacuo.* The filtrate was evaporated, the residue was dissolved and the solution was submit lyophilisation. The solids were mixed together to afford 1-((2-oxo-2,3-dihydro-1H-benzo[d]imidazol-5-yl)carbamoyl)indoline-4-carboxylic acid (67 mg, 58.1 %). Off-white solid. MS (ISP): 339.1 ([M+H]⁺).

### c) N4-[5-[[2-[(9S)-7-(4-Chlorophenyl)-4,5,13-trimethyl-3-thia-1,8,11,12-tetrazatricyclo[8.3.0.02,6]trideca-2(6),4,7,10,12-pentaen-9-yl]acetyl]amino]pentyl]-N1-(2-oxo-1,3-dihydrobenzimidazol-5-yl)indoline-1,4-dicarboxamide

The title compound was obtained in analogy to example A using 1-((2-oxo-2,3-dihydro-1H-benzo[d]imidazol-5-yl)carbamoyl)indoline-4-carboxylic acid in place of 2-ethyl-2'-oxo-2',3'-dihydro-1'*H*-[1,5'-bibenzo[d]imidazole]-5-carboxylic acid for step e. White foam. MS (ISP): 805.3 ([M+H]⁺).
¹ Collins et al., Biochem J. 2017, 474(7), 1127-47
² WO2013020557
³ WO2013063560
⁴ WO 2013106643
⁵ WO2015160845
⁶ WO2016011906
⁷ WO2016105518
⁸ WO2017007612
⁹ WO2017024318
¹⁰ WO2017117473
¹¹ WO2011035124
¹² WO02072576
¹³ WO2010085684
¹⁴ WO2014134240
¹⁵ WO2011130628
¹⁶ WO2007065518
¹⁷ WO2016176460
¹⁸ WO2009135651
¹⁹ WO2011156245
²⁰ WO2017046318
²¹ WO2010130794
²² WO2012021382
²³ WO2005060967
²⁴ WO2012174199
²⁵ EP385850
²⁶ WO2016176449
²⁷ WO2016040508
²⁸ WO0020358
²⁹ EP1256578
³⁰ WO2006058338
³¹ WO2004108133
³² WO2010108187
³³ WO2005113489
³⁴ WO2012061708
³⁵ WO2010023161
³⁶ WO2007041598
³⁷ WO2009050232
³⁸ Terefenko et al., Bioorganic & Med Chem Lett., 2005, 15(15), 3600-03
³⁹ Smolyar et al., Russian Journal of Organic Chemistry 2011, 47(8), 119-3
⁴⁰ Jun Yon Choi et al., J Med Chem, 2012, 55(13), 852-70
⁴¹ Hyeju Choi et al., J. Materials Chem. A, 2014, 2, 12931-9
⁴² Mitsuaki et al., Chem. Pharm. Bull. 1996, 44(9), 1717-22
⁴³ Yu et al., Chemistry of Heterocyclic Compounds 1968, 4(4), 698-701
⁴⁴ Vincent et al., Tetrahedron Lett. 1994, 35(16), 2601-2
⁴⁵ CAS 1493-27-2
⁴⁶ CAS 475216-25-2
⁴⁷ CAS 658700-20-0
⁴⁸ CAS 329-59-9
⁴⁹ CAS 185629-31-6
⁵⁰ CAS 95-23-8
⁵¹ CAS 54732-89-7
⁵² CAS 73778-92-4
³³ CAS 53439-88-6
⁵⁷ Sigma-Aldrich Cat. No. A7089
⁵⁸ Yen, H.-C. S., Xu, Q., Chou, D. M., Zhao, Z. & Elledge, S. J. Global Protein Stability Profiling in Mammalian Cells. Science 2008, 322, 918-923, doi:10.1126/science.1160489
⁵⁹ Birdsall, B., King, R. W., Wheeler, M. R., Lewis, C. A. Jr, Goode, S. R., Dunlap, R. B. & Roberts, G. C. Anal. Biochem. 1983, 132, 353-361
⁶⁰ Eftink Methods Enzymol. 1997, 278, 221-257
⁶¹ CAS 432517-57-2
⁶² CAS 4363-93-3
⁶³ CAS 123-38-6
⁶⁴ CAS 75-07-0
⁶⁵ CAS 100-52-7
⁶⁶ CAS 872-85-5
⁶⁷ CAS 123-72-8
⁶⁸ CAS 3034-50-2
⁶⁹ CAS 78-84-2
⁷⁰ CAS 39513-26-3
⁷¹ CAS 1692-25-7
⁷² CAS 458532-96-2
⁷³ CAS 1218791-01-5
⁷⁴ CAS 13889-98-0
⁷⁵ CAS 160357-94-8
⁷⁶ CAS 496-15-1
⁷⁷ CAS 838891-16-0
⁷⁸ CAS 33259-72-2
⁷⁹ CAS 122-78-1
⁸⁰ CAS 62-53-3
⁸¹ CAS 1553181-04-6
⁸² CAS 65292-99-1
⁸³ CAS 5810-56-0
⁸⁴ CAS 74-89-5
⁸⁵ CAS 36947-68-9
⁸⁶ CAS 616-45-5
⁸⁷ CAS 879658-18-1
⁸⁸ CAS 1013914-22-1
⁸⁹ CAS 335255-72-6
⁹⁰ CAS 335255-70-4
⁹¹ CAS 238765-06-5
⁹² CAS 1521702-21-5
⁹³ CAS 1258867-72-9
⁹⁴ CAS 39513-26-3
⁹⁵ CAS 355386-94-6
⁹⁶ CAS 94-09-7
⁹⁷ CAS 98-80-6
⁹⁸ CAS 40852-06-0
⁹⁹ CAS 33567-59-8
¹⁰⁰ CAS 630-19-3
¹⁰¹ CAS 1214353-57-7
¹⁰² CAS 87120-72-7
¹⁰³ CAS 20870-91-1
¹⁰⁴ CAS 155135-61-8
¹⁰⁵ CAS 3945-69-5
¹⁰⁶ CAS 1388048-97-2
¹⁰⁷ CAS 591-31-1
¹⁰⁸ CAS 57260-71-6
¹⁰⁹ CAS 149771-44-8
¹¹⁰ CAS 79-14-1
¹¹¹ CAS 50675-18-8
¹¹² CAS 17289-26-8
¹¹³ CAS 7126-39-8
¹¹⁴ CAS 26537-68-8
¹¹⁵ CAS 14381-42-1
¹¹⁶ CAS 1197-55-3
¹¹⁷ CAS 85977-52-2
¹¹⁸ CAS 23357-47-3
¹¹⁹ CAS 65-85-0
¹²⁰ CAS 556-08-1
¹²¹ CAS 587-48-4
¹²² CAS 100-61-8
¹²³ CAS 1790471-53-2
¹²⁴ CAS 18699-02-0
¹²⁵ CAS 1781710-76-6
¹²⁶ CAS 107-95-9
¹²⁷ CAS 108-30-5
¹²⁸ CAS 81927-55-1
¹²⁹ CAS 64-18-6
¹³⁰ CAS 50-21-5
¹³¹ CAS 611-72-3
¹³² CAS 291289-41-3
¹³³ CAS 71879-46-4
¹³⁴ CAS 101-54-2
¹³⁵ CAS 3025-95-4
¹³⁶ CAS 25503-90-6
¹³⁷ CAS 141452-01-9
¹³⁸ CAS 3173-56-6
¹³⁹ CAS 635-46-1
¹⁴⁰ CAS 1788023-93-7
¹⁴¹ CAS 1790760-29-0
¹⁴² CAS 939758-71-1
¹⁴³ CAS 22958-64-1
¹⁴⁴ CAS 138-41-0
¹⁴⁵ CAS 122-80-5
¹⁴⁶ CAS 110-89-4
¹⁴⁷ CAS 190661-29-1
¹⁴⁸ CAS 261524-34-9
¹⁴⁹ CAS 72716-87-1
¹⁵⁰ CAS 78471-43-9
¹⁵¹ CAS 21744-88-7
¹⁵² CAS 34603-55-9
¹⁵³ CAS 69812-51-7
¹⁵⁴ CAS 885270-06-4
¹⁵⁵ CAS 941867-91-0
¹⁵⁶ CAS 7111-77-5
¹⁵⁷ CAS 612-35-1
¹⁵⁸ CAS 61324-93-4
¹⁵⁹ CAS 782501-25-1
¹⁶⁰ CAS 372-19-0
¹⁶¹ CAS 35386-24-4
¹⁶² CAS 52537-00-5
¹⁶³ CAS 175202-37-6
¹⁶⁴ CAS 120552-57-0
¹⁶⁵ CAS 654663-32-8
¹⁶⁶ CAS 170464-32-1
¹⁶⁷ CAS 23145-88-2
¹⁶⁸ CAS 133362-99-9
¹⁶⁹ CAS 1051942-46-1
¹⁷⁰ CAS 912773-03-6
¹⁷¹ CAS 50541-93-0
¹⁷² CAS 157837-31-5
¹⁷³ CAS 208774-11-2
¹⁷⁴ CAS 4141-08-6
¹⁷⁵ CAS 4376-18-5
¹⁷⁶ CAS 36052-24-1
¹⁷⁷ CAS 742666-57-5
¹⁷⁸ CAS 27642-27-9
¹⁷⁹ CAS 1892-57-5
¹⁸⁰ CAS 51644-96-3
¹⁸¹ CAS 153086-78-3

## Claims

1. A compound, or a pharmaceutically acceptable salt thereof, of formula: 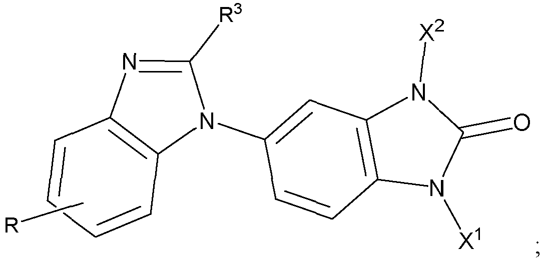 wherein:
X¹ is H or -C₁₋₆alkyl,
X² is H or -C₁₋₆alkyl,
R is H, -C(O)NHMe, or -C(O)OMe;
R³ is independently selected from the group consisting of
i) -(CH₂)₀₋₁-(C₃₋₇cycloalkyl)-aryl substituted by R¹⁰,
ii) -(CH₂)₀₋₁-(C₃₋₇cycloalkyl)-aryl,
iii) -(CH₂)₀₋₁-aryl substituted by R¹⁰,
iv) -(CH₂)₀₋₁-aryl,
v) -(CH₂)₀₋₁-C₃₋₇cycloalkyl substituted by R¹⁰,
vi) -(CH₂)₀₋₁-C₃₋₇cycloalkyl,
vii) -(CH₂)₀₋₁-heteroaryl substituted by R⁹,
viii) -(CH₂)₀₋₁-heteroaryl,
ix) -(CH₂)₀₋₁-heterocycloalkyl,
x) -C(=O)C₁₋₆alkyl
xi) -(CH₂)₀₋₂-C(=O)-N(R⁷,R⁸),
xii) -C(=O)OC₁₋₆alkyl,
xiii) -C₁₋₆alkoxy,
xiv) -C₁₋₆alkyl,
xv) -C₁₋₆alkyl-N(R¹¹)-C(=O)-R¹²,
xvi) -CH₂-O-(CH₂)₀₋₁-aryl substituted by R¹⁰,
xvii) -CH₂-O-(CH₂)₀₋₁-aryl,
xviii) -O-(CH₂)₀₋₁-aryl,
xix) -halogen,
xx) halogen-C₁₋₆alkyl,
xxi) hydroxy-C₁₋₆alkyl
xxii) hydroxy-C₁₋₆alkyl-aryl,
xxiii) -N(R⁵,R⁶),
xxiv) -NH-C(=O)C₁₋₆alkyl,
xxv) -NH-C(=O)OC₁₋₆alkyl, and
xxvi) =O;
R⁵ is each independently selected from H, C₁₋₆alkyl and phenyl,
R⁶ is each independently selected from H and C₁₋₆alkyl,
R⁷ is each independently selected from H and C₁₋₆alkyl,
R⁸ is each independently selected from H and C₁₋₆alkyl,
or R⁷ and R⁸ form together with the nitrogen they are attached to a heterocycloalkyl,
R⁹ is each independently selected from the group consisting of
i) C₁₋₆alkoxy,
ii) C₁₋₆alkyl
iii) halogen,
iv) halogen-C₁₋₆alkyl,
v) heteroaryl, and
vi) heteroaryl substituted by C₁₋₆alkyl or C₁₋₆alkoxy,
R¹⁰ is each independently selected from the group consisting of
i) C₁₋₆alkoxy,
ii) C₁₋₆alkyl
iii) C₁₋₆alkyl-C₁₋₆alkoxy,
iv) halogen, and
v) halogen-C₁₋₆alkyl;
R¹¹ is each independently selected from H and C₁₋₆alkyl,
R¹² is each independently selected from C₁₋₆alkyl and C₃₋₇cycloalkyl, and
for use as therapeutically active substance.

2. The compound, or a pharmaceutically acceptable salt thereof, for use according to claim 1, wherein
R³ is independently selected from the group consisting of
i) -(CH₂)₀₋₁-(C₃₋₇cycloalkyl)-aryl substituted by R¹⁰,
ii) -(CH₂)₀₋₁-(C₃₋₇cycloalkyl)-aryl,
iii) -(CH₂)₀₋₁-aryl substituted by R¹⁰,
iv) -(CH₂)₀₋₁-aryl,
v) -(CH₂)₀₋₁-C₃₋₇cycloalkyl substituted by R¹⁰,
vi) -(CH₂)₀₋₁-C₃₋₇cycloalkyl,
vii) -(CH₂)₀₋₁-heteroaryl substituted by R⁹,
viii) -(CH₂)₀₋₁-heteroaryl,
ix) -(CH₂)₀₋₁-heterocycloalkyl,
x) -C(=O)C₁₋₆alkyl
xi) -C(=O)-N(R⁷,R⁸),
xii) -C(=O)OC₁₋₆alkyl,
xiii) -C₁₋₆alkoxy,
xiv) -C₁₋₆alkyl,
xv) -C₁₋₆alkyl-N(R¹¹)-C(=O)-R¹²,
xvi) -CH₂-O-(CH₂)₀₋₁-aryl substituted by R¹⁰,
xvii) -CH₂-O-(CH₂)₀₋₁-aryl,
xviii) -halogen,
xix) halogen-C₁₋₆alkyl,
xx) hydroxy-C₁₋₆alkyl,
xxi) -N(R⁵,R⁶), and
xxii) -NH-C(=O)C₁₋₆alkyl.

3. The compound, or pharmaceutically acceptable salts thereof, for use according to any one of claims 1-2, selected from the group consisting of 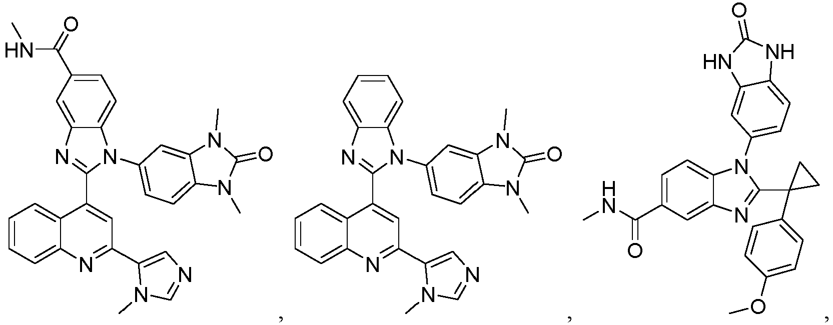 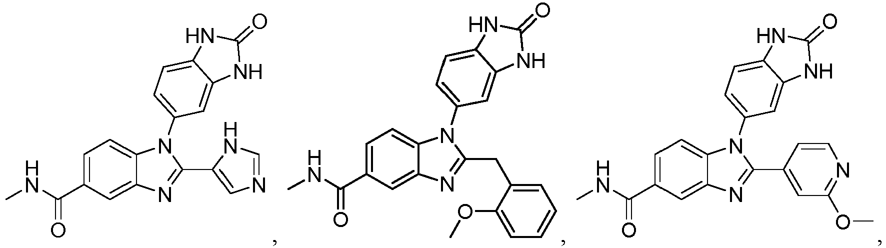 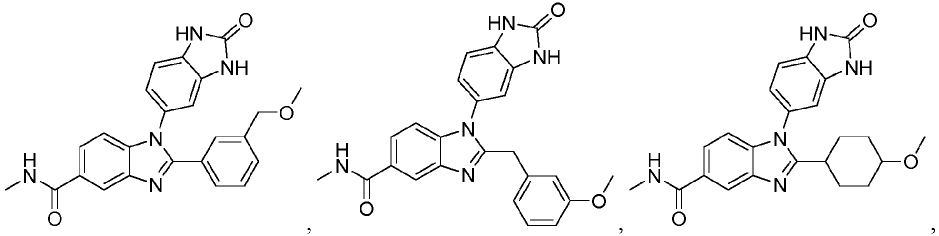 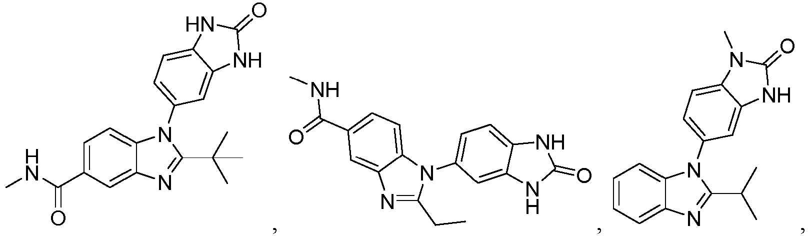 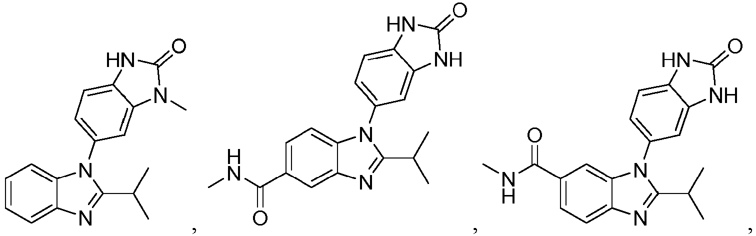 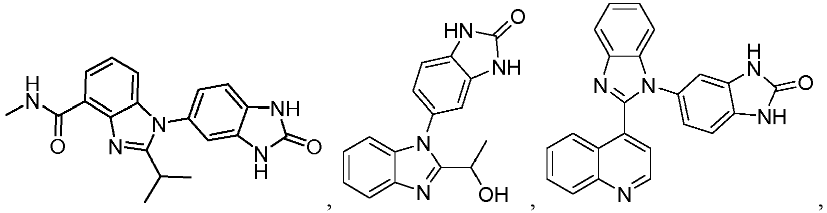 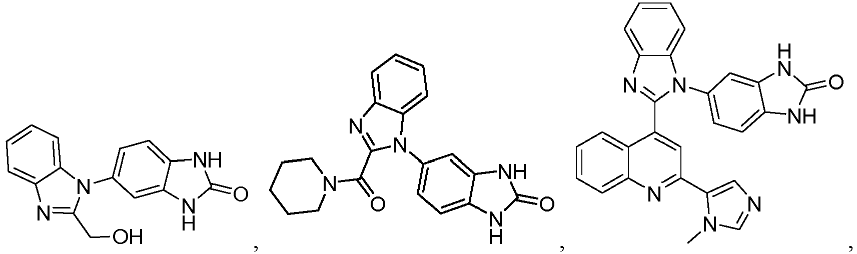 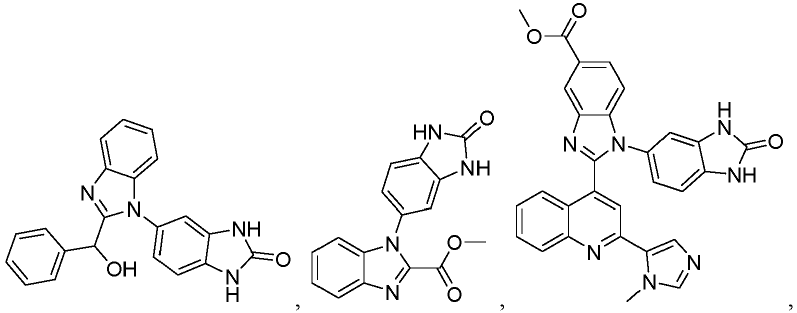 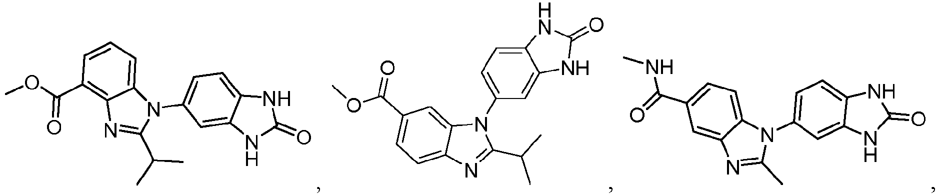 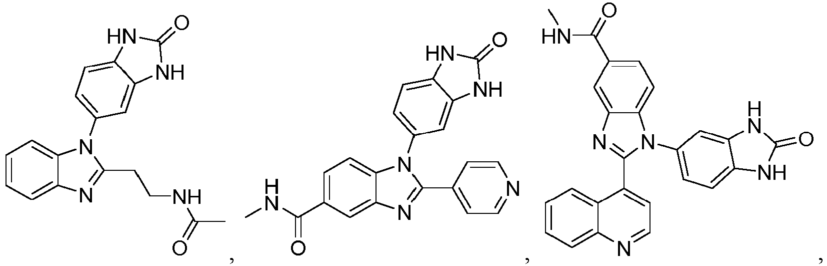 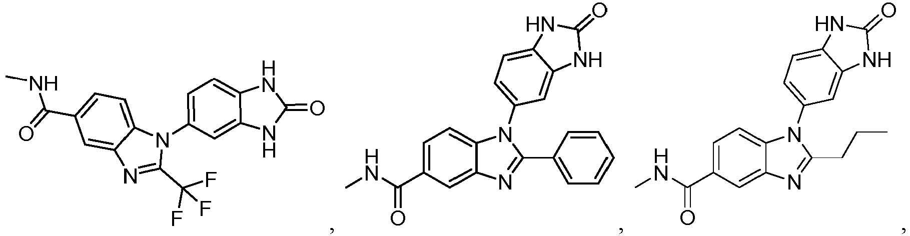 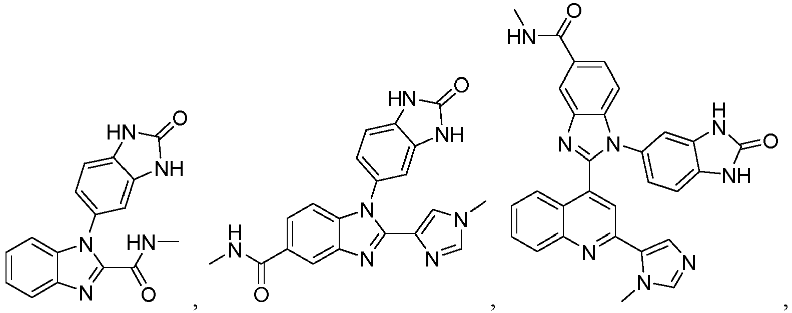 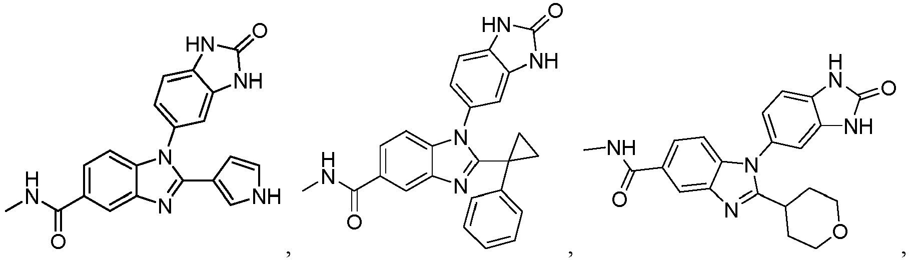 and

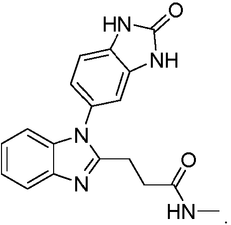

4. The compound, or pharmaceutically acceptable salts thereof, for use according to any one of claims 1-3, selected from the group consisting of

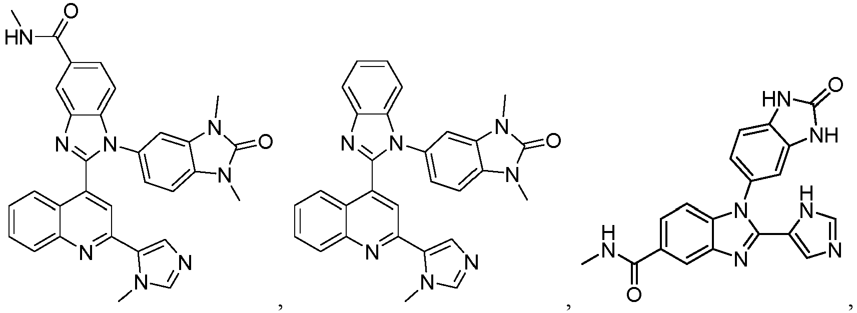

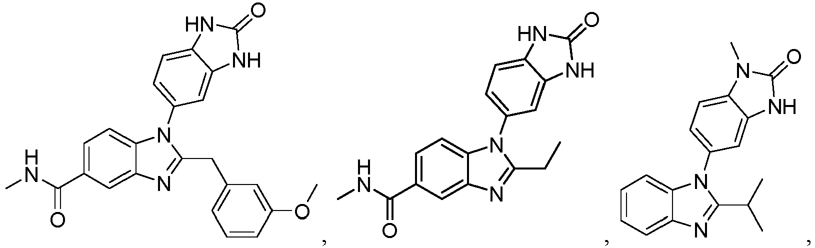

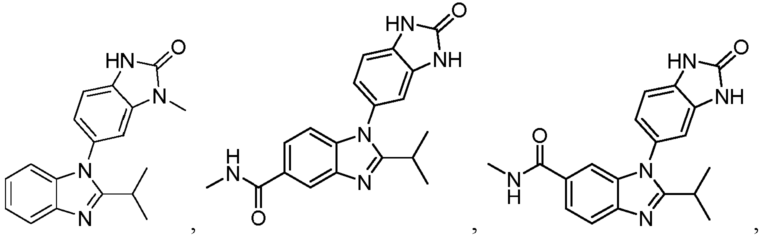

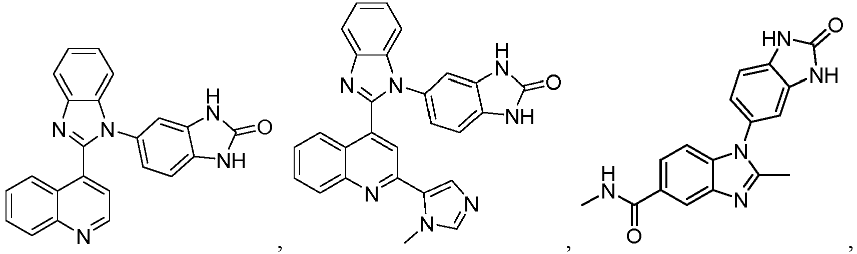

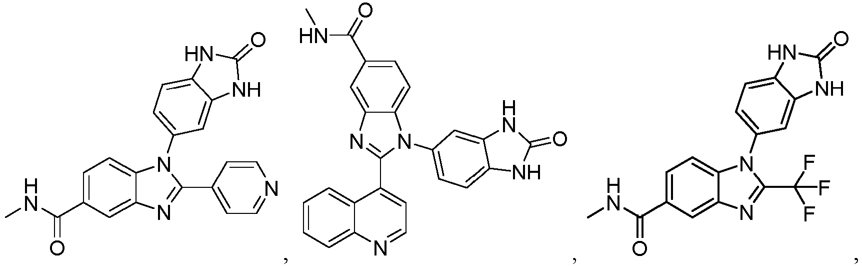

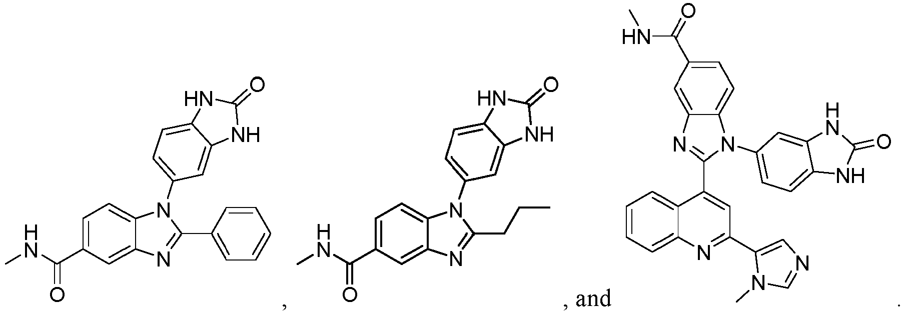

5. A compound, or a pharmaceutically acceptable salt thereof, of formula 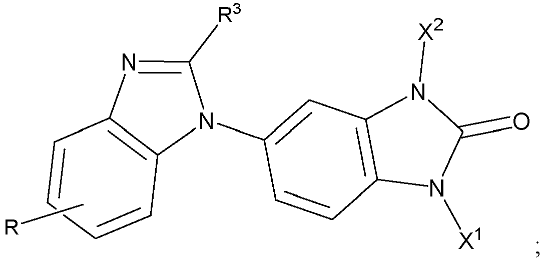 wherein:
X¹ is H or -C₁₋₆alkyl,
X² is H or -C₁₋₆alkyl,
R is H, -C(O)NHMe, or -C(O)OMe;
R³ is independently selected from the group consisting of
i) -(CH₂)₀₋₁-(C₃₋₇cycloalkyl)-aryl substituted by R¹⁰,
ii) -(CH₂)₀₋₁-(C₃₋₇cycloalkyl)-aryl,
iii) -(CH₂)₀₋₁-aryl substituted by R¹⁰,
iv) -(CH₂)₀₋₁-aryl,
v) -(CH₂)₀₋₁-C₃₋₇cycloalkyl substituted by R¹⁰,
vi) -(CH₂)₀₋₁-C₃₋₇cycloalkyl,
vii) -(CH₂)₀₋₁-heteroaryl substituted by R⁹,
viii) -(CH₂)₀₋₁-heteroaryl,
ix) -(CH₂)₀₋₁-heterocycloalkyl,
x) -C(=O)C₁₋₆alkyl
xi) -C(=O)-N(R⁷,R⁸),
xii) -C(=O)OC₁₋₆alkyl,
xiii) -C₁₋₆alkoxy,
xiv) -C₁₋₆alkyl,
xv) -C₁₋₆alkyl-N(R¹¹)-C(=O)-R¹²,
xvi) -CH₂-O-(CH₂)₀₋₁-aryl substituted by R¹⁰,
xvii) -CH₂-O-(CH₂)₀₋₁-aryl,
xviii) -halogen,
xix) halogen-C₁₋₆alkyl,
xx) hydroxy-C₁₋₆alkyl,
xxi) -N(R⁵,R⁶), and
xxii) -NH-C(=O)C₁₋₆alkyl,
R⁵ is each independently selected from H and C₁₋₆alkyl,
R⁶ is each independently selected from H and C₁₋₆alkyl,
R⁷ is each independently selected from H and C₁₋₆alkyl,
R⁸ is each independently selected from H and C₁₋₆alkyl,
or R⁷ and R⁸ form together with the nitrogen they are attached to a heterocycloalkyl,
R⁹ is each independently selected from the group consisting of
i) C₁₋₆alkoxy,
ii) C₁₋₆alkyl
iii) halogen,
iv) halogen-C₁₋₆alkyl,
v) heteroaryl, and
vi) heteroaryl substituted by C₁₋₆alkyl,
R¹⁰ is each independently selected from the group consisting of
i) C₁₋₆alkoxy,
ii) C₁₋₆alkyl,
iii) halogen, and
iv) halogen-C₁₋₆alkyl;
R¹¹ is each independently selected from H and C₁₋₆alkyl, and
R¹² is each independently selected from C₁₋₆alkyl and C₃₋₇cycloalkyl.

6. The compound for use according to claim 1 or the compound according to claim 5, or a pharmaceutically acceptable salt thereof, wherein X¹ and X² are each H.

7. The compound for use according to claim 1 or the compound according to any one of claims 5-6, or a pharmaceutically acceptable salt thereof, wherein R³ is -C(=O)-N(R⁷,R⁸) or -C(=O)OC₁₋₆alkyl, wherein R⁷ and R⁸ is each independently selected from H and C₁₋₆alkyl.

8. The compound for use according to claim 1 or the compound according to any one of claims 5-7, or pharmaceutically acceptable salts thereof, selected from the group consisting of

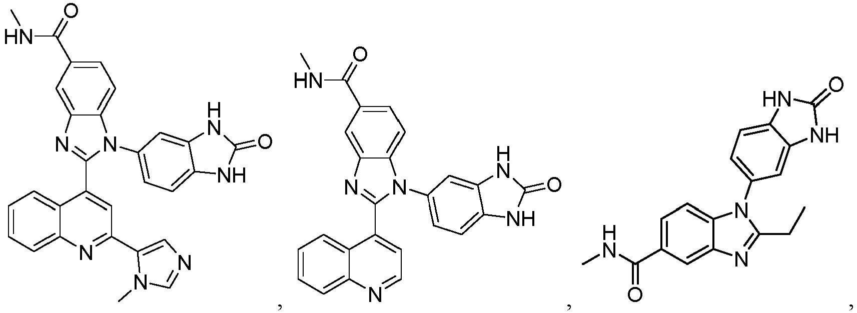

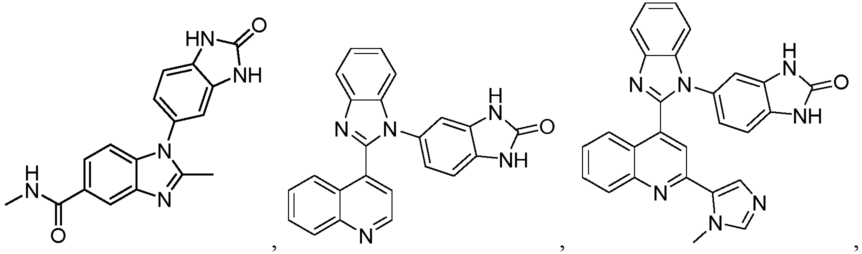

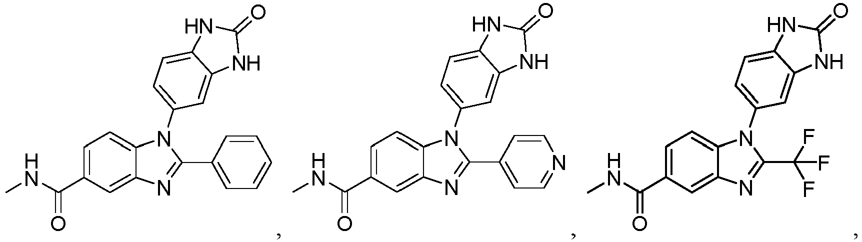

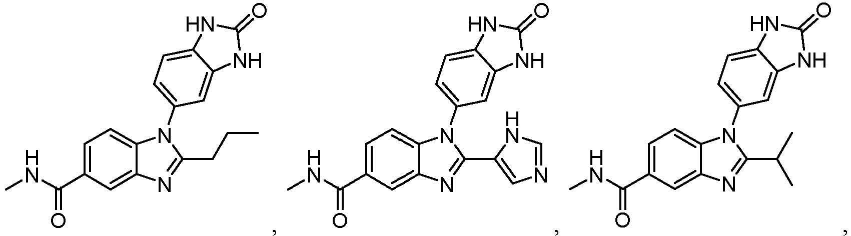

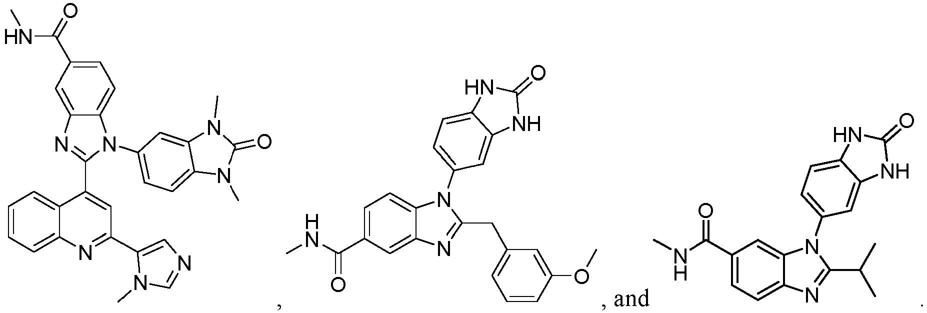

9. The compound according to any one of claims 5-8, or pharmaceutically acceptable salts thereof, for use in the therapeutic and/or prophylactic treatment of cancer.

10. The compound for use according to any one of claims 1-4, or pharmaceutically acceptable salts thereof, wherein the compound is for use in the therapeutic and/or prophylactic treatment of cancer.

11. A pharmaceutical composition comprising a compound as defined in any one of claims 1-8, and a therapeutically inert carrier.

## Patentansprüche

1. Verbindung oder pharmazeutisch unbedenkliches Salz davon der Formel: 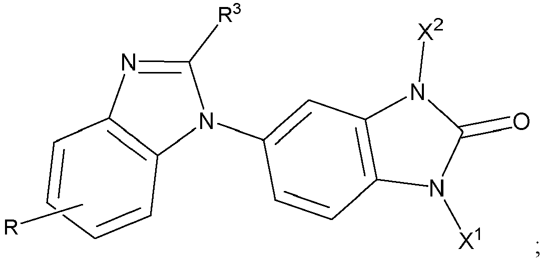 wobei:
X¹ für H oder -C₁₋₆-Alkyl steht,
X² für H oder -C₁₋₆-Alkyl steht,
R für H, -C(O)NHMe oder -C(O)OMe steht;
R³ unabhängig ausgewählt ist aus der Gruppe bestehend aus
i) -(CH₂)₀₋₁-(C₃₋₇-Cycloalkyl) -aryl, das durch R¹⁰ substituiert ist,
ii) - (CH₂)₀₋₁-(C₃₋₇-Cycloalkyl) -aryl,
iii) - (CH₂)₀₋₁-Aryl, das durch R¹⁰ substituiert ist,
iv) -(CH₂)₀₋₁-Aryl,
v) - (CH₂)₀₋₁-C₃₋₇-Cycloalkyl, das durch R¹⁰ substituiert ist,
vi) - (CH₂)₀₋₁-C₃₋₇-Cycloalkyl,
vii) - (CH₂)₀₋₁-Heteroaryl, das durch R⁹ substituiert ist,
viii) - (CH₂)₀₋₁-Heteroaryl,
ix) - (CH₂)₀₋₁-Heterocycloalkyl,
x) -C(=O)-C₁₋₆-Alkyl,
xi) - (CH₂)₀₋₂-C(=O) -N(R⁷,R⁸),
xii) -C(=O)O-C₁₋₆-Alkyl,
xiii) -C₁₋₆-Alkoxy,
xiv) -C₁₋₆-Alkyl,
xv) -C₁₋₆-Alkyl-N(R¹¹)-C (=O)-R¹²,
xvi) -CH₂-O-(CH₂)₀₋₁-Aryl, das durch R¹⁰ substituiert ist,
xvii) -CH₂-O-(CH₂)₀₋₁-Aryl,
xviii) -O- (CH₂)₀₋₁-Aryl,
xix) -Halogen,
xx) Halogen-C₁₋₆-alkyl,
xxi) Hydroxy-C₁₋₆-alkyl,
xxii) Hydroxy-C₁₋₆alkyl-aryl,
xxiii) -N(R⁵,R⁶),
xxiv) -NH-C (=O)-C₁₋₆-Alkyl,
xxv) -NH-C (=O)O-C₁₋₆-Alkyl und
xxvi) =O;
R⁵ jeweils unabhängig aus H, C₁₋₆-Alkyl und Phenyl ausgewählt ist,
R⁶ jeweils unabhängig aus H und C₁₋₆-Alkyl ausgewählt ist,
R⁷ jeweils unabhängig aus H und C₁₋₆-Alkyl ausgewählt ist,
R⁸ jeweils unabhängig aus H und C₁₋₆-Alkyl ausgewählt ist,
oder R⁷ und R⁸ zusammen mit dem Stickstoff, an den sie gebunden sind, ein Heterocycloalkyl bilden,
R⁹ jeweils unabhängig ausgewählt ist aus der Gruppe bestehend aus
i) C₁₋₆-Alkoxy,
ii) C₁₋₆-Alkyl,
iii) Halogen,
iv) Halogen-C₁₋₆-alkyl,
v) Heteroaryl und
vi) Heteroaryl, das durch C₁₋₆-Alkyl oder C₁₋₆-Alkoxy substituiert ist,
R¹⁰ jeweils unabhängig ausgewählt ist aus der Gruppe bestehend aus
i) C₁₋₆-Alkoxy,
ii) C₁₋₆-Alkyl,
iii) C₁₋₆-Alkyl-C₁₋₆-alkoxy,
iv) Halogen und
v) Halogen-C₁₋₆-alkyl;
R¹¹ jeweils unabhängig aus H und C₁₋₆-Alkyl ausgewählt ist,
R¹² jeweils unabhängig aus C₁₋₆-Alkyl und C₃₋₇-Cycloalkyl ausgewählt ist, und
zur Verwendung als therapeutischer Wirkstoff.

2. Verbindung oder pharmazeutisch unbedenkliches Salz davon zur Verwendung nach Anspruch 1, wobei
R³ unabhängig ausgewählt ist aus der Gruppe bestehend aus
i) - (CH₂)₀₋₁-(C₃₋₇-Cycloalkyl) -aryl, das durch R¹⁰ substituiert ist,
ii) - (CH₂)₀₋₁-(C₃₋₇-Cycloalkyl) -aryl,
iii) - (CH₂)₀₋₁-Aryl, das durch R¹⁰ substituiert ist,
iv) -(CH₂)₀₋₁-Aryl,
v) - (CH₂)₀₋₁-C₃₋₇-Cycloalkyl, das durch R¹⁰ substituiert ist,
vi) - (CH₂)₀₋₁-C₃₋₇-Cycloalkyl,
vii) - (CH₂)₀₋₁-Heteroaryl, das durch R⁹ substituiert ist,
viii) - (CH₂)₀₋₁-Heteroaryl,
ix) - (CH₂)₀₋₁-Heterocycloalkyl,
x) -C(=O)-C₁₋₆-Alkyl,
xi) -C(=O)-N(R⁷,R⁸),
xii) -C(=O)O-C₁₋₆-Alkyl,
xiii) -C₁₋₆-Alkoxy,
xiv) -C₁₋₆-Alkyl,
xv) -C₁₋₆-Alkyl-N(R¹¹)-C(=O)-R¹²,
xvi) -CH₂-O-(CH₂)₀₋₁-Aryl, das durch R¹⁰ substituiert ist,
xvii) -CH₂-O-(CH₂)₀₋₁-Aryl,
xviii) -Halogen,
xix) Halogen-C₁₋₆-alkyl,
x) Hydroxy-C₁₋₆-alkyl,
xxi) -N(R⁵,R⁶) und
xxii) -NH-C (=O)-C₁₋₆-Alkyl.

3. Verbindung oder pharmazeutisch unbedenkliche Salze davon zur Verwendung nach einem der Ansprüche 1-2, ausgewählt aus der Gruppe bestehend aus 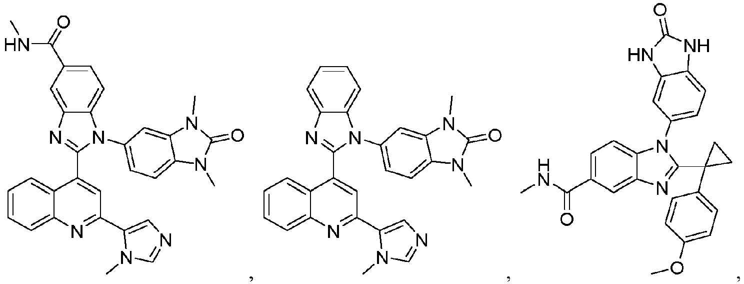 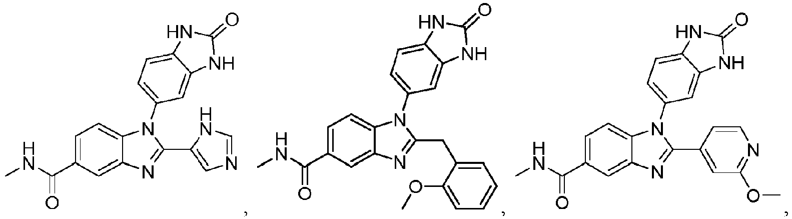 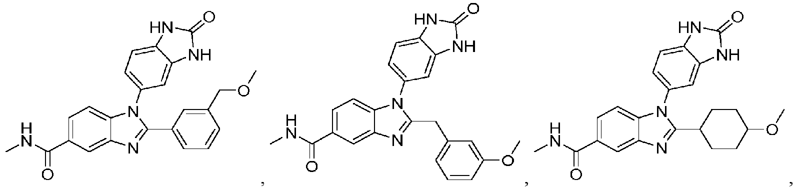 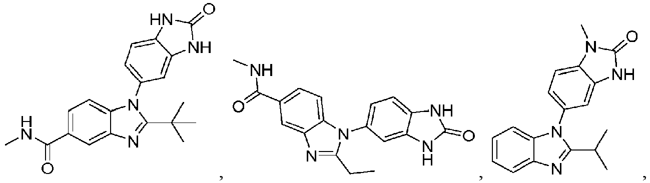 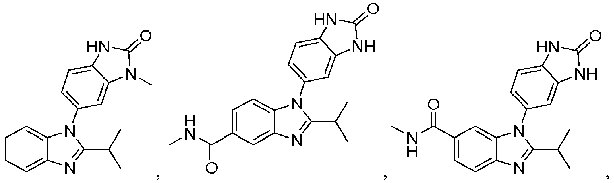 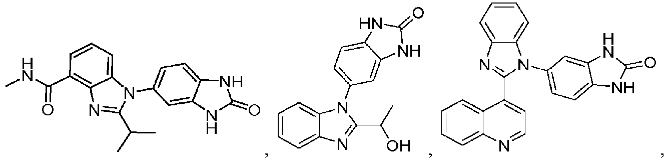 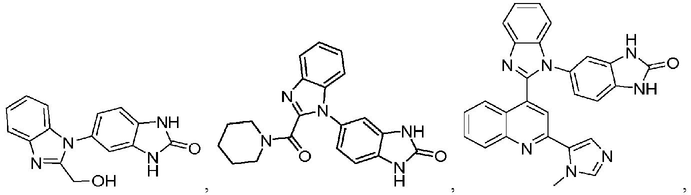 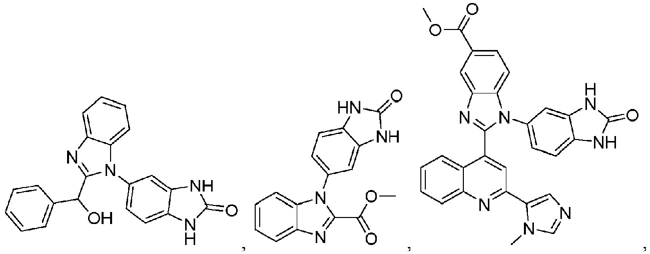 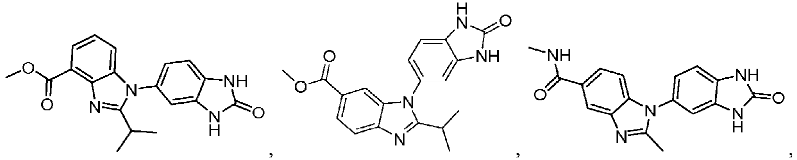 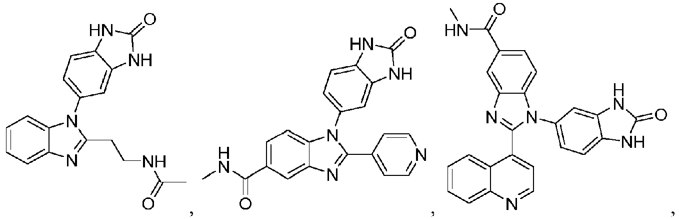 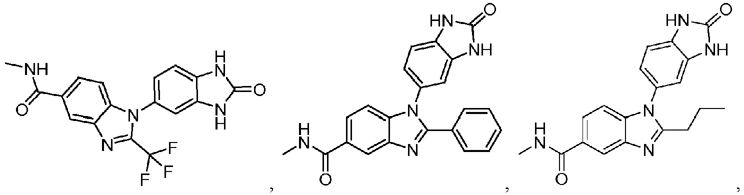 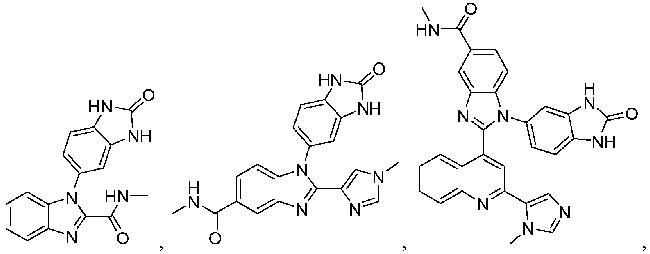 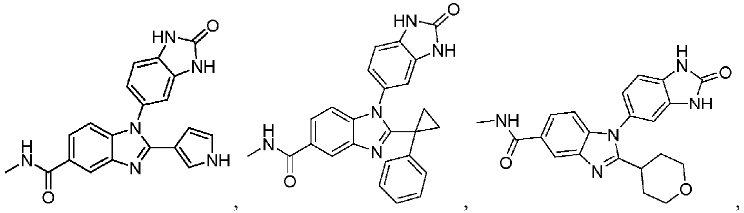 und

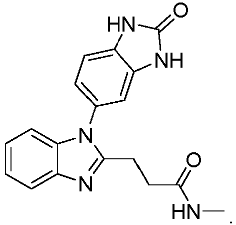

4. Verbindung oder pharmazeutisch unbedenkliche Salze davon zur Verwendung nach einem der Ansprüche 1-3, ausgewählt aus der Gruppe bestehend aus 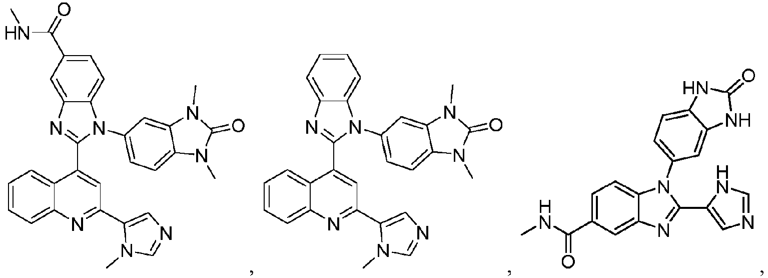 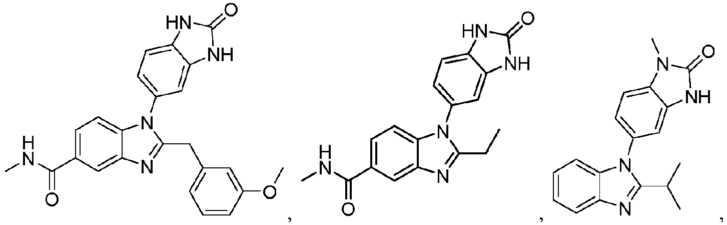 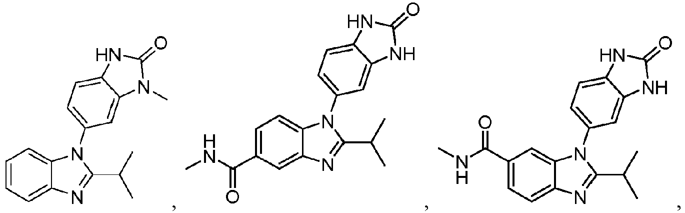 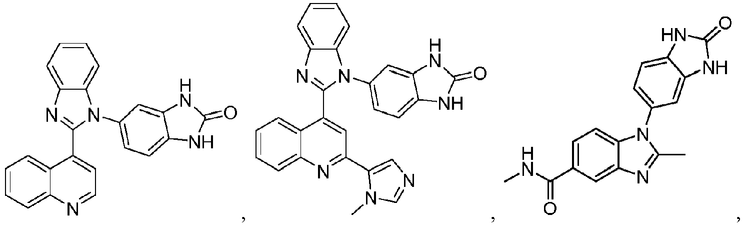 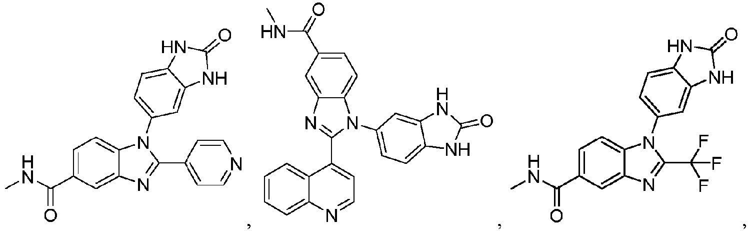 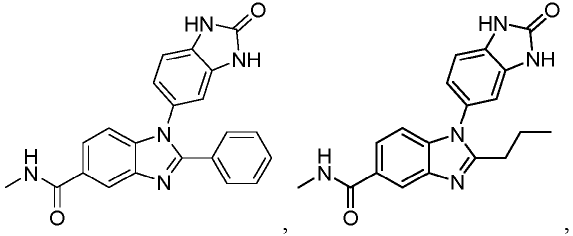 und

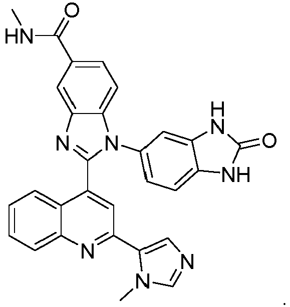

5. Verbindung oder pharmazeutisch unbedenkliches Salz davon der Formel 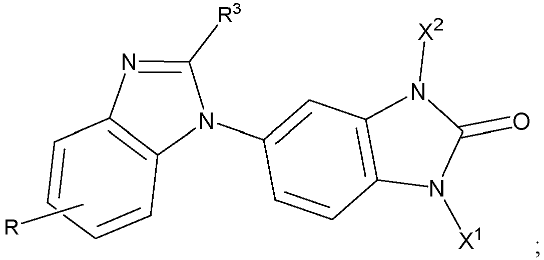 wobei:
X¹ für H oder -C₁₋₆-Alkyl steht,
X² für H oder -C₁₋₆-Alkyl steht,
R für H, -C(O)NHMe oder -C(O)OMe steht;
R³ unabhängig ausgewählt ist aus der Gruppe bestehend aus
i) - (CH₂)₀₋₁-(C₃₋₇-Cycloalkyl) -aryl, das durch R¹⁰ substituiert ist,
ii) - (CH₂)₀₋₁-(C₃₋₇-Cycloalkyl) -aryl,
iii) - (CH₂)₀₋₁-Aryl, das durch R¹⁰ substituiert ist,
iv) -(CH₂)₀₋₁-Aryl,
v) - (CH₂)₀₋₁-C₃₋₇-Cycloalkyl, das durch R¹⁰ substituiert ist,
vi) - (CH₂)₀₋₁-C₃₋₇-Cycloalkyl,
vii) - (CH₂)₀₋₁-Heteroaryl, das durch R⁹ substituiert ist,
viii) - (CH₂)₀₋₁-Heteroaryl,
ix) - (CH₂)₀₋₁-Heterocycloalkyl,
x) -C(=O)-C₁₋₆-Alkyl,
xi) -C(=O)-N(R⁷,R⁸),
xii) -C(=O)O-C₁₋₆-Alkyl,
xiii) -C₁₋₆-Alkoxy,
xiv) -C₁₋₆-Alkyl,
xv) -C₁₋₆-Alkyl-N(R¹¹)-C(=O)-R¹²,
xvi) -CH₂-O-(CH₂)₀₋₁-Aryl, das durch R¹⁰ substituiert ist,
xvii) -CH₂-O-(CH₂)₀₋₁-Aryl,
xviii) -Halogen,
xix) Halogen-C₁₋₆-alkyl,
x) Hydroxy-C₁₋₆-alkyl,
xxi) -N(R⁵,R⁶) und
xxii) -NH-C (=O)-C₁₋₆-Alkyl,
R⁵ jeweils unabhängig aus H und C₁₋₆-Alkyl ausgewählt ist,
R⁶ jeweils unabhängig aus H und C₁₋₆-Alkyl ausgewählt ist,
R⁷ jeweils unabhängig aus H und C₁₋₆-Alkyl ausgewählt ist,
R⁸ jeweils unabhängig aus H und C₁₋₆-Alkyl ausgewählt ist,
oder R⁷ und R⁸ zusammen mit dem Stickstoff, an den sie gebunden sind, ein Heterocycloalkyl bilden,
R⁹ jeweils unabhängig ausgewählt ist aus der Gruppe bestehend aus
i) C₁₋₆-Alkoxy,
ii) C₁₋₆-Alkyl,
iii) Halogen,
iv) Halogen-C₁₋₆-alkyl,
v) Heteroaryl und
vi) Heteroaryl, das durch C₁₋₆-Alkyl substituiert ist, R¹⁰ jeweils unabhängig ausgewählt ist aus der Gruppe bestehend aus
i) C₁₋₆-Alkoxy,
ii) C₁₋₆-Alkyl,
iii) Halogen und
iv) Halogen-C₁₋₆-alkyl;
R¹¹ jeweils unabhängig aus H und C₁₋₆-Alkyl ausgewählt ist und
R¹² jeweils unabhängig aus C₁₋₆-Alkyl und C₃₋₇-Cycloalkyl ausgewählt ist.

6. Verbindung zur Verwendung nach Anspruch 1 oder Verbindung nach Anspruch 5 oder ein pharmazeutisch unbedenkliches Salz davon, wobei X¹ und X² jeweils für H stehen.

7. Verbindung zur Verwendung nach Anspruch 1 oder Verbindung nach einem der Ansprüche 5-6 oder ein pharmazeutisch unbedenkliches Salz davon, wobei R³ für - C(=O)-N(R⁷,R⁸) oder -C(=O)O-C₁₋₆-Alkyl steht, wobei R⁷ und R⁸ jeweils unabhängig aus H und C₁₋₆-Alkyl ausgewählt sind.

8. Verbindung zur Verwendung nach Anspruch 1 oder Verbindung nach einem der Ansprüche 5-7 oder pharmazeutisch unbedenkliche Salze davon, ausgewählt aus der Gruppe bestehend aus 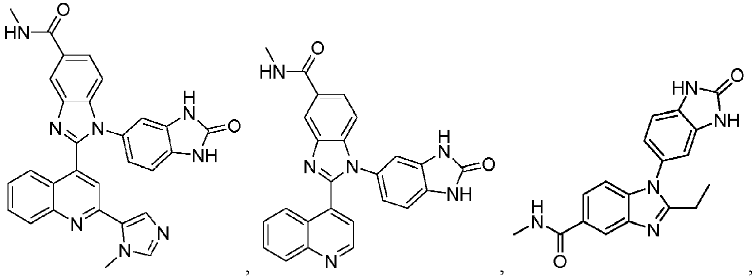 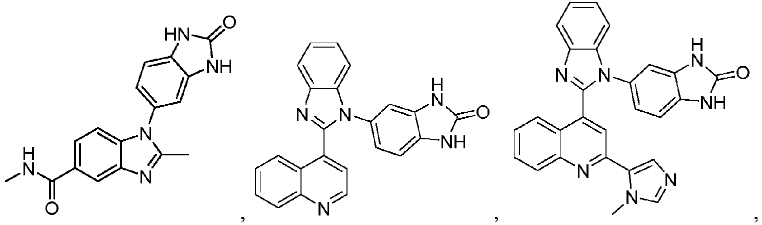 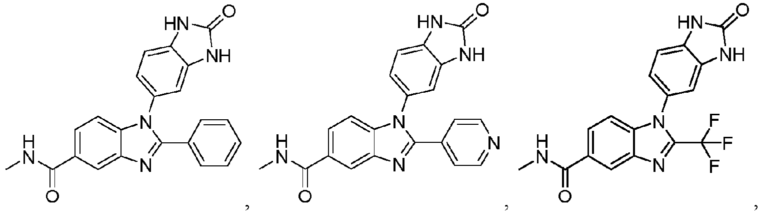 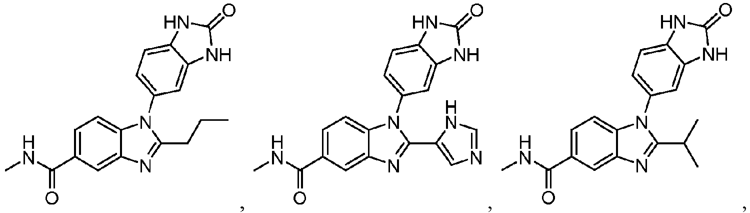 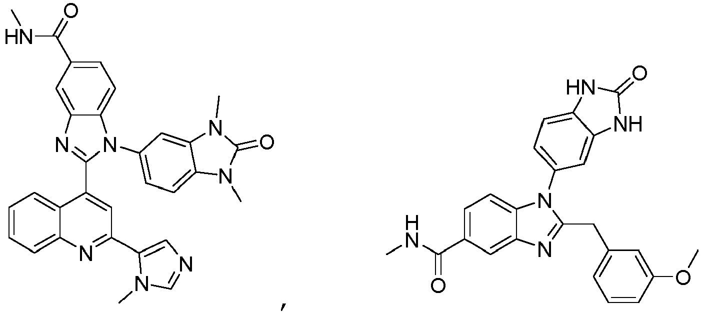 und

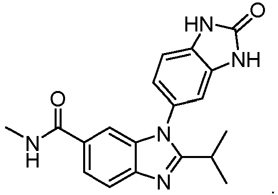

9. Verbindung nach einem der Ansprüche 5-8 oder pharmazeutisch unbedenkliche Salze davon zur Verwendung bei der therapeutischen und/oder prophylaktischen Behandlung von Krebs.

10. Verbindung zur Verwendung nach einem der Ansprüche 1-4 oder pharmazeutisch unbedenkliche Salze davon, wobei die Verbindung zur Verwendung bei der therapeutischen und/oder prophylaktischen Behandlung von Krebs bestimmt ist.

11. Pharmazeutische Zusammensetzung, umfassend eine Verbindung gemäß einem der Ansprüche 1-8 und einen therapeutisch inerten Träger.

## Revendications

1. Composé, ou sel pharmaceutiquement acceptable correspondant, de formule : 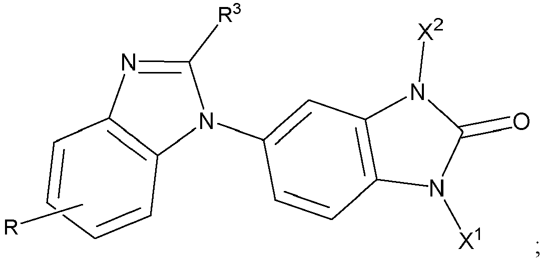
X¹ étant H ou -C₁₋₆alkyle,
X² étant H ou -C₁₋₆alkyle,
R étant H, -C(O)NHMe, ou -C(O)OMe ;
R³ étant indépendamment choisi dans le groupe constitué par
i) -(CH₂)₀₋₁-(C₃₋₇cycloalkyl) -aryle substitué par R¹⁰,
ii) - (CH₂)₀₋₁-(C₃₋₇cycloalkyl) -aryle,
iii) - (CH₂)₀₋₁-aryle substitué par R¹⁰,
iv) - (CH₂)₀₋₁-aryle,
v) - (CH₂)₀₋₁-C₃₋₇cycloalkyle substitué par R¹⁰,
vi) - (CH₂)₀₋₁-C₃₋₇cycloalkyle,
vii) - (CH₂)₀₋₁-hétéroaryle substitué par R⁹,
viii) - (CH₂)₀₋₁-hétéroaryle,
ix) - (CH₂)₀₋₁-hétérocycloalkyle,
x) -C (=O) C₁₋₆alkyle,
xi) - (CH₂)₀₋₂-C(=O) -N(R⁷,R⁸),
xii) -C(=O)OC₁₋₆alkyle,
xiii) -C₁₋₆alcoxy,
xiv) -C₁₋₆alkyle,
xv) -C₁₋₆alkyl-N(R¹¹)-C (=O)-R¹²,
xvi) -CH₂-O-(CH₂)₀₋₁-aryle substitué par R¹⁰,
xvii) -CH₂-O-(CH₂)₀₋₁-aryle,
xviii) -O-(CH₂)₀₋₁-aryle,
xix) halogène,
xx) halogéno-C₁₋₆alkyle,
xxi) hydroxy-C₁₋₆alkyle,
xxii) hydroxy-C₁₋₆alkyl-aryle,
xxiii) -N(R⁵,R⁶),
xxiv) -NH-C(=O)C₁₋₆alkyle,
xxv) -NH-C (=O)OC₁₋₆alkyle, et
xxvi) =O ;
R⁵ étant chacun indépendamment choisi parmi H, C₁₋₆alkyle et phényle,
R⁶ étant chacun indépendamment choisi parmi H et C₁₋₆alkyle,
R⁷ étant chacun indépendamment choisi parmi H et C₁₋₆alkyle,
R⁸ étant chacun indépendamment choisi parmi H et C₁₋₆alkyle,
ou R⁷ et R⁸ formant conjointement avec l'azote auquel ils sont fixés un hétérocycloalkyle,
R⁹ étant chacun indépendamment choisi dans le groupe constitué par
i) C₁₋₆alcoxy,
ii) C₁₋₆alkyle,
iii) halogène,
iv) halogéno-C₁₋₆alkyle,
v) hétéroaryle, et
vi) hétéroaryle substitué par C₁₋₆alkyle ou C₁₋₆alcoxy,
R¹⁰ étant chacun indépendamment choisi dans le groupe constitué par
i) C₁₋₆alcoxy,
ii) C₁₋₆alkyle,
iii) C₁₋₆alkyl-C₁₋₆alcoxy,
iv) halogène, et
v) halogéno-C₁₋₆alkyl;
R¹¹ étant chacun indépendamment choisi parmi H et C₁₋₆alkyle,
R¹² étant chacun indépendamment choisi parmi C₁₋₆alkyle et C₃₋₇cycloalkyle, et
pour utilisation en tant que substance thérapeutiquement active.

2. Composé, ou sel pharmaceutiquement acceptable correspondant, pour utilisation selon la revendication 1,
R³ étant indépendamment choisi dans le groupe constitué par
i) - (CH₂)₀₋₁-(C₃₋₇cycloalkyl)-aryle substitué par R¹⁰,
ii) -(CH₂)₀₋₁-(C₃₋₇cycloalkyl)-aryle,
iii) -(CH₂)₀₋₁-aryle substitué par R¹⁰,
iv) -(CH₂)₀₋₁-aryle,
v) -(CH₂)₀₋₁-C₃₋₇cycloalkyle substitué par R¹⁰,
vi) -(CH₂)₀₋₁-C₃₋₇cycloalkyle,
vii) -(CH₂)₀₋₁-hétéroaryle substitué par R⁹,
viii) -(CH₂)₀₋₁-hétéroaryle,
ix) -(CH₂)₀₋₁-hétérocycloalkyle,
x) -C(=O)C₁₋₆alkyle,
xi) -C(=O)-N(R⁷,R⁸),
xii) -C(=O)OC₁₋₆alkyle,
xiii) -C₁₋₆alcoxy,
xiv) -C₁₋₆alkyle,
xv) -C₁₋₆alkyl-N(R¹¹)-C(=O)-R¹²,
xvi) -CH₂-O-(CH₂)₀₋₁-aryle substitué par R¹⁰,
xvii) -CH₂-O-(CH₂)₀₋₁-aryle,
xviii) -halogène,
xix) halogéno-C₁₋₆alkyle,
xx) hydroxy-C₁₋₆alkyle,
xxi) -N(R⁵,R⁶), et
xxii) -NH-C(=O)C₁₋₆alkyle.

3. Composé, ou sels pharmaceutiquement acceptables correspondants, pour utilisation selon l'une quelconque des revendications 1 à 2, choisi dans le groupe constitué par 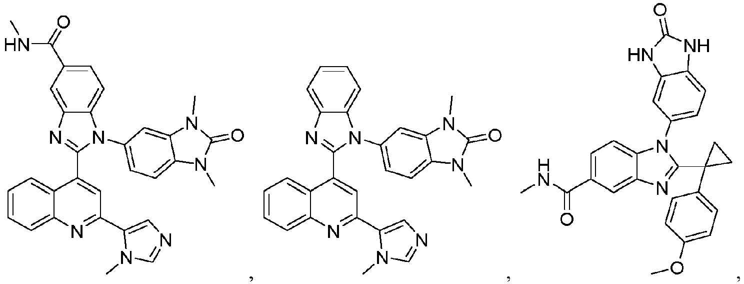 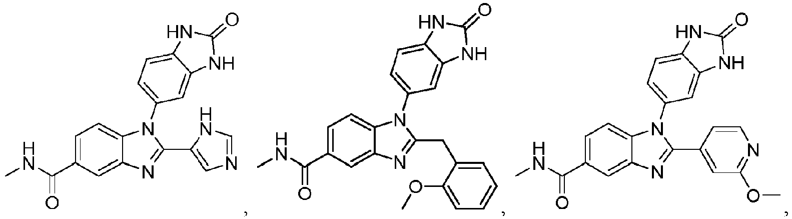 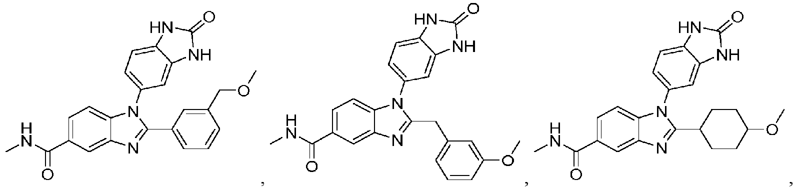 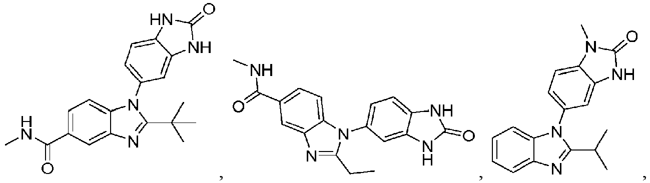 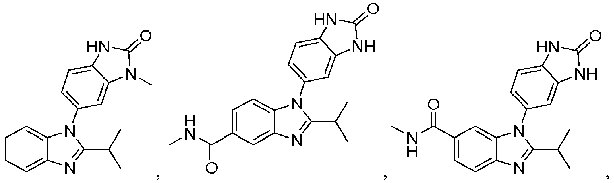 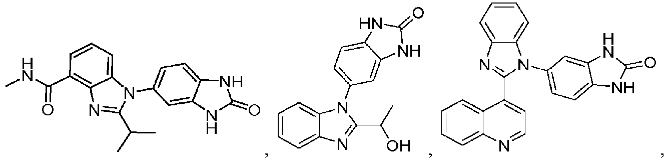 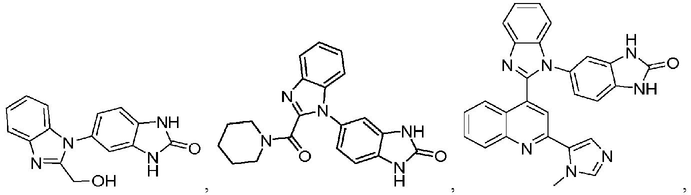 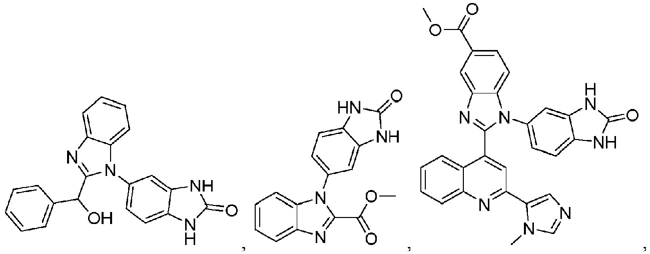 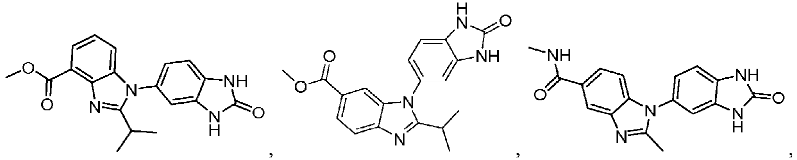 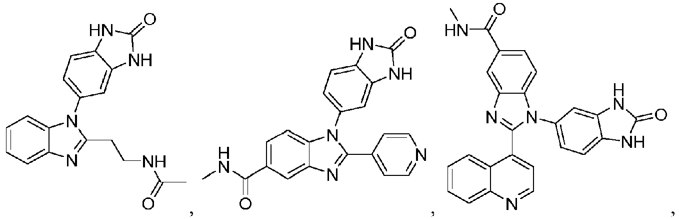 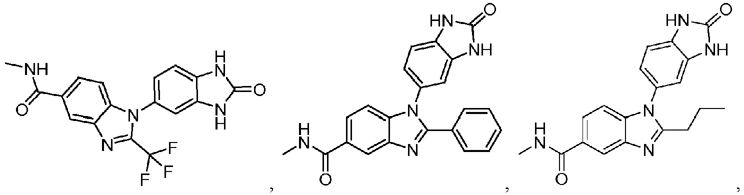 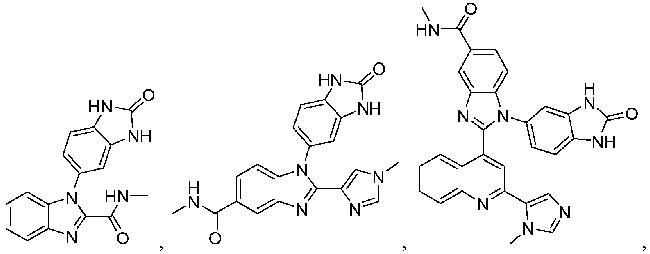 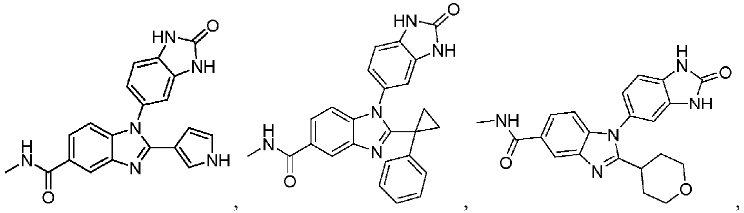 et

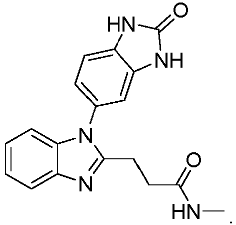

4. Composé, ou sels pharmaceutiquement acceptables correspondants, pour utilisation selon l'une quelconque des revendications 1 à 3, choisi dans le groupe constitué par 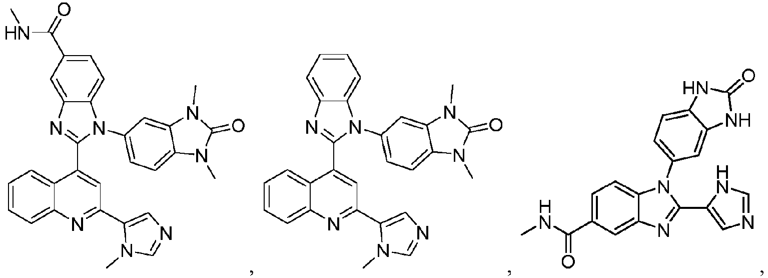 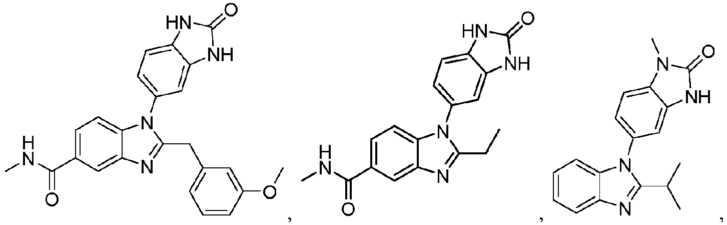 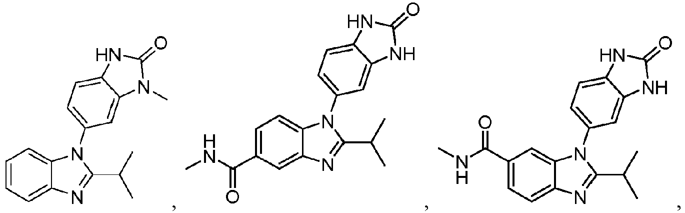 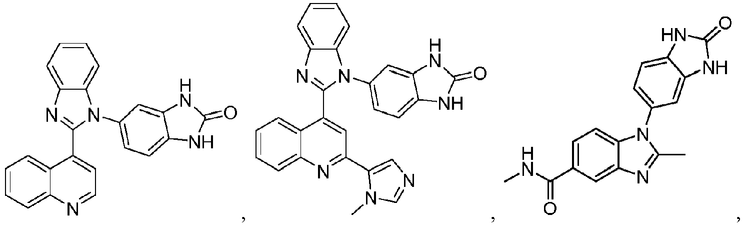 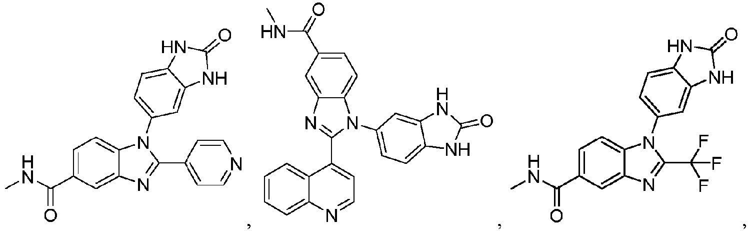 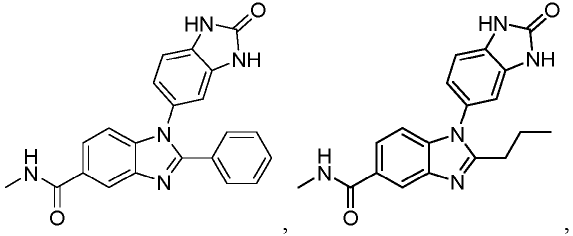 et

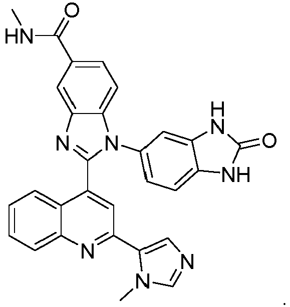

5. Composé, ou sel pharmaceutiquement acceptable correspondant, de formule 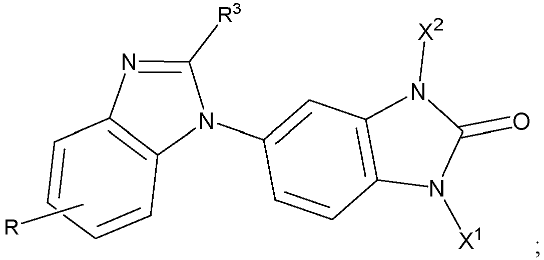
X¹ étant H ou -C₁₋₆alkyle,
X² étant H ou -C₁₋₆alkyle,
R étant H, -C(O)NHMe, ou -C(O)OMe ;
R³ étant indépendamment choisi dans le groupe constitué par
i) -(CH₂)₀₋₁-(C₃₋₇cycloalkyl)-aryle substitué par R¹⁰,
ii) -(CH₂)₀₋₁-(C₃₋₇cycloalkyl)-aryle,
iii) -(CH₂)₀₋₁-aryle substitué par R¹⁰,
iv) -(CH₂)₀₋₁-aryle,
v) -(CH₂)₀₋₁-C₃₋₇cycloalkyle substitué par R¹⁰,
vi) -(CH₂)₀₋₁-C₃₋₇cycloalkyle,
vii) -(CH₂)₀₋₁-hétéroaryle substitué par R⁹,
viii) -(CH₂)₀₋₁-hétéroaryle,
ix) -(CH₂)₀₋₁-hétérocycloalkyle,
x) -C(=O)C₁₋₆alkyle,
xi) -C(=O)-N(R⁷,R⁸),
xii) -C(=O)OC₁₋₆alkyle,
xiii) -C₁₋₆alcoxy,
xiv) -C₁₋₆alkyle,
xv) -C₁₋₆alkyl-N(R¹¹)-C(=O)-R¹²,
xvi) -CH₂-O-(CH₂)₀₋₁-aryle substitué par R¹⁰,
xvii) -CH₂-O-(CH₂)₀₋₁-aryle,
xviii) -halogène,
xix) halogéno-C₁₋₆alkyle,
xx) hydroxy-C₁₋₆alkyle,
xxi) -N(R⁵,R⁶), et
xxii) -NH-C(=O)C₁₋₆alkyle,
R⁵ étant chacun indépendamment choisi parmi H et C₁₋₆alkyle,
R⁶ étant chacun indépendamment choisi parmi H et C₁₋₆alkyle,
R⁷ étant chacun indépendamment choisi parmi H et C₁₋₆alkyle,
R⁸ étant chacun indépendamment choisi parmi H et C₁₋₆alkyle,
ou R⁷ et R⁸ formant conjointement avec l'azote auquel ils sont fixés un hétérocycloalkyle,
R⁹ étant chacun indépendamment choisi dans le groupe constitué par
i) C₁₋₆alcoxy,
ii) C₁₋₆alkyle,
iii) halogène,
iv) halogéno-C₁₋₆alkyle,
v) hétéroaryle, et
vi) hétéroaryle substitué par C₁₋₆alkyle,
R¹⁰ étant chacun indépendamment choisi dans le groupe constitué par
i) C₁₋₆alcoxy,
ii) C₁₋₆alkyle,
iii) halogène, et
iv) halogéno-C₁₋₆alkyle ;
R¹¹ étant chacun indépendamment choisi parmi H et C₁₋₆alkyle, et
R¹² étant chacun indépendamment choisi parmi C₁₋₆alkyle et C₃₋₇cycloalkyle.

6. Composé pour utilisation selon la revendication 1 ou composé selon la revendication 5, ou sel pharmaceutiquement acceptable correspondant, X¹ et X² étant chacun H.

7. Composé pour utilisation selon la revendication 1 ou composé selon l'une quelconque des revendications 5 à 6, ou sel pharmaceutiquement acceptable correspondant, dans lequel R³ est -C(=O)-N(R⁷,R⁸) ou -C(=O)OC₁₋₆alkyle, dans lequel R⁷ et R⁸ sont chacun indépendamment choisis parmi H et C₁₋₆alkyle.

8. Composé pour utilisation selon la revendication 1 ou composé selon l'une quelconque des revendications 5 à 7, ou sels pharmaceutiquement acceptables correspondants, choisi(s) dans le groupe constitué par 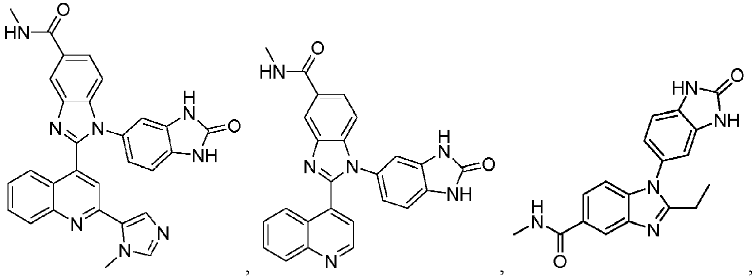 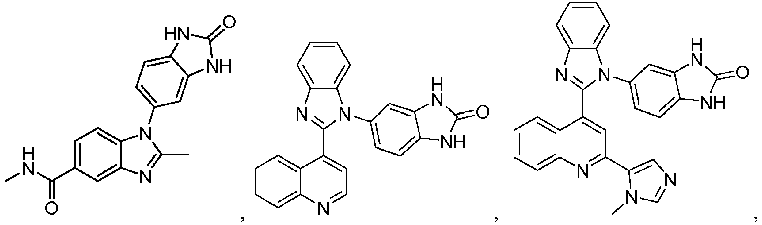 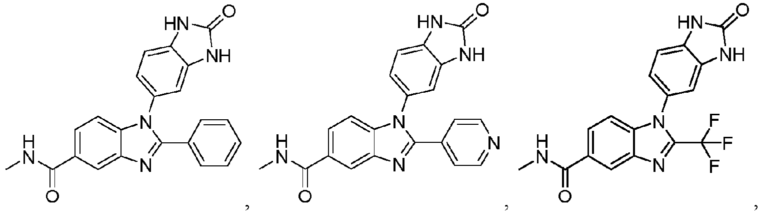 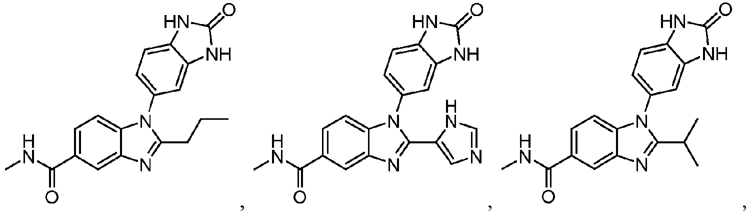 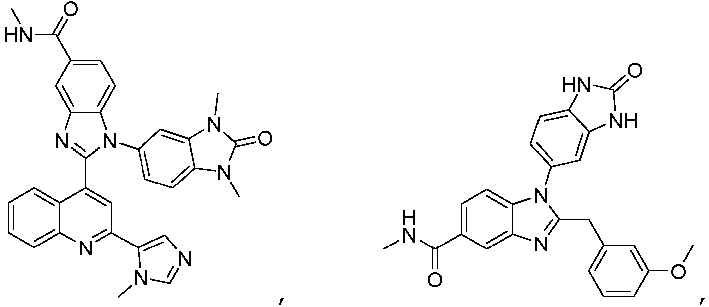 et

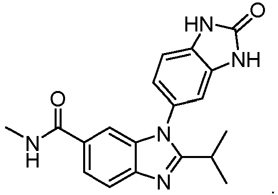

9. Composé selon l'une quelconque des revendications 5 à 8, ou sels pharmaceutiquement acceptables correspondants, pour utilisation dans le traitement thérapeutique et/ou prophylactique d'un cancer.

10. Composé pour utilisation selon l'une quelconque des revendications 1 à 4, ou sels pharmaceutiquement acceptables correspondants, dans lequel le composé est pour utilisation dans le traitement thérapeutique et/ou prophylactique d'un cancer.

11. Composition pharmaceutique comprenant un composé selon l'une quelconque des revendications 1 à 8 et un support thérapeutiquement inerte.
